# EUROPEAN PATENT SPECIFICATION

(11) **EP 1 157 002 B1**
(45) Date of publication and mention of the grant of the patent: **10.08.2005**
(21) Application number: 00901861.5
(22) Date of filing: 18.02.2000
(51) Int. Cl.: C07C 237/20, C07C 237/48, C07C 311/37, C07D 249/06, C07C 229/50

(54) **1,2,3,4-TETRAHYDRO-1-NAPHTHALENAMINE COMPOUNDS USEFUL IN THERAPY**
1, 2, 3, 4-TETRAHYDRO-1-NAPHTHALENAMIN VERBINDUNGEN ZUR THERAPEUTSICHEN VERWENDUNG
COMPOSES 1,2,3,4-TETRAHYDRO-1-NAPHTHALENAMINES UTILES EN THERAPIE

(30) Priority: 01.03.1999 GB 9904691; 09.09.1999 GB 9921314
(43) Date of publication of application: 28.11.2001
(73) Proprietor: Pfizer Limited, Sandwich, Kent CT13 9NJ (GB); PFIZER INC., New York, N.Y. 10017 (US)
(72) Inventor: MIDDLETON, Donald Stuart, Sandwich, Kent CT13 9NJ (GB); STOBIE, Alan, Sandwich, Kent CT13 9NJ (GB)
(74) Representative: Duncan, Garreth Andrew
(86) International application number: PCT/IB2000/000182
(87) International publication number: WO 2000/051972

(56) References cited:
- EP-A- 0 030 081
- EP-A- 0 415 613
- EP-A- 0 714 663
- WO-A-97/13770
- WO-A-99/21508
- US-A- 4 507 323
- US-A- 5 151 448
- US-A- 5 276 042

## Description

This invention relates to 1,2,3,4-tetrahydro-1-naphthalenamine compounds useful in the treatment or prevention of a variety of disorders, including those in which the regulation of monoamine transporter function is implicated, such as depression, attention deficit hyperactivity disorder, obsessive-compulsive disorder, post-traumatic stress disorder, substance abuse disorders and sexual dysfunction including premature ejaculation, and to pharmaceutical formulations containing such compounds.

European Patent N° 30081 discloses a group of 1,2,3,4-tetrahydro-1-naphthalenamine compounds indicated as antidepressants, including cis-(1S)-N-methyl-4-(3,4-dichlorophenyl)-1,2,3,4-tetrahydro-1-naphthalenamine hydrochloride (see Example 2 therein). This compound is known as sertraline and is available as LUSTRAL™ and ZOLOFT™. European Patent N° 30081 also discloses N-methyl-4-(4-chlorophenyl)-7-methoxy-1,2,3,4-tetrahydro-1-naphthalenamine hydrochloride (see Example 21 therein).

European Patent N° 415613 discloses the use of sertraline in the treatment of premature ejaculation. It also gives a vague indication that some of the other compounds disclosed in the sertraline patent may be effective in the treatment of premature ejaculation.

According to the present invention, there is provided a compound of formula I, 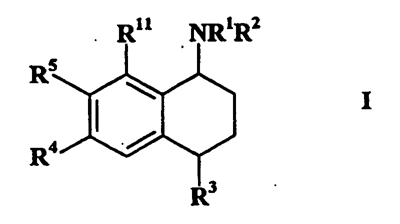 wherein
R¹ and R² independently represent H or C₁₋₆ alkyl;
R³ represents phenyl substituted by at least one group selected from halo, CF₃, OCF₃, CN, OH, C₁₋₆ alkyl and C₁₋₆ alkoxy; and
R⁴, R⁵ and R¹¹ independently represent H or -(CH₂)ₙ-A, wherein n represents 0, 1 or 2, provided that at least one of R⁴, R⁵ and R¹¹ is other than H;
A represents:
CONR⁶R⁷ or SO₂NR⁶R⁷, wherein R⁶ and R⁷ independently represent H, C₃₋₆ cycloalkyl or C₁₋₆ alkyl,
   the C₁₋₆ alkyl group being optionally substituted by one or more groups selected from OH, CO₂H, C₃₋₆ cycloalkyl, NH₂, CONH₂, C₁₋₆ alkoxy, C₁₋₆ alkoxycarbonyl and a 5- or 6-membered heterocylic ring (containing 1, 2 or 3 heteroatoms selected from N, S and O);
   in addition, R⁶ and R⁷ may, together with the N atom to which they are attached, represent a pyrrolidine or piperidine ring (which rings are optionally substituted by OH or CONH₂) or a morpholine ring (which is optionally substituted by CONH₂);
CO₂R⁸, wherein R⁸ represents H or C₁₋₆ alkyl;
NR⁹R¹⁰, wherein R⁹ and R¹⁰ independently represent H, C₁₋₆ alkyl (optionally substituted by OH or C₁₋₆ alkoxy), (C₁₋₆ alkyl)SO₂-, (C₁₋₆ alkyl)CO-, H₂NSO₂- or H₂NCO-:
a 5- or 6-membered heterocyclic ring containing 1, 2 or 3 heteroatoms selected from N, S and O, optionally substituted by one or more groups selected from C₁₋₆ alkyl, NH₂, OH, =O and CONHCH₃;
S(O)ₓ(C₁₋₆ alkyl), wherein x represents 0, 1 or 2;
OH;
CN;
NO₂; or
C₁₋₆ alkoxy substituted by one or more groups selected from SO₂NH₂ and CONH₂;
and pharmaceutically acceptable salts thereof.

The pharmaceutically acceptable salts of the compounds of formula I which contain a basic centre are, for example, non-toxic acid addition salts formed with inorganic acids such as hydrochloric, hydrobromic, sulphuric and phosphoric acid, with carboxylic acids, ammonia or with organo-sulphonic acids. Examples include the hydrochloride, hydrobromide, sulphate or bisulphate, phosphate or hydrogen phosphate, acetate, benzoate, succinate, fumarate, maleate, lactate, citrate, tartrate, glucontate, camsylate, hydrobromide, sulphate or bisulphate, phosphate or hydrogen phosphate, acetate, benzoate, succinate, fumarate, maleate, lactate, citrate, tartrate, gluconate, camsylate, ammonium, methanesulphonate, benzenesulphonate, and p-toluenesulphonate salts. Compounds of formula I containing an acidic centre may also form pharmaceutically acceptable metal salts, in particular non-toxic alkali or alkaline earth metal salts, with bases. Examples include the calcium, sodium and potassium salts. For a review of suitable pharmaceutical salts see J. Pharm, Sci., 1977, 66, 1.

"Halo" includes fluorine, chlorine, bromine and iodine.

Alkyl groups which R¹⁻⁵ and R¹¹ may represent or comprise may be straight chain or branched.

Heterocyclic rings that A may represent or comprise may be saturated or unsaturated. A specific heterocyclic ring that R⁶ or R⁷ may comprise is furanyl. Specific heterocyclic rings that A may represent include oxadiazolyl, triazolyl, pyrazolyl, pyridinyl and pyrimidinyl.

Because R⁴ and R⁵ are defined independently, when both represent -(CH₂)ₙ-A, the nature of these two groups is not necessarily the same. The same applies to other substituents that are defined independently.

The compounds of formula I may possess one or more chiral centres and so exist in a number of stereoisomeric forms. All stereoisomers and mixtures thereof are included in the scope of the present invention. Racemic compounds may either be separated using preparative HPLC and a column with a chiral stationary phase or resolved to yield individual enantiomers utilising methods known to those skilled in the art. In addition, chiral intermediate compounds may be resolved and used to prepare chiral compounds of formula I.

The compounds of formula I may exist in one or more tautomeric forms. All tautomers and mixtures thereof are included in the scope of the present invention. For example, a claim to 2-hydroxypyridinyl would also cover its tautomeric form, α-pyridonyl.

The invention also includes radiolabelled compounds of formula I.

Preferred groups of compounds include those in which:
(a) NR¹R² represents NH(C₁₋₆ alkyl), more preferably N(H)methyl;
(b) R³ represents phenyl disubstituted with halo, more preferably 3,4-dichlorophenyl;
(c) R⁴ represents H;
(d) R⁵ represents -(CH₂)ₙ-A;
(e) R¹¹ represents H;
(f) A represents CONR⁶R⁷, SO₂NR⁶R⁷ or NO₂;
(g) one of R⁶ and R⁷ represents H, and the other represents H or C₁₋₆ alkyl optionally substituted by OH;
(h) n represents 0; and
(i) the stereochemistry is as shown in formula Ia, 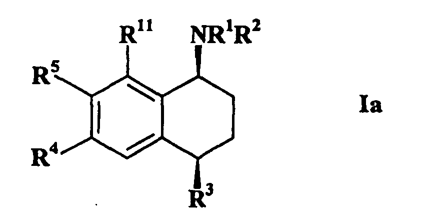
wherein R¹⁻⁵ and R¹¹ are as defined above.

Another group of compounds that may be mentioned is compounds of formula I', 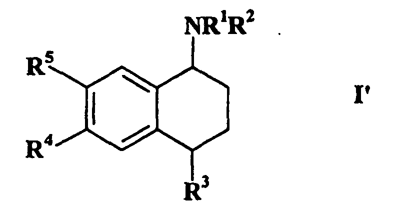 wherein
R¹ and R² independently represent H or C₁₋₆ alkyl; R³, R⁴ and R⁵ are as defined in formula I and H represents CONR⁶R⁷ or SO₂NR⁶R⁷, wherein R⁶ and R⁷ independently represent H, C₃₋₆ cycloalkyl or C₁₋₆ alkyl,
the C₁₋₆ alkyl group being optionally substituted by one or more groups selected from OH, CO₂H, C₃₋₆ cycloalkyl, NH₂, CONH₂, C₁₋₆ alkoxy, C₁₋₆ alkoxycarbonyl and a 5- or 6-membered heterocyclic ring (containing 1, 2 or 3 heteroatoms selected from N, S and O);
in addition, R⁶ and R⁷ may, together with the N atom to which they are attached, represent a pyrrolidine or piperidine ring (which rings are optionally substituted by OH or CONH₂) or a morpholine ring (which is optionally substituted by CONH₂);
CO₂R⁸, wherein R⁸ represents H or C₁₋₆ alkyl;
NR⁹R¹⁰, wherein R⁹ and R¹⁰ independently represent H, C₁₋₆ alkyl (optionally substituted by OH or C₁₋₆ alkoxy), (C₁₋₆ alkyl)SO₂-, (C₁₋₆ alkyl)CO-, H₂NSO₂- or H₂NCO-;
a 5- or 6-membered heterocyclic ring containing 1, 2 or 3 heteroatoms selected from N, S and O, optionally substituted by one or more groups selected from C₁₋₆ alkyl, NH₂, OH₂, =O and CONHCH₃;
S(O)ₓ(C₁₋₆ alkyl), wherein x represents 0, 1 or 2;
OH;
CN;
NO₂; or
C₁₋₆ alkoxy substituted by one or more groups selected from SO₂NH₂ and CONH₂;
and pharmaceutically acceptable salts thereof.

The invention also provides a process for the production of a compound of formula I, as defined above, or a pharmaceutically acceptable salt thereof, which includes:
(a) when R⁴, R⁵ or R¹¹ represents CONR⁶R⁷, reaction of a compound of formula II, 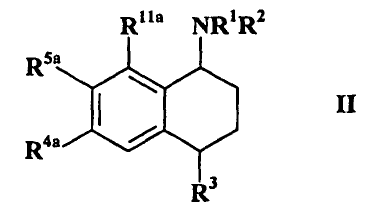 wherein R¹⁻³ are as defined above, the corresponding group R^{4a}, R^{5a} or R^{11a} represents iodo and the remainder represent H or -(CH₂)ₙ-A, wherein n and A are as defined above; with CC and an amine of formula III,

   **HNR**^{**6**}**R**^{**7**} **III**

   wherein R⁶ and R⁷ are as defined above, in the presence of a Pd(0) catalyst;
(b) when R⁴, R⁵ or R¹¹ represents SO₂NR⁶R⁷, reaction of a compound of formula IV, 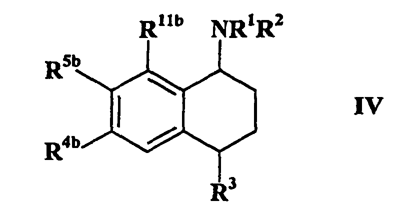 wherein R¹⁻³ are as defined above, the corresponding group R^{4b}, R^{5b} or R^{11b} represents SO₂Cl and the remainder represent H or -(CH₂)ₙ-A, wherein n and A are as defined above; with an amine of formula III, as defined above;
(c) when R⁴, R⁵ or R¹¹ represents CO₂(C₁₋₆ alkyl), reaction of a compound of formula II, as defined above; with CO and a C₁₋₆ alkanol in the presence of a Pd(0) catalyst;
(d) when R⁴, R⁵ or R¹¹ represents NO₂, reaction of a compound of formula V, 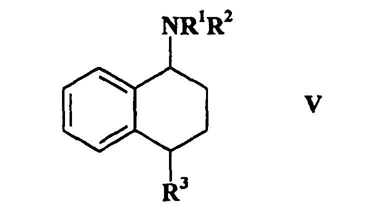 wherein R¹⁻³ are as defined above; with an alkali metal nitrate and a sulphonic acid;
(e) when R⁴, R⁵ or R¹¹ represents a heterocyclic ring attached to the rest of the molecule by a N atom, reaction of a compound of formula II as defined above; with a heterocyclic compound containing an NH group in the ring, in the presence of a copper catalyst;
(f) when R⁴, R⁵ or R¹¹ represents a heterocyclic ring attached to the rest of the molecule by a C atom, reaction of a compound of formula VI, 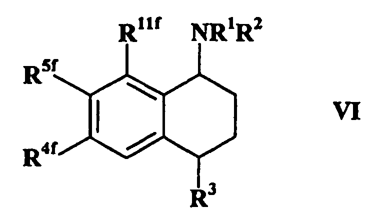 wherein R¹⁻³ are as defined above, the corresponding group R^{4f}, R^{5f} or R^{11f} represents a group of formula F, 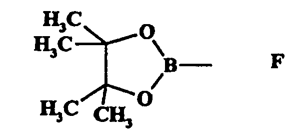 and the remainder represent H or -(CH₂)ₙ-A, wherein n and A are as defined above; with a heterocyclic compound containing a C-Br or C-I group in the ring, in the presence of a Pd(0) catalyst;
(g) when R⁴, R⁵ or R¹¹ represents a heterocyclic ring attached to the rest of the molecule by a C atom, reaction of a compound of formula II, as defined above; with a heterocyclic compound which is optionally substituted with iodo on the C atom by which the heterocyclic ring will be attached to the rest of the molecule, in the presence of butyl lithium and a Pd(0) catalyst;
(h) when R⁴, R⁵ or R¹¹ represents S(O)ₓ(C₁₋₆ alkyl), reaction of a compound of formula II, as defined above; with a compound of formula VII,

   **NaS(C**_{**1-6**} **alkyl) VII**

   in the presence of a copper or palladium catalyst, followed by oxidation if desired to give compounds in which x is 1 or 2;
(i) when R⁴, R⁵ or R¹¹ represents CN, reaction of a compound of formula II, as defined above; with zinc cyanide, in the presence of a Pd(0) catalyst;
(j) when R⁴, R⁵ or R¹¹ represents CH₂CH₂SO₂NR⁶R⁷ or CH₂CH₂SO₂(C₁₋₆ alkyl), reaction of a compound of formula II, as defined above; with a compound of formula IX,

   **CH**_{**2**}**=CHA**^{**k**} **IX**

   wherein A^{k} represents SO₂NR⁶R⁷ or SO₂(C₁₋₆ alkyl) as appropriate, in which R⁶ and R⁷ are as defined above, in the presence of a Pd(II) catalyst, followed by reduction of the resulting alkene;
(k) when R⁴, R⁵ or R¹¹ represents CH₂CH₂CO₂(C₁₋₆ alkyl), reaction of a compound of formula II, as defined above; with a compound of formula X,

   **CH**_{**2**}**=CHCO**_{**2**}**(C**_{**1-6**} **alkyl) X**

   in the presence of a Pd(II) catalyst, followed by reduction of the resulting alkene; or
(l) when one of R¹ and R² represents C₁₋₆ alkyl and the other represents H, removing a protecting group from a compound of formula XI, 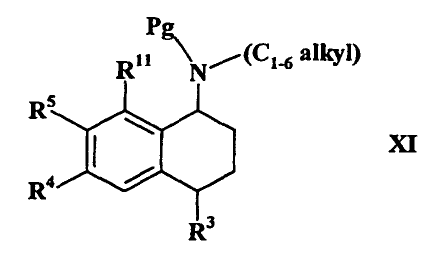 wherein R³⁻⁵ and R¹¹ are as defined above, and Pg is a protecting group;
and where desired or necessary, converting the resulting compound into a pharmaceutically acceptable salt, or vice versa.

In process (a), the reaction is preferably carried out in a solvent that does not adversely affect the reaction (for example dimethylformamide), at an elevated temperature. Suitable Pd(0) catalysts include tetrakis(triphenylphosphine)palladium.

In process (b), the reaction is preferably carried out in a solvent that does not adversely affect the reaction (for example dichloromethane or acetonitrile), at or around room temperature.

In process (c), the reaction is preferably carried out in a solvent that does not adversely affect the reaction (for example dimethylformamide), at an elevated temperature. The solvent may be the reacting C₁₋₆ alkanol (for example methanol). Suitable Pd(0) catalysts include tetrakis(triphenylphosphine)palladium.

In process (d), the reaction is preferably carried out in a solvent that does not adversely affect the reaction (for example trifluoroacetic acid), below room temperature. The alkali metal nitrate may be potassium nitrate and the sulphonic acid may be triflic acid.

In process (e), the reaction is preferably carried out without a solvent, at an elevated temperature (for example 160°C) in the presence of a base such as potassium carbonate.

In process (f), the reaction is preferably carried out in a solvent that does not adversely affect the reaction (for example dioxan/water), at an elevated temperature. Suitable Pd(0) catalysts include tetrakis(triphenylphosphine)palladium. Preferably, caesium carbonate is also present.

In process (g), the reaction is preferably carried out in a solvent that does not adversely affect the reaction (for example diethyl ether or tetrahydrofuran), at an elevated temperature. Suitable Pd(0) catalysts include tetrakis(triphenylphosphine)palladium. Preferably, zinc chloride is also present.

In process (h), the reaction is preferably carried out in a solvent that does not adversely affect the reaction (for example ethylene glycol), at an elevated temperature in the presence of a base such as potassium carbonate. Suitable palladium catalysts include tetrakis(triphenylphosphine)palladium and palladium acetate. Suitable oxidizing agents include hydrogen peroxide in trifluoroacetic acid.

In process (i), suitable palladium catalysts include tetrakis(triphenylphosphine)palladium The reaction is preferably carried out in a solvent that does not adversely affect the reaction (for example dimethylformamide), at an elevated temperature.

In processes (j) and (k), the reaction is preferably carried out in a solvent that does not adversely affect the reaction (for example acetonitrile), at an elevated temperature.

Suitable Pd(II) catalysts include palladium acetate. Preferably, tri(o-tolyl)phosphine and triethylamine are also present. The resulting alkene may be reduced using tosylhydrazine in toluene at an elevated temperature.

In process (1), suitable protecting groups include tert-butyloxycarbonyl. In this case, the protecting group is preferably removed by the action of HCl, for example by bubbling HCl gas through a solution of the compound of formula XI, or by adding a saturated solution of HCl in a solvent such as dichloromethane to a compound of formula XI. Preferably, the reaction is carried out below room temperature, for example 0°C.

The invention also provides compounds of formulae II, IV, VI and XI as defined above. When R⁴ represents H, compounds of formulae II, IV and VI may be obtained from compounds of formula V as shown in the following scheme (in which R¹⁻³ are as defined above) and as illustrated by the accompanying Examples:

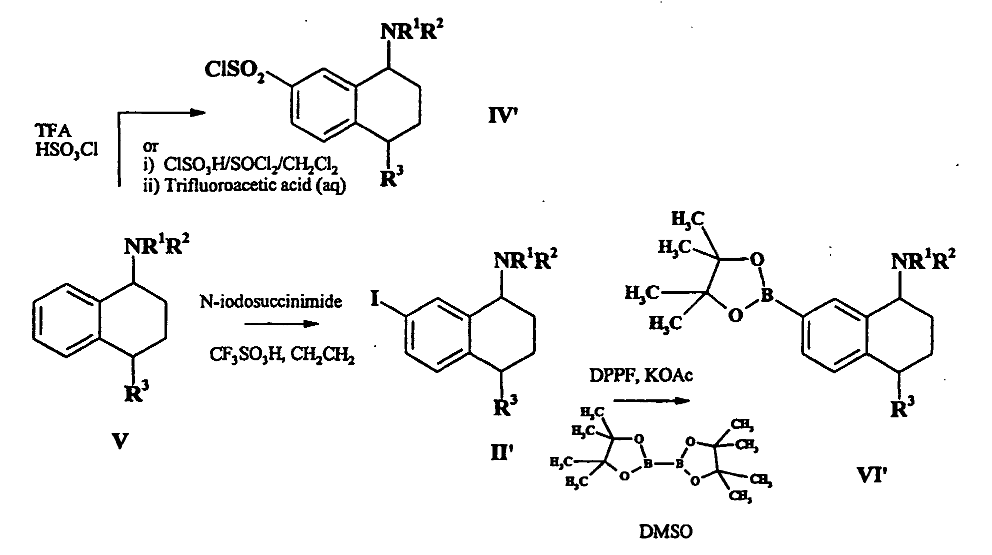

Compounds of formula V are disclosed in European Patent N° 30081. The following general method is given on page 5 of European Patent N° 30081, and is used to produce sertraline (the compound in which W represents H, and X and Y each represent Cl):

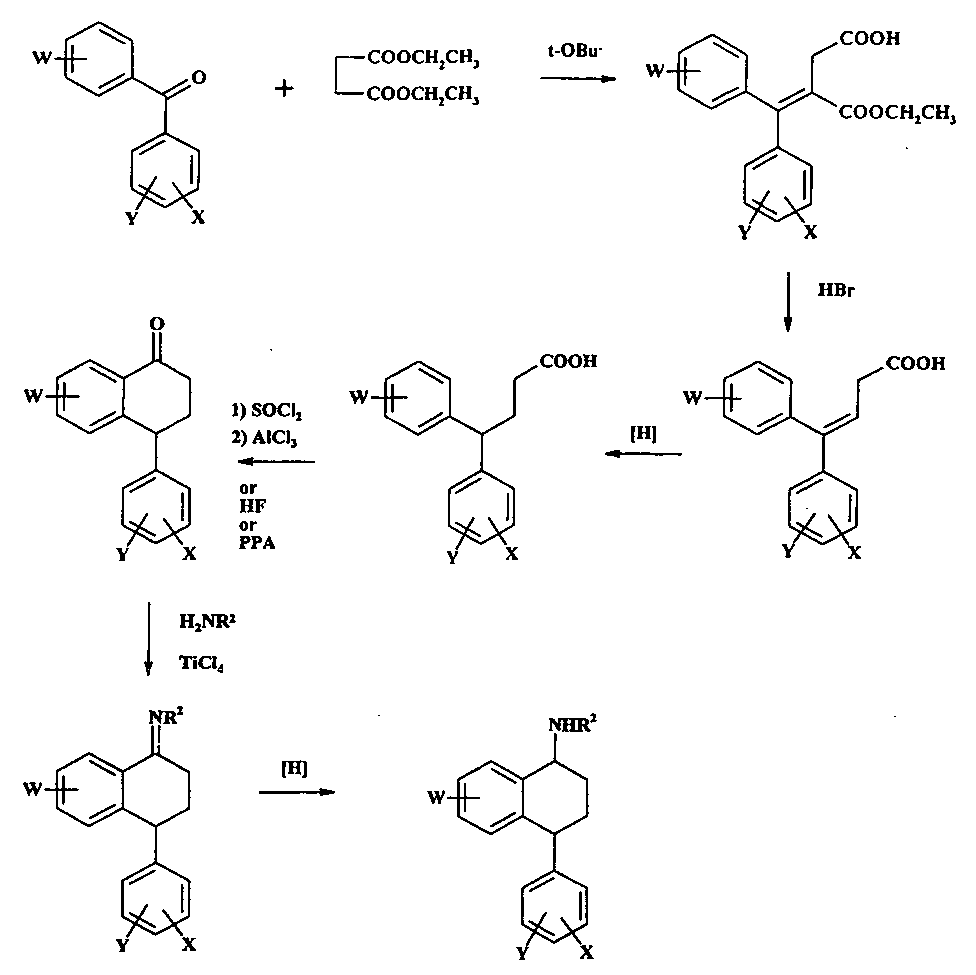

Compounds of formula I may be converted into other compounds of formula I using known techniques, as illustrated by the examples. For example when A represents:
(1) NH₂, it may be converted to OH by reaction with formic acid and acetic anhydride and then reacting the resulting formamide with sodium nitrite and concentrated sulphuric acid;
(2) NH₂, it may be converted to NH(C₁₋₆ alkyl) by reductive amination with a C₁₋₆ aldehyde and sodium triacetoxyborohydride;
(3) CO₂(C₁₋₆ alkyl), it may be reduced to CH₂OH, which may in turn be converted to CH₂Cl, both of which groups may undergo a wide variety of reactions;
(4) CO₂(C₁₋₆ alkyl), it may be converted to CONR⁶R⁷ by reaction with HNR⁶R⁷;
(5) NO₂, it may be converted to NH₂ by reaction with iron powder and calcium chloride;
(6) OH, it may be converted to optionally substituted C₁₋₆ alkoxy by reaction with Cl-(optionally substituted C₁₋₆ alkyl);
(7) CN, it may be converted to CH₂NH₂ by reduction using lithium aluminium hydride;
(8) CN, it may be converted to CONH₂ by reaction with concentrated sulphuric acid; and
(9) CONH₂, it may be converted to CH₂NH₂ by reaction with B₂H₆ in tetrahydrofuran (see Kruijtzer *et al*, Tetrahedron Lett, vol 38(30), 1997, pp 5335-5338), which may then be reacted with MeSO₂Cl in pyridine (see Ito *et al*, Chem Pharm Bull, vol 25(7), 1977, pp 1732-1739) to give a group of formula CH₂NHSO₂Me.

When R⁴, R⁵ or R¹¹ represents CH₂OH, it may be converted to CH₂Cl by reaction with hydrogen chloride and thionyl chloride, which group may undergo displacement of the chlorine atom by, for example a heterocyclic compound containing an NH group, to give compounds of formula I in which n is 1. The CH₂Cl group may also be reacted with thiourea and sodium hydroxide (see Yamada *et al*, J Med Chem, vol 39(2), 1996, pp 594-604) to give a group of formula CH₂SH, which may then be reacted with KNO₃ and SO₂Cl₂ in CH₃CN (see Park *et al*, Chem Lett, vol 8, 1992, pp 1483-1486) to give a group of formula CH₂SO₂NH₂.

In the above processes (a)-(k), preferably the reaction is carried out to produce a compound of formula I in which one of R⁴ and R⁵ is -(CH₂)ₙ-A and the other is H and R¹¹ is H.

As illustrated in Example 88 below, an iodine atom may be introduced into the 6-position of the 1,2,3,4-tetrahydro-1-naphthalenamine ring using benzyltrimethylammonium dichloroiodate, giving access to compounds of formula I in which R⁴ and R⁵ both independently represent -(CH₂)ₙ-A. In compounds of this type, when R⁵ represents NH₂, this group can be replaced by hydrogen by reaction with tert-butylnitrite to give compounds in which R⁴ represents -(CH₂)ₙ-A and R⁵ represents H (see Example 90 below).

As illustrated in Example 112 below, a bromine atom may be introduced into the 8-position of the 1,2,3,4-tetrahydro-1-naphthalenamine system using N-bromosuccinimide, giving access to compounds of formula I in which R¹¹ represents -(CH₂)ₙ-A.

Compounds of formulae III, V, VII, IX and X are either known or are available using known techniques.

It will be apparent to those skilled in the art that sensitive functional groups may need to be protected and deprotected during synthesis of a compound of formula I. This may be achieved by conventional techniques, for example as described in 'Protective Groups in Organic Synthesis', 3rd edition, by T W Greene and P G M Wuts, John Wiley and Sons Inc, 1999. Compounds of formula XI in which the amine group at position 1 of the 1,2,3,4-tetrahydro-1-naphthalenamine system is protected are illustrated by the accompanying examples.

The compounds of formula I, and their pharmaceutically acceptable salts, are useful because they have pharmacological activity in animals, including humans. More particularly, they are useful in the manufacture of a medicament for the treatment or prevention of a disorder in which the regulation of monoamine transporter function is implicated. Disease states that may be mentioned include hypertension, depression (e.g. depression in cancer patients, depression in Parkinson's patients, postmyocardial infarction depression, subsyndromal symptomatic depression, depression in infertile women, paediatric depression, major depression, single episode depression, recurrent depression, child abuse induced depression, and post partum depression), generalized anxiety disorder, phobias (e.g. agoraphobia, social phobia and simple phobias), posttraumatic stress syndrome, avoidant personality disorder, premature ejaculation, eating disorders (e.g. anorexia nervosa and bulimia nervosa), obesity, chemical dependencies (e.g. addictions to alcohol, cocaine, heroin, phenobarbital, nicotine and benzodiazepines), cluster headache, migraine, pain, Alzheimer's disease, obsessive-compulsive disorder, panic disorder, memory disorders (e.g. dementia, amnestic disorders, and age-related cognitive decline (ARCD)), Parkinson's diseases (e.g. dementia in Parkinson's disease, neuroleptic-induced parkinsonism and tardive dyskinesias), endocrine disorders (e.g. hyperprolactinaemia), vasospasm (particularly in the cerebral vasculature), cerebellar ataxia, gastrointestinal tract disorders (involving changes in motility and secretion), negative symptoms of schizophrenia, premenstrual syndrome, fibromyalgia syndrome, stress incontinence, Tourette's syndrome, trichotilloinania, kleptomania, male impotence, attention deficit hyperactivity disorder (ADHD), chronic paroxysmal hemicrania, headache (associated with vascular disorders); emotional lability, pathological crying and sleeping disorder (cataplexy).

Disorders of particular interest include depression, attention deficit hyperactivity disorder, obsessive-compulsive disorder, post-traumatic stress disorder, substance abuse disorders and sexual dysfunction including (in particular) premature ejaculation. The compounds of formula I, and their pharmaceutically acceptable salts, may be administered alone or as part of a combination therapy.

Premature ejaculation may be defined as persistent or recurrent ejaculation before, upon or shortly after penile penetration of a sexual partner. It may also be defined as ejaculation occurring before the individual wishes [see 'The Merck Manual', 16^{th} edition, p 1576, published by Merck Research Laboratories, 1992].

Thus, according to a further aspect of the invention, there is provided a compound of formula I, as defined above, or a pharmaceutically acceptable salt thereof, for use as a pharmaceutical.

There is further provided a pharmaceutical formulation containing a compound of formula I, as defined above, or a pharmaceutically acceptable salt thereof, and a pharmaceutically acceptable adjuvant, diluent or carrier.

The invention also provides the use of a compound of formula I, as defined above, or a pharmaceutically acceptable salt thereof, in the manufacture of a medicament for the treatment or prevention of a disorder in which the regulation of monoamine transporter function is implicated, for example depression, attention deficit hyperactivity disorder, obsessive-compulsive disorder, post-traumatic stress disorder, substance abuse disorders or sexual dysfunction including premature ejaculation.

For human use the compounds of formula I, and their pharmaceutically acceptable salts, can be administered alone but will generally be administered in admixture with a suitable pharmaceutical excipient, diluent or carrier selected with regard to the intended route of administration and standard pharmaceutical practice.

For example, the compounds of formula I, and their pharmaceutically acceptable salts, can be administered orally, buccally or sublingually in the form of tablets, capsules, ovules, elixirs, solutions or suspensions, which may contain flavouring or colouring agents, for immediate-, delayed-, modified-, sustained-, pulsed- or controlled-release applications.

Such tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycollate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included.

Solid compositions of a similar type may also be employed as fillers in gelatin capsules. Preferred excipients in this regard include lactose, starch, a cellulose, milk sugar or high molecular weight polyethylene glycols. For aqueous suspensions and/or elixirs, the compounds of formula I, and their pharmaceutically acceptable salts, may be combined with various sweetening or flavouring agents, colouring matter or dyes, with emulsifying and/or suspending agents and with diluents such as water, ethanol, propylene glycol and glycerin, and combinations thereof.

The compounds of formula I, and their pharmaceutically acceptable salts, can also be administered parenterally, for example, intravenously, intra-arterially, intraperitoneally, intrathecally, intraventricularly, intraurethrally, intrasternally, intracranially, intramuscularly or subcutaneously, or they may be administered by infusion techniques. For such parenteral administration they are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well known to those skilled in the art.

The compounds of formula I, and their pharmaceutically acceptable salts, can also be administered intranasally or by inhalation and are conveniently delivered in the form of a dry powder inhaler or an aerosol spray presentation from a pressurised container, pump, spray or nebulizer with the use of a suitable propellant, e.g. dichlorodifluoromethane, trichlorofluoromethane, dichlorotetrafluoroethane, a hydrofluoroalkane such as 1,1,1,2-tetrafluoroethane (HFA 134A [trade mark]) or 1,1,1,2,3,3,3-heptafluoropropane (HFA 227EA [trade mark]), carbon dioxide or other suitable gas. In the case of a pressurised aerosol, the dosage unit may be determined by providing a valve to deliver a metered amount. The pressurised container, pump, spray or nebulizer may contain a solution or suspension of the active compound, e.g. using a mixture of ethanol and the propellant as the solvent, which may additionally contain a lubricant, e.g. sorbitan trioleate. Capsules and cartridges (made, for example, from gelatin) for use in an inhaler or insufflator may be formulated to contain a powder mix of a compound of formula I, and their pharmaceutically acceptable salts, and a suitable powder base such as lactose or starch.

Alternatively, the compounds of formula I, and their pharmaceutically acceptable salts, can be administered in the form of a suppository or pessary, or they may be applied topically in the form of a gel, hydrogel, lotion, solution, cream, ointment or dusting powder. The compounds of formula I, and their pharmaceutically acceptable salts, may also be dermally or transdermally administered, for example, by the use of a skin patch. They may also be administered by the pulmonary or rectal routes.

They may also be administered by the ocular route, particularly for treatment of the eye. For ophthalmic use, the compounds can be formulated as micronized suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

For application topically to the skin, the compounds of formula I, and their pharmaceutically acceptable salts, can be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

Oral administration is preferred. Preferably, administration takes place shortly before an effect is required.

Without being limited by theory, the compounds of formula I are believed to be serotonin re-uptake inhibitors (SRIs). Compounds that selectively inhibit the re-uptake of serotonin, but not noradrenaline or dopamine, are preferred.

The compounds of formula I, and their pharmaceutically acceptable salts, have the advantage that they are selective inhibitors of the re-uptake of serotonin (and so are likely to have reduced side effects), they have a rapid onset of action (making them suitable for administration shortly before an effect is required), they are more potent, or have other more desirable properties than the compounds of the prior art.

The biological activity of the compounds of formula I, and their pharmaceutically acceptable salts, may be demonstrated in the following test:

### Test A

### Uptake of ³H-biogenic amines into human embryonic kidney cells expressing the human serotonin (5-HT), noradrenalin or dopamine transporter

### Cell Culture

Human embryonic kidney cells (HEK-293) stably transfected with either the human serotonin transporter (hSERT), noradrenalin transporter (hNET) or dopamine transporter (hDAT) were cultured under standard cell culture techniques (cells were grown at 37°C and 5% CO₂ in DMEM-culture media (supplemented with 10% dialysed foetal calf serum (FCS), 2mM 1-glutamine and 250µg/ml geneticin)). Cells were harvested for the assay to yield a cell suspension of 750,000 cells/ml.

### Determination of inhibitor potency

All test compounds were dissolved in 100% DMSO and diluted down in assay buffer to give appropriate test concentrations. Assays were carried out in 96-well filter bottom plates. Cells (7500 cells/assay well) were pre-incubated in standard assay buffer containing either test compound, standard inhibitor or compound vehicle (1% DMSO) for 5 minutes. Reactions were started by addition of either ³H-Serotonin, ³H-Noradrenaline or ³H-Dopamine substrates. All reactions were carried out at room temperature in a shaking incubator. Incubation times were 5 minutes for the hSERT and hDAT assays and 15 minutes for the hNET assay. Reactions were terminated by removal of the reaction mixture using a vacuum manifold followed by rapid washing with ice cold assay buffer. The quantity of ³H-substrate incorporated into the cells was then quantified.

Assay plates were dried in a microwave oven, scintillation fluid added, and radioactivity measured. Potency of test compounds was quantified as IC₅₀ values (concentration of test compound required to inhibit the specific uptake of radiolabelled substrate into the cells by 50%).

**Standard Assay Buffer Composition:**
Trizma hydrochloride (26mM)
NaCl (124mM)
KCl (4.5mM)
KH₂PO₄ (1.2mM)
MgCl₂.6H₂O (1.3mM)
Ascorbic acid (1.136mM)
Glucose (5.55mM)
*pH 7.40*
CaCl₂ (2.8mM)
Pargyline (100µM)

Note: The pH of the buffer was adjusted to 7.40 with 1M NaOH before addition of CaCl₂ and pargyline.

### Summary of Assay Parameters

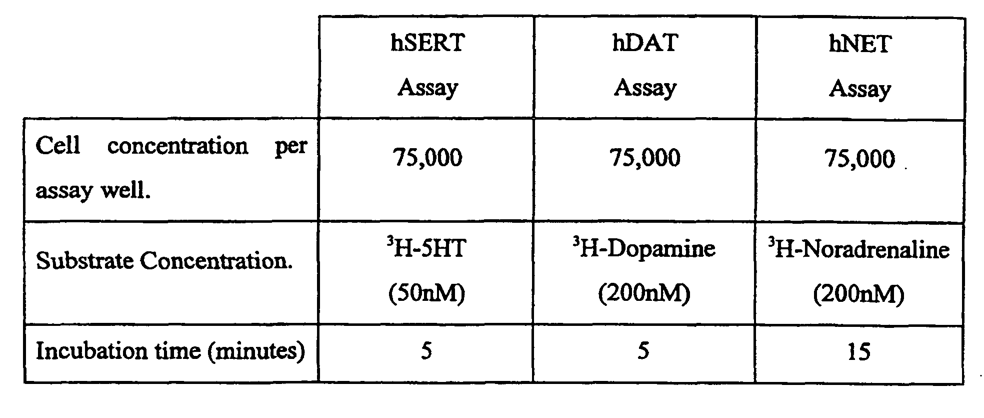

The invention is illustrated by the following Examples, in which the following abbreviations are used:
- 0.88 ammonia: concentrated ammonium hydroxide solution, 0.88 SG
- BOC: tert-butyloxycarbonyl
- DMAP: 4-N,N-dimethylaminopyridine
- EDC: 1-(3-dimethylaminopropyl)-3-ethylcarbodiimide
- Et₂O: diethylether
- h: hour
- HOBT: 1-hydroxybenzotriazole hydrate
- min: minute
- DMSO: dimethylsulphoxide
- EtOAc: ethyl acetate
- MS: mass spectrum
- NMR: nuclear magnetic resonance
- TBTU: O-benzothiazol-1-yl-N,N,N',N'-tetramethyluronium tetrafluoroborate

### Example 1

### Cis-(1S)-N-methyl-7-carboxamido-4-(3,4-dichlorophenyl)-1,2,3,4-tetrahydro-1-naphthalenamine

### (a) Cis-(1S)-N-methyl-7-iodo-4-(3,4-dichlorophenyl)-1,2,3,4-tetrahydro-1-naphthalenamine

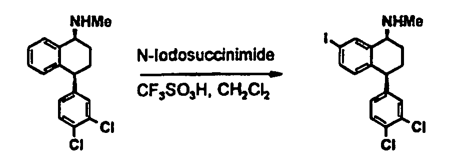

N-Iodosuccinimide (19.7g, 0.088mol) was added to a stirred solution of cis-(1S)-N-methyl-4-(3,4-dichlorophenyl)-1,2,3,4-tetrahydro-1-naphthalenamine (Sertraline, see European Patent 0030081, Example 2) (30g, 0.098mol) and trifluoromethanesulphonic acid (29ml) in dichloromethane (150ml) at 0°C. The reaction was stirred for 16h and a further portion of N-iodosuccinimide (4.41g, 0.0196mol) added. After 7 hours, 2N aqueous sodium hydroxide solution (200ml) was added and the mixture extracted with diethyl ether (3x200ml). The combined organic extracts were washed with saturated aqueous sodium thiosulphate solution (200ml), dried (MgSO₄), filtered and evaporated under reduced pressure to give a brown oil. The crude compound was purified on silica gel eluting with 50:50:1 ethyl acetate:pentane:diethylamine to give the subtitle compound as a yellow oil (29.5g, 79%). MS *m*/*z* 431 (M)⁺. ¹H-NMR (CDCl₃): δ = 1.79 (1H, m), 1.99 (3H, m), 2.53 (3H, m), 3.66 (1H, t), 3.92 (1H, dd), 6.54 (1H, d), 6.94 (1H, d), 7.22 (1H, s), 7.35 (1H, d), 7.42 (1H, d), 7.73 (1H, s).

### (b) Cis-(1S)-N-methyl-7-carboxamido-4-(3,4-dichlorophenyl)-1,2,3,4-tetrahydro-1-naphthalenamine

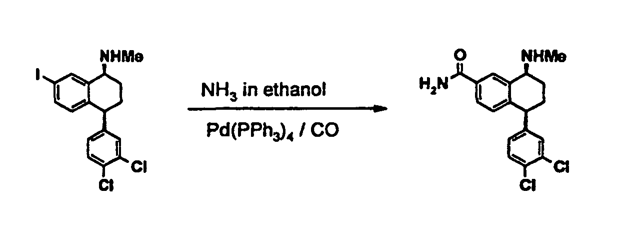

The product iodide from step (a) (0.25g, 0.00058mol), tetrakis(triphenylphosphine)palladium (0.033g, 0.00003mol) and triethylamine (0.12ml) in ammoniacal ethanol (15ml) were heated at 80°C at 690 kPa (100 p.s.i.) pressure under an atmosphere of carbon monoxide for 3 hours. The reaction was cooled, the solvent removed under reduced pressure and the crude product purified on silica gel eluting with a gradient of 97:3:0.5 to 90:10:2 dichloromethane:methanol: 0.88 ammonia to give the subtitle compound as a white foam (0.03g, 13%). MS *m*/*z* 348 (M)⁺. ¹H-NMR (CDCl₃): δ = 1.90 (1H, m), 2.06 (3H, m), 2.57 (3H, s), 3.81 (1H, t), 4.02 (1H, dd), 6.90 (1H, d), 6.97 (1H, d), 7.25 (1H, s), 7.38 (1H, d), 7.55 (1H, d), 7.93 (1H, s).

An alternative route to the title compound of Example 1 is given in steps (c) and (d) below:

### (c) Cis-(1S)-N-methyl-7-cyano-4-(3,4-dichlorophenyl)-1,2,3,4-tetrahydro-1-naphthalenamine hydrochloride

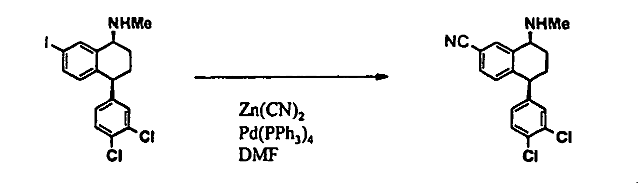

Zinc cyanide (0.07g, 0.0006mol) and tetrakis(triphenylphosphine)palladium (0.08g, 0.00007mol) were added to a stirred solution of the iodide produced in step (a) (0.37g, 0.0085mol) in N,N-dimethylformamide (10ml) and the mixture heated at 100°C for 3 hours. The mixture was cooled, poured into water (30ml) and extracted with diethyl ether (50ml). The organic phase was washed with water (3x50ml), dried (MgSO₄), filtered and the solvent removed under reduced pressure. The crude product was purified on silica gel eluting with 97.5:2.5:0.25 dichloromethane:methanol:0.88 ammonia. The solvent was removed under reduced pressure and the residue treated with a saturated solution of hydrogen chloride in diethyl ether (15ml). The solvent was removed under reduced pressure, the residue dissolved in methanol (15ml) and the solvent removed *in vacuo* to give the subtitle compound (0.173g, 62%). MS *m*/*z* 330 (M)⁺. ¹H-NMR (CDCl₃): δ = 2.13 (2H, m), 2.36 (2H, m), 2.70 (3H, s), 4.00 (1H, dd), 4.40 (1H, t), 7.02 (1H, d), 7.22 (1H, d), 7.37 (1H, d), 7.49 (2H, m), 8.17 (1H, s).

### (d) Cis-(1S)-N-methyl-7-carboxamido-4-(3,4-dichlorophenyl)-1,2,3,4-tetrahydro-1-naphthalenamine

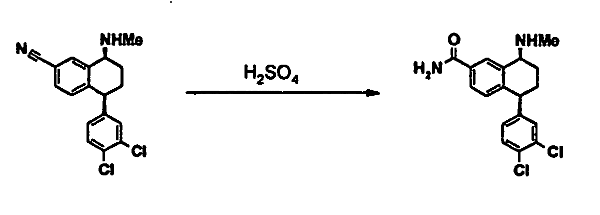

A solution of the nitrile produced in step (c) (1.3g, 0.0039mol) in concentrated sulphuric acid (40ml) was heated at 100°C for 70 minutes. The mixture was cooled and poured into water (300ml). The mixture was extracted with ethyl acetate (2x100ml), the organic extracts combined, dried (MgSO₄), filtered and the solvent removed under reduced pressure. The crude product was purified on silica gel eluting with a gradient of 90:10:2 to 80:20:3 dichloromethane:methanol:0.88 ammonia to give the title compound (0.34g, 25%), identical with the material obtained in step (b).

Using the general procedure described in Example 1(b), the following amides were prepared by reaction of the iodide produced in Example 1(a) and the appropriate amine:

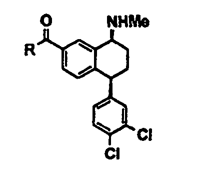

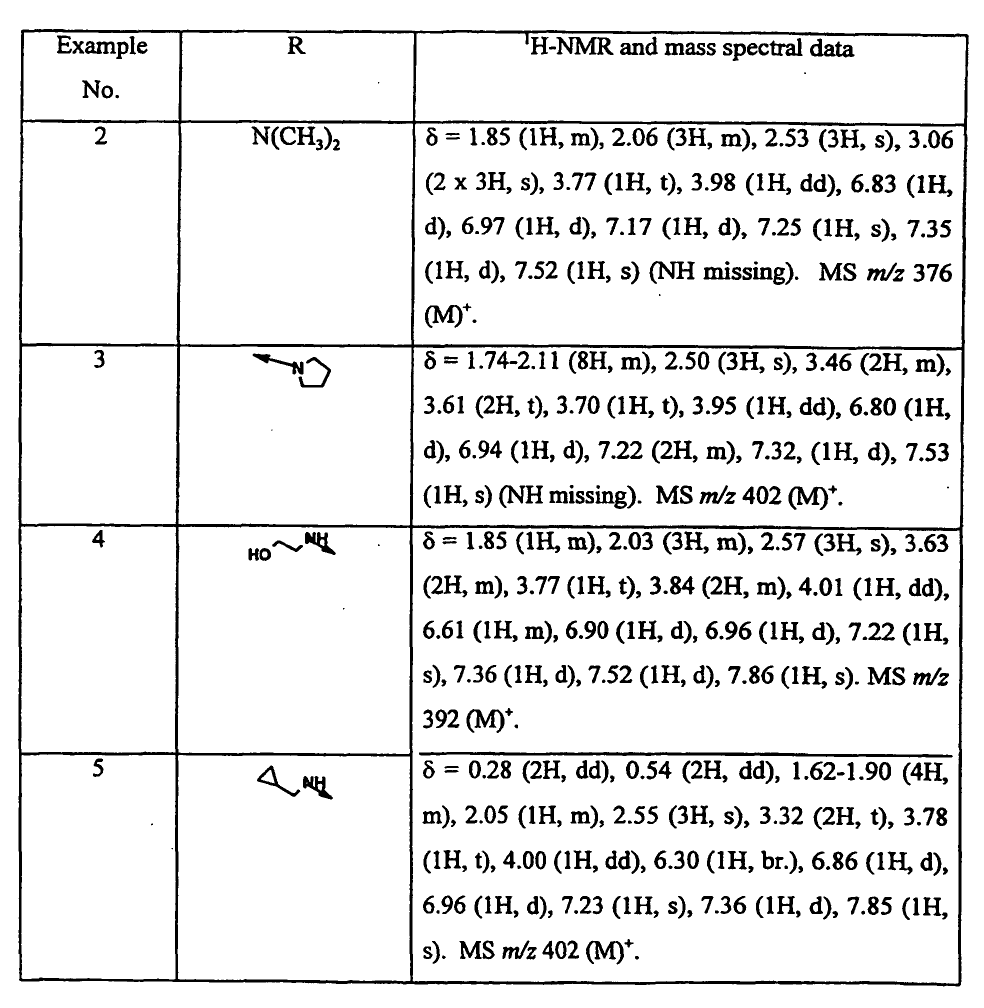

### Example 6

### Cis-(1S)-N-methyl-7-(N-methylamido)-4-(3,4-dichlorophenyl)-1,2,3,4-tetrahydro-1-naphthalenamine

### (a) Cis-(1S)-N-methyl-7-(methoxycarbonyl)-4-(3,4-dichlorophenyl)-1,2,3,4-tetrahydro-1-naphthalenamine

The iodide product of Example 1(a) (0.5g, 0.0012mol), tetrakis(triphenylphosphine)palladium (0.067g, 0.00006mol) and triethylamine (0.27ml) in methanol (12ml) were heated at 80°C at 690 kPa (100 p.s.i.) pressure under an atmosphere of carbon monoxide for 2 hours. The reaction was cooled, the solvent removed under reduced pressure and the crude reaction mixture partitioned between saturated aqueous potassium carbonate (50ml) and ethyl acetate (50ml). The organic layer was dried (MgSO₄), filtered, the solvent removed under reduced pressure and the product purified on silica gel eluting with a gradient of 100:0 to 97:3 dichloromethane:methanol to give the subtitle compound (0.424g, 100%). MS *m*/*z* 364 (MH)⁺. ¹H-NMR (CDCl₃): δ = 1.83 (1H, m), 2.05 (3H, m), 2.55 (3H, s), 3.77 (1H, t), 3.92 (3H, s), 4.21 (1H, dd), 6.89 (1H, d), 6.96 (1H, d), 7.25 (1H, s), 7.37 (1H, d), 7.77 (1H, d), 8.05 (1H, s).

### (b) Cis-(1S)-N-methyl-7-(N-methylamido)-4-(3,4-dichlorophenyl)-1,2,3,4-tetrahydro-1-naphthalenamine

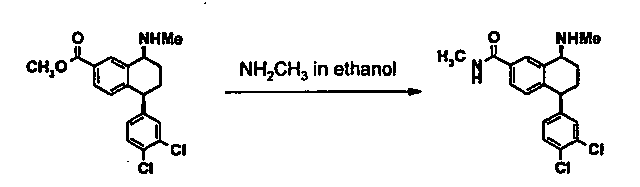

A solution of the ester product of step (a) (0.15g, 0.0004mol) in ethanolic methylamine (5ml) was heated at 100°C for 16 hours. The reaction was cooled, the solvent removed under reduced pressure and the residue purified on silica gel eluting with a gradient of 100:0:0 to 95:5:0.5 dichloromethane:methanol:0.88 ammonia. The solvent was removed under reduced pressure, the product azeotroped in toluene (3x5ml) and then stirred with diethyl ether (10ml) to give the title compound. (0.07g, 47%). ¹H-NNM (CDCl₃): δ = 1.83 (1H, m), 2.03 (3H, m), 2.55 (3H, s), 3.02 (3H, d), 3.75 (1H, t), 4.00 (1H, dd), 6.17 (1H, br.), 6.85 (1H, d), 6.94 (1H, d), 7.21 (1H, s), 7.35 (1H, d), 7.47 (1H, d), 7.82 (1H, s). MS *m*/*z* 363 (MH)⁺.

### Example 7

### Cis-(1S)-N-methyl-7-(N-(carboxymethyl)amido)-4-(3,4-dichlorophenyl)-1,2,3,4-tetrahydro-1-naphthalenamine

### (a) Cis-(1S)-N-methyl-7-(N-(methoxycarbonylmethyl)amino)-4-(3,4-dichlorophenyl)-1,2,3,4-tetrahydro-1-naphthalenamine

The iodide product of step (a) (0.4g, 0.0009mol), tetrakis(triphenylphosphine)palladium (0.054g, 0.00005mol), glycine methyl ester hydrochloride (1.16g, 10 equivs) and triethylamine (0.32ml) in N,N-dimethylformamide (10ml) were heated at 100°C under an atmosphere of carbon monoxide for 14 hours. The reaction was cooled, the solvent removed under reduced pressure and the crude reaction mixture purified on silica gel eluting with a gradient of 97:3:0.25 to 90:10:2 dichloromethane:methanol:0.88 ammonia to give the subtitle compound (0.28g, 72%). ¹H-NMR (CDCl₃): δ = 1.96 (1H, m), 2.11 (3H, m), 2.58 (3H, s), 3.76 (3H, s), 4.05 (3H, m), 4.20 (1H, t), 6.89 (1H, d), 7.02 (1H, d), 7.27 (1H, s), 7.36 (1H, d), 7.62 (1H, d), 8.12 (1H, s). MS *m*/*z* 421 (MH)⁺.

### (b) Cis-(1S)-N-methyl-7-(N-(carboxymethyl)amido)-4-(3,4-dichlorophenyl)-1,2,3,4-tetrahydro-1-naphthalenamine

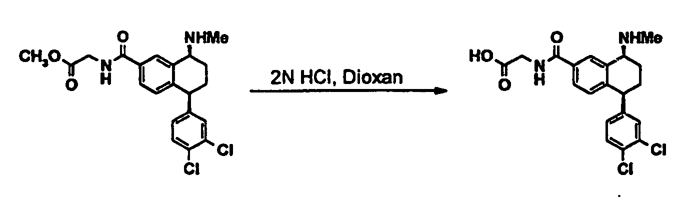

A solution of the ester product of step (a) (0.274g, 0.00065mol) and 2N aqueous hydrochloric acid (4ml) in dioxan (10ml) was heated at reflux for 16 hours. The mixture was cooled to room temperature, the solvent removed under reduced pressure and the crude product purified on silica gel eluting with a gradient of 90:10:2 to 80:20:3 dichloromethane:methanol:0.88 ammonia to give the title compound as a white powder (0.148g, 56%). ¹H-NMR (d₆-DMSO): δ = 1.79-2.15 (4H, m), 2.50 (3H, d), 3.67 (2H, s), 4.09 (1H, m), 4.15 (1H, m), 6.71 (5/8H, d), 6.80 (3/8H, d), 7.23 (1H, m), 7.50 (1H, s), 7.55 (1H, d), 7.59-7.72 (1H, m), 8.12 (3/8H, s), 8.38 (5/8H, s).

### Example 8

### Cis-(1S)-N-methyl-7-sulphonamido-4-(3,4-dichlorophenyl)-1,2,3,4-tetrahydro-1-naphthalenamine

### (a) Cis-(1S)-N-methyl-7-chlorosulphonyl-4-(3,4-dichlorophenyl)-1,2,3,4-tetrahydro-1-naphthalenamine hydrogen sulphate

To a vigorously stirred solution of trifluoroacetic acid (60ml) was added cis-(1S)-N-methyl-4-(3,4-dichlorophenyl)-1,2,3,4-tetrahydro-1-naphthalenamine hydrochloride (sertraline, 20g, 0.0584mol) in portions. To this colourless solution was carefully added chlorosulphonic acid (20ml, 0.292mol) and the mixture stirred at room temperature under nitrogen for 16 hours. A further portion of chlorosulphonic acid was then added (20ml) and the reaction stirred for 24 hours. The mixture was poured onto ice-water (1L), the solid filtered off and dried under suction. The white precipitate was dissolved in dichloromethane (700ml), dried (MgSO₄), filtered and the solvent removed under reduced pressure to give the subtitle compound (21.1g, 72%) as a white foam.

### (b) Cis-(1S)-N-methyl-7-sulphonamido-4-(3,4-dichlorophenyl)-1,2,3,4-tetrahydro-1-naphthalenamine

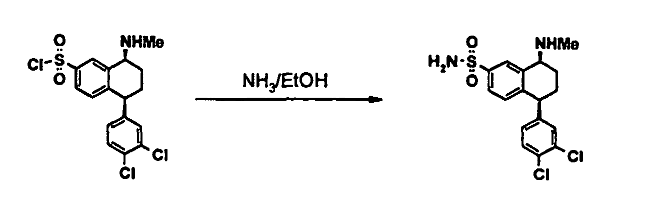

A solution of the sulphonyl chloride product of step (a) (0.38g) in dichloromethane (10ml) was added to a saturated ammoniacal ethanol solution (10ml) and the reaction allowed to stand at room temperature for 16 hours. The reaction was diluted with diethyl ether (20ml) and washed with water (3x20ml) and brine (20ml). The solution was dried (MgSO₄), filtered and the solvent removed under reduced pressure. The crude product purified on silica gel eluting with 95:5:0.5 dichloromethane:methanol:0.88 ammonia, the solvent removed under reduced pressure and the product then azeotroped with dichloromethane and diethyl ether to give the title compound (0.117g). MS *m*/*z* 385 (MH)⁺. ¹H-NMR (d₄-MeOH): δ = 1.93 (1H, m), 2.08 (3H, m), 2.50 (3H, s), 3.30 (1H, s), 3.82 (1H, m), 4.13 (1H, t), 6.96 (1H, d), 7.15 (1H, d), 7.38 (1H, s), 7.43 (1H, d), 7.65 (1H, d), 7.95 (1H, s).

### Example 8A

### Alternative preparation of cis-(1S)-N-methyl-7-sulphonamido-4-(3,4-dichlorophenyl)-1,2,3,4-tetrahydro-1-naphthalenamine

To a solution of cis-(1S)-N-methyl-4-(3,4-dichlorophenyl)-1,2,3,4-tetrahydro-1-naphthalenamine hydrochloride (375g, 1.09 mole) in dichloromethane (2875 ml) was added chlorosulphonic acid (728 ml, 10.9 mole) over about 30 minutes, maintaining the temperature below 20°C. Thionyl chloride (158 ml, 2.19 mole) was then added over about 5 minutes and the reaction allowed to stir at room temperature for 16 hours. The reaction mixture was slowly quenched into a solution of water (3125ml) and trifluoroacetic acid (232ml) over about 2 hours, maintaining the temperature below 20°C. The organic phase was then separated and concentrated to about 750 ml, under reduced pressure.

The above solution was added to a mixture of 0.88 ammonia (750 ml) in acetonitrile (2.5 l), maintaining the temperature below 10°C (ice-water bath cooling), and the resulting suspension allowed to warm to room temperature and stir for 16 hours. The solid was filtered off, washed with acetonitrile (375 ml) and dried under vacuum at 60°C for 16 hours to yield crude title compound (387g, 85%w/w, 78%). This product was added to methanol (1875 ml) and the suspension stirred at reflux for 16 hours, then cooled to ambient. The white precipitate was filtered off, washed with methanol (375 ml) and dried at 60°C under vacuum for 4 hours to give the title compound (246g, 58% from sertraline.HCl).

Using the procedure described in Example 8(b), the following sulphonamides were prepared by reaction of the sulphonyl chloride product of Example 8(a) and the appropriate amine:

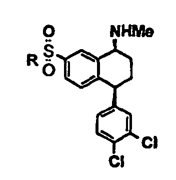

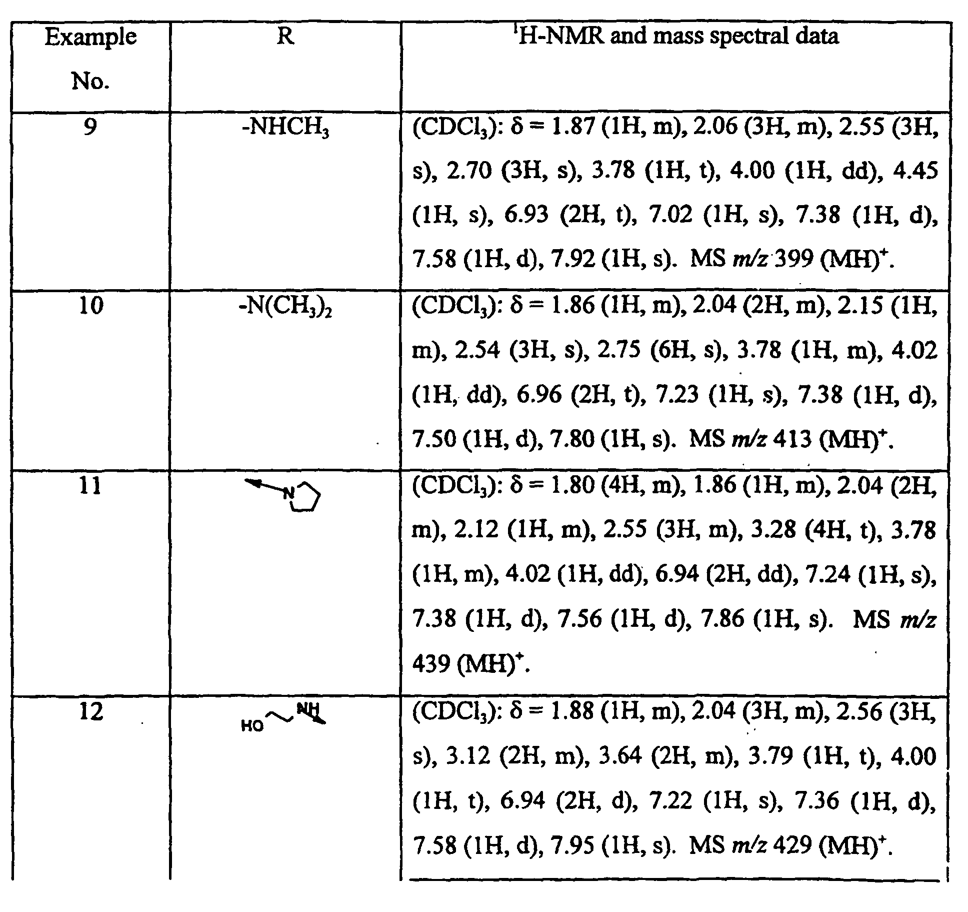

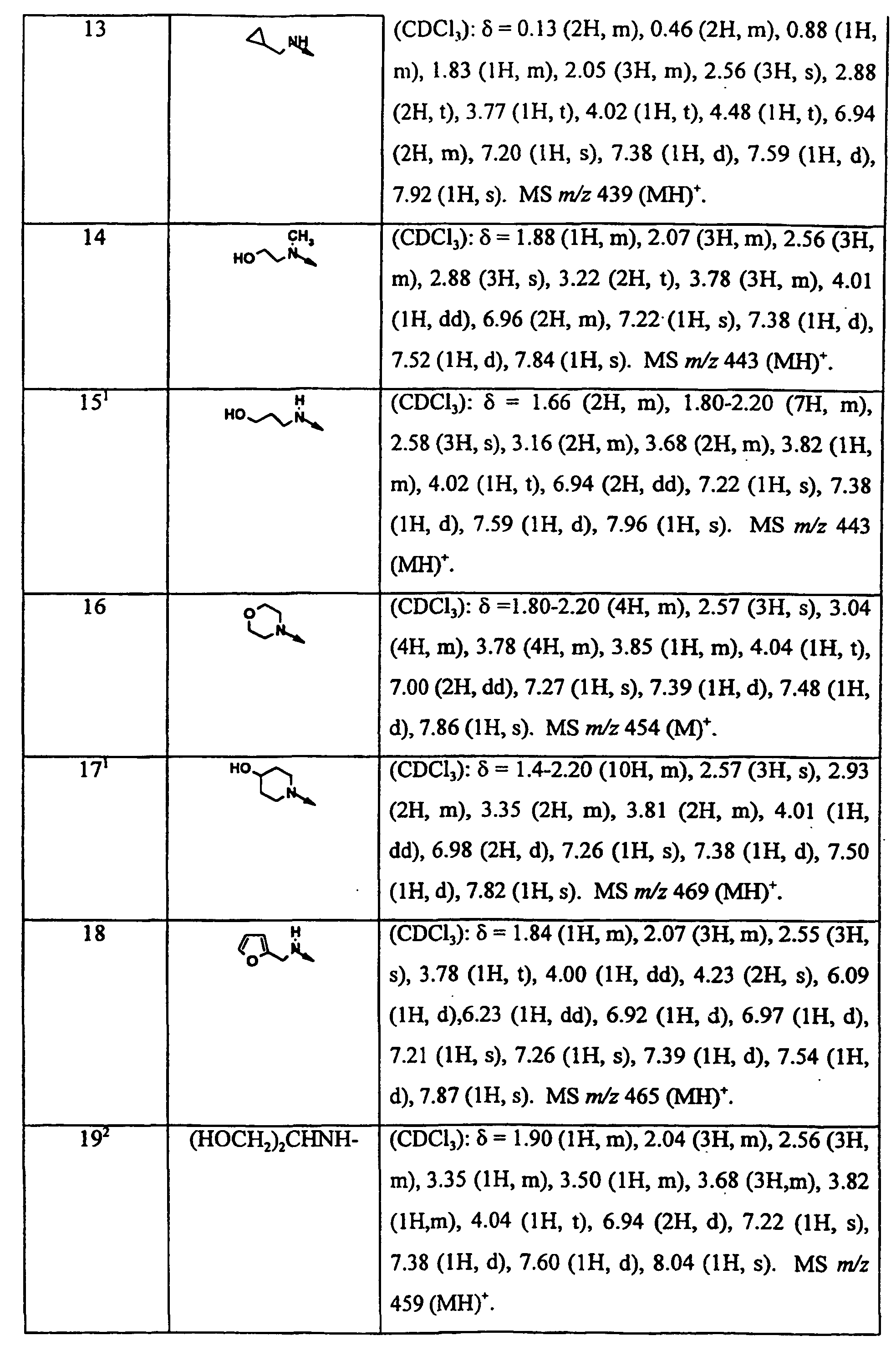

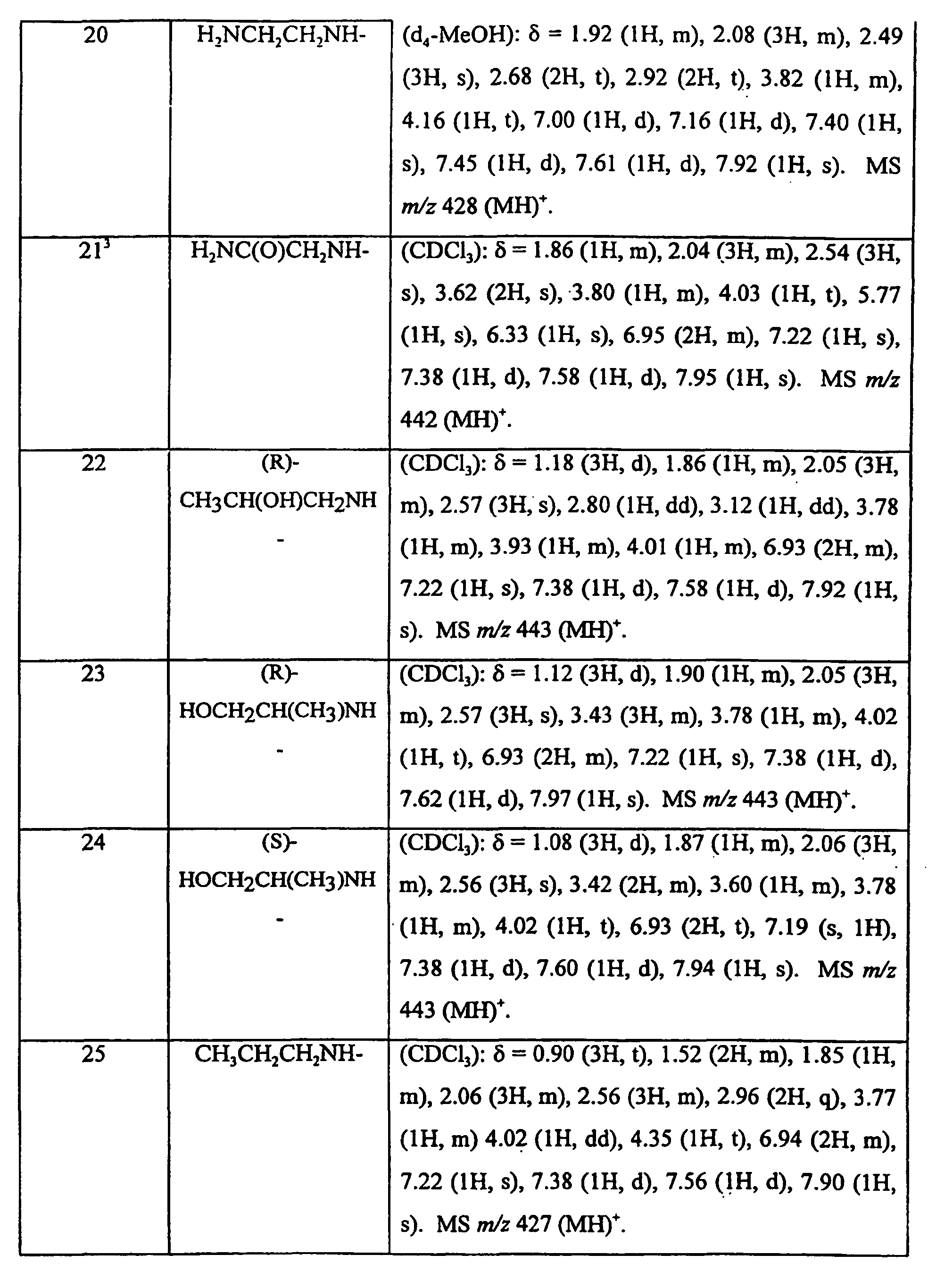

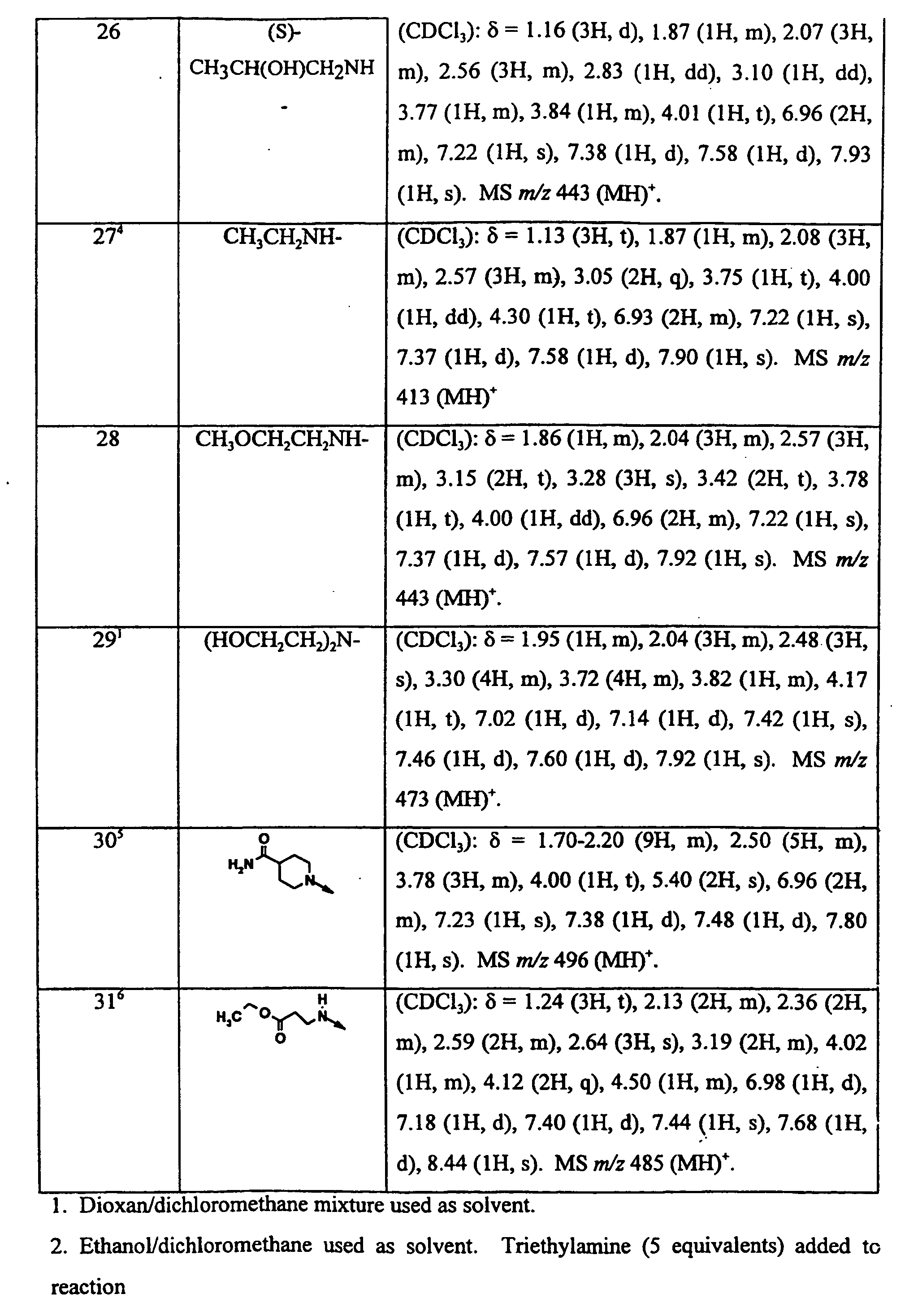

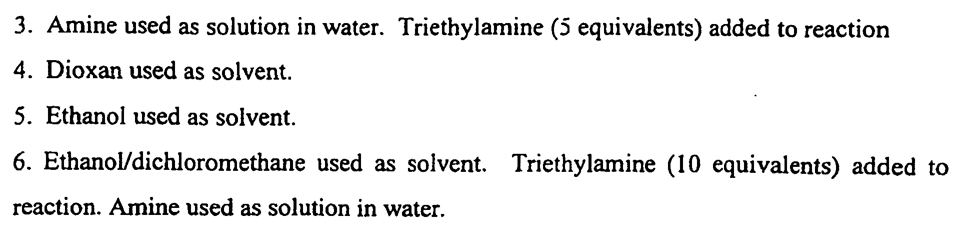

### Example 32

### Cis-(1S)-N-methyl-7-(2-carboxyethyl(sulphonamido))-4-(3,4-dichlorophenyl)-1,2,3,4-tetrahydro-1-naphthalenamine hydrochloride

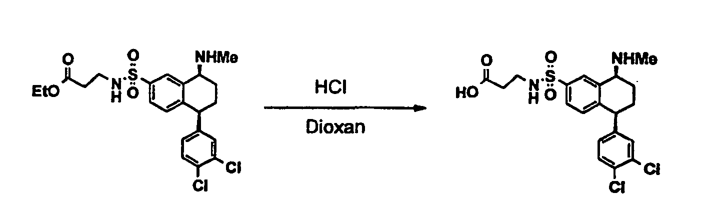

To a solution of the product of Example 31 (1.3g, 0.0027mol) in dioxan (20ml) was added 2N aqueous hydrochloric acid solution (5ml) and the reaction heated at 90°C for 2 hours. The reaction was cooled to room temperature, the solvent removed under reduced pressure and the residue azeotroped with toluene (x1) and dichloromethane (x5). Diethyl ether was added and the solution filtered. The solid was collected and dried to give the title compound (1.07g, 81%), as a white powder. MS *m*/*z* 457 (MH)⁺. ¹H-NMR (d₄-MeOH): δ = 2.08 (1H, m), 2.16-2.44 (3H, m), 2.52 (2H, t), 2.88 (3H, s), 3.17 (2H, t), 4.26 (1H, m), 4.63 (1H, s), 4.84 (4H, s), 7.14 (1H, d), 7.24 (1H, d), 7.48 (1H, s), 7.52 (1H, d), 7.79 (1H, d), 8.12 (1H, s).

### Example 33

### Cis-(1S)-N-methyl-7-carboxy-4-(3,4-dichlorophenyl)-1,2,3,4-tetrahydro-1-naphthalenamine

### (a) Cis-(1S)-N-methyl-7-(ethyoxycarbonyl)-4-(3,4-dichlorophenyl)-1,2,3,4-tetrahydro-1-naphthalenamine

The subtitle compound was prepared by an analogous procedure to that described in Example 6(a), but substituting ethanol for methanol.

### (b) Cis-(1S)-N-methyl-7-carboxy-4-(3,4-dichlorophenyl)-1,2,3,4-tetrahydro-1-naphthalenamine

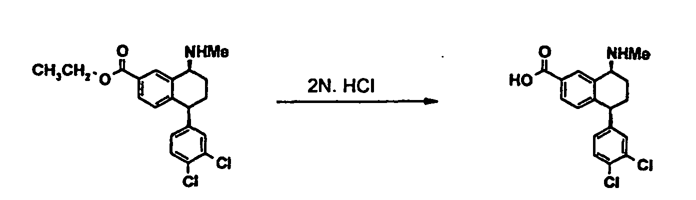

A solution of the ethyl ester from step (a) (0.14g, 0.00037mol) in dioxan (10ml) and 2N aqueous hydrochloric acid (3ml) was heated at reflux for 16 hours. The reaction was cooled to room temperature and the solvent removed under reduced pressure. The crude product was purified on silica gel eluting with a gradient of 80:20:3 to 80:20:5 dichloromethane:methanol:0.88 ammonia to give the title compound (0.121g, 93%). MS *m*/*z* 350 (MH)⁺. ¹H-NMR (d₆-DMSO): δ = 1.78-2.10 (4H, m), 2.53 (3H, s), 3.98 (1H, br), 4.13 (1H, m), 6.72 (1H, d), 7.20 (1H, d), 7.46 (1H, s), 7.55 (1H, d), 7.65 (1H, d), 8.38 (1H, s).

### Example 34

### Cis-(1S)-N-methyl-7-(methanesulphonamidomethyl)-4-(3,4-dichlorophenyl)-1,2,3,4-tetrahydro-1-naphthalenamine

### (a) Cis-(1S)-N-methyl-7-(aminomethyl)-4-(3,4-dichlorophenyl)-1,2,3,4-tetrahydro-1-naphthalenamine hydrochloride

A solution of the nitrile product of Example 1(c) (1.22g, 0.0037mol) in tetrahydrofuran (50ml) was added to a stirred suspension of lithium aluminium hydride (0.42g, 0.011mol) in tetrahydrofuran (50ml) at 0°C. After 1 hour, the reaction was heated to 50°C for 3 hours, cooled in an ice-bath and quenched with 2N aqueous sodium hydroxide solution (5ml). The reaction was dried (MgSO₄), filtered and the solvent removed under reduced pressure. The crude product was purified on silica gel eluting with 93:7:1 dichloromethane:methanol:0.88 ammonia and the solvent removed under reduced pressure to give a gum which was azeotroped with dichloromethane (3x10ml). The product was treated with a saturated solution of hydrogen chloride gas in diethyl ether (5ml) and the solvent removed under reduced pressure to give the subtitle compound as a white solid (0.96g). MS *m*/*z* 335 (MH)⁺. ¹H-NMR (d₄-MeOH): δ = 1.94-2.38 (4H, m), 2.85 (3H, s), 4.16 (2H, s), 4.19 (1H, dd), 4.47 (1H, t), 7.00 (1H, d), 7.22 (1H, d), 7.41 (2H, m), 7.50 (1H, d), 7.72 (1H, s).

### (b) Cis-(1S)-N-methyl-7-(methanesulphonamidomethyl)-4-(3,4-dichlorophenyl)-1,2,3,4-tetrahydro-1-naphthalenamine

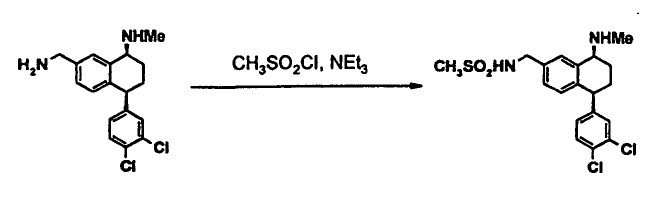

A solution of the amine product of step (a) (0.27g, 0.00066mol) in dichloromethane (20ml) was cooled in an ice-water bath and to this was added a solution of triethylamine (0.234g, 0.0023mol) in dichloromethane (10ml) followed by a solution of methanesulphonyl chloride (0.076g, 0.00066mol) in dichloromethane (20ml), added over 20 minutes. After 2 hours, the solvent was removed under reduced pressure and the crude product purified on silica gel eluting with 93:7:1 dichloromethane:methanol:0.88 ammonia to give the title compound as a white foam (0.155g, 57%). MS *m*/*z* 413 (MH)⁺. ¹H-NMR (CDCl₃): δ = 1.82 (1H, m), 2.02 (3H, m), 2.53 (3H, m), 2.93 (3H, s), 3.63 (1H, t), 3.97 (1H, t), 4.30 (2H, s), 6.80 (1H, d), 6.96 (1H, d), 7.09 (1H, d), 7.22 (1H, s), 7.36 (2H, m).

### Example 35

### Cis-(1S)-N-methyl-7-((N-acetyl)aminomethyl)-4-(3,4-dichlorophenyl)-1,2,3,4-tetrahydro-1-naphthalenamine

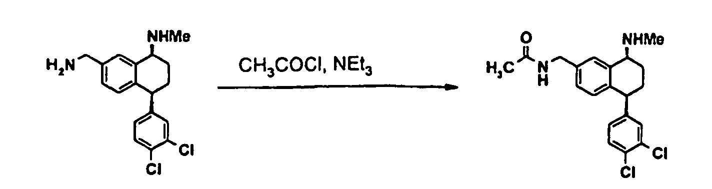

A solution of the amine product of Example 34(a) (0.15g, 0.00037mol) in dichloromethane (15ml) was cooled in an ice-water bath and to this was added triethylamine (0.123g, 0.0012mol). After 5 minutes, acetyl chloride (0.031g, 0.00039mol) was added. The reaction was allowed to warm to room temperature and stirred for 68 hours. Dichloromethane (20ml) was added and the reaction washed with water (20ml), dried (MgSO₄), filtered and the solvent removed under reduced pressure. The crude product was purified on silica gel eluting with 95:5:0.5 dichloromethane:methanol:0.88 ammonia to give the title compound as a white foam (0.042g, 30%). MS *m*/*z* 377 (MH)⁺. ¹H-NMR (d₄-MeOH): δ = 1.97 (1H, m), 1.99 (3H, s), 2.22 (3H, m), 2.84 (3H, s), 4.15 (1H, dd), 4.36 (2H, s), 4.45 (1H, br), 6.86 (1H, d), 7.18 (1H, dd), 7.24 (1H, d), 7.41 (1H, d), 7.46 (1h, d), 7.49 (1H, s).

### Example 36

### Cis-(1S)-N-methyl-7-((N-aminosulphonyl)aminomethyl)-4-(3,4-dichlorophenyl)-1,2,3,4-tetrahydro-1-naphthalenamine

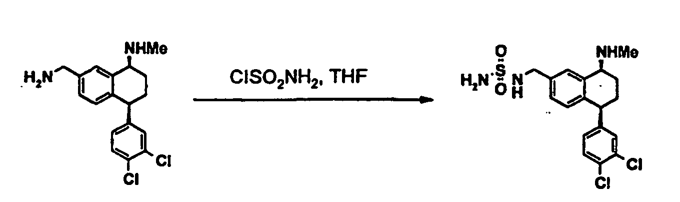

To a solution of the amine product of Example 34(a) (0.155g, 0.00046mol) in tetrahydrofuran (10ml) was added sulphamoyl chloride (Chem. Abstract., 1958, 52, 19655f) (0.051g, 0.00044mol) and the mixture heated at reflux for 7 hours. The reaction was cooled, poured into 10% aqueous potassium carbonate solution (30ml) and extracted with ethyl acetate (30ml). The organic phase was washed with brine (30ml), dried (MgSO₄), filtered and the solvent removed under reduced pressure. The crude product was purified on silica gel eluting with 93:7:1 dichloromethane:methanol:0.88 ammonia to give the title compound as a white foam (0.055g, 29%). MS *m*/*z* 414 (MH)⁺. ¹H-NMR (d₄-MeOH): δ = 1.83 (1H, m), 1.92 (1H, m), 2.03 (1H, m), 2.25 (1H, m), 2.63 (3H, s), 4.22 (3H, s), 5.10 (1H, dd), 6.95 (2H, m), 7.10 (1H, s), 7.25 (1H, d), 7.41 (1H, d), 7.59 (1H, s).

### Example 37

### Cis-(1S)-N-methyl-7-((N-aminocarboxy)aminomethyl)-4-(3,4-dichlorophenyl)-1,2,3,4-tetrahydro-1-naphthalenamine hydrochloride

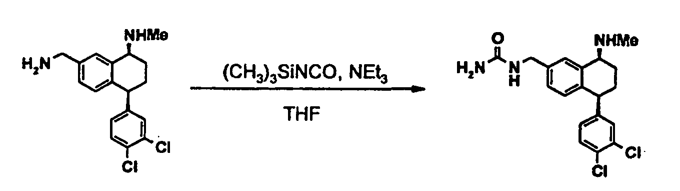

To a suspension of the amine product of Example 34(a) (0.251g, 0.00061mol) in tetrahydrofuran (10ml) was added triethylamine (175µl). After 5 minutes, the reaction was cooled in an ice-bath, trimethylsilyl isocyanate (80µl) added and the reaction allowed to stir for 15 minutes. The reaction was then stirred at room temperature for 16 hours. Water (1ml) was added, the reaction stirred for 5 minutes and then partitioned between ethyl acetate (25ml) and 10% aqueous potassium carbonate solution (25ml). The aqueous layer was extracted with ethyl acetate and the combined organic layers washed with brine (50ml), dried (MgSO₄), filtered and the solvent removed under reduced pressure. The residue was then dissolved in hot ethyl acetate, filtered and the solvent removed under reduced pressure. The crude product was purified on silica gel eluting with 93:7:1 dichloromethane:methanol:0.88 ammonia and the solvent removed under reduced pressure. The material obtained was then purified on silica gel eluting with a gradient of 96:4 to 94:6 ethyl acetate:diethylamine and the solvent removed under reduced pressure. This material was then purified on silica gel eluting with 93:7:1 dichloromethane:methanol:0.88 ammonia. The solvent was removed under reduced pressure and the residue treated with a saturated solution of hydrogen chloride in diethyl ether (5ml). The solvent was removed under reduced pressure, the residue dissolved in methanol (5ml) and the solvent removed *in vacuo* to give the title compound (0.055g, 24%). MS *m*/*z* 378 (MH)⁺. ¹H-NMR (d₄-MeOH): δ = 1.97 (1H, m), 2.19 (3H, m), 2.80 (3H, s), 4.14 (1H, dd), 4.28 (2H, s), 4.37 (1H, br), 6.86 (1H, d), 7.19 (1H, d), 7.22 (1H, d), 7.40 (2H, m), 7.47 (1H, d).

### Example 38

### Cis-(1S)-N-methyl-7-(3-(5-methyl-1,2,4-oxadiazolyl))-4-(3,4-dichlorophenyl)-1,2,3,4-tetrahydro-1-naphthalenamine

### (a) Cis-(1S)-N-methyl-7-(aminooximyl)-4-(3,4-dichlorophenyl)-1,2,3,4-tetrahydro-1-naphthalenamine

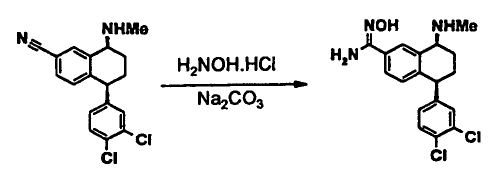

To a solution of hydroxylamine hydrochloride (1.67g, 0.024mol) in water (30ml) was added sodium carbonate (2.54g, 0.024mol). This solution was then added to a solution of the nitrile product of Example 1(c) (1.06g, 0.0032mol) in methanol (60ml) and the reaction heated under reflux under an atmosphere of nitrogen for 16 hours. The reaction was cooled, partitioned between dichloromethane (50ml) and water (50xnl) and the aqueous phase extracted with dichloromethane (2x50ml). The combined organics were dried (MgSO₄), filtered, the solvent removed under reduced pressure and the residue triturated with diethyl ether and dried to give the subtitle compound (1.1g) which was used without further purification. MS *m*/*z* 366 (MH)⁺. ¹H-NMR (CDCl₃): δ = 1.85 (1H, m), 2.05 (3H, m), 2.54 (3H, m), 3.76 (1H, m), 3.99 (1H, m), 4.91 (1H, s), 6.79-6.90 (1H, m), 6.95 (1H, d), 7.22 (1H, d), 7.35 (1H, d), 7.40-7.57 (1H, m), 7.66 (0.5H, s), 7.93 (0.5H, s).

### (b) Cis-(1S)-N-methyl-7-(3-(5-methyl-1,2,4-oxadiazolyl))-4-(3,4-dichlorophenyl)-1,2,3,4-tetrahydro-1-naphthalenamine

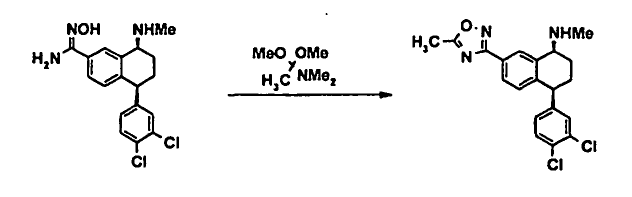

The amidoxime product of step (a) (0.4g, 0.0011mol) and N,N-dimethylacetamide dimethylacetal (2ml) were heated at reflux for 4 hours. The reaction was cooled and the excess N,N-dimethylacetamide dimethylacetal removed *in vacuo*. This material was then purified on silica gel eluting with a gradient of 97:3:0 dichloromethane:methanol:0.88 ammonia to 97:3:1 dichloromethane : methanol : 0.88 ammonia. The product was collected and the solvent removed under reduced pressure. This was purified further on silica gel eluting with 95:5:0.5 ethyl acetate : methanol : 0.88 ammonia to give the title compound, after trituration with diethyl ether, as a colourless foam (0.05g, 12%). MS *m*/*z* 388 (MH)⁺. ¹H-NMR (CDCl₃): δ = 1.78 (2H, m), 2.00 (1H, m), 2.40 (1H, m), 2.53 (3H, s), 2.66 (3H, s), 3.94 (1H, t), 4.18 (1H, t), 6.86 (1H, d), 6.96 (1H, d), 7.13 (1H, s), 7.33 (1H, d), 8.01 (1H, d), 8.17 (1H, s).

### Example 39

### Cis-(1S)-N-methyl-7-(5-(3-methyl-1,2,4-oxadiazolyl))-4-(3,4-dichlorophenyl)-1,2,3,4-tetrahydro-1-naphthalenamine

### (a) N-Hydroxyethanimidamide

To a solution of hydroxylamine hydrochloride (35g, 0.5mol) in ethanol (200ml) was added phenolphthalein (0.05g). Sodium ethoxide solution (324ml, 21%w/v) was added over 1 hour. After 3 hours, acetonitrile (13.8g) was added, the reaction stirred at room temperature for 2 hours and then heated at 40°C for 48 hours. The reaction was cooled to room temperature, filtered and the solvent removed under reduced pressure. The residue was allowed to stand at room temperature for 48 hours, methanol (1 litre) added and the crude product absorbed on silica. The product was purified on a silica column eluting with 9 : 1 dichloromethane : methanol to give the subtitle compound, (20.33g, 81%). ¹H NMR (d₆-DMSO): δ = 1.60 (3H, s), 5.33 (2H, br), 8.65 (1H, s).

### (b) Cis-(1S)-N-methyl-7-(5-(3-methyl-1,2,4-oxadiazolyl))-4-(3,4-dichlorophenyl)-1,2,3,4-tetrahydro-1-naphthalenamine

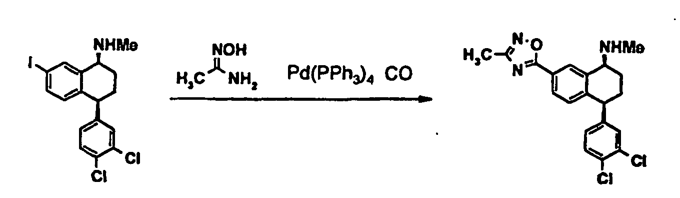

A solution of the iodide from Example 1(a) (0.3g, 0.00069mol), the compound of step (a) (0.205g, 5equiv), tetrakis(triphenylphosphine)palladium (0.40g) and triethylamine (0.24ml) in toluene (5ml) was heated at reflux under an atmosphere of carbon monoxide for 16 hours. The reaction was cooled to room temperature, concentrated under reduced pressure and partitioned between ethyl acetate (15ml) and water (15ml). The aqueous phase was extracted with ethyl acetate (15ml) and the combined organics dried (MgSO₄), filtered and the solvent removed under reduced pressure. The crude product was purified on silica gel eluting with 95:5:0.5 dichloromethane : methanol : 0.88 ammonia. The solvent was removed under reduced pressure and the compound purified further by chromatography on silica gel, eluting with a solvent gradient of 98:2 dichloromethane : methanol to 95:5 dichloromethane : methanol. The solvent was removed under reduced pressure and the compound finally purified on silica gel eluting with 95:5:0.5 ethyl acetate : methanol : 0.88 ammonia to give the title compound as a colourless solid after trituration with diethyl ether (0.083g, 31%). MS *m*/*z* 388 (MH)⁺. ¹H-NMR (CDCl₃): δ = 1.87 (1H, m), 2.09 (3H, m), 2.47 (3H, m), 2.57 (3H, m), 3.80 (1H, t), 4.03 (1H, dd), 6.98 (2H, m), 7.26 (1H, s), 7.37 (1H, d), 7.83 (1H, d), 8.14 (1H, s).

### Examples 40 and 41

### Cis-(1S)-N-methyl-7-(N(2)-1,2,3-triazolyl)-4-(3,4-dichlorophenyl)-1,2,3,4-tetrahydro-1-naphthalenamine hydrochloride and Cis-(1S)-N-methyl-7-(N(1)-1,2,3-triazolyl)-4-(3,4-dichlorophenyl)-1,2,3,4-tetrahydro-1-naphthalenamine hydrochloride

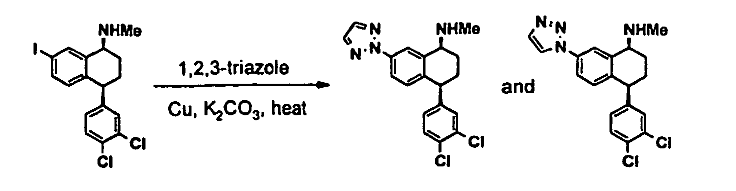

A mixture of the iodide from Example 1(a) (0.432g, 0.001mol), 1,2,3-triazole (1.6g), copper powder (0.064g) and potassium carbonate (0.138g) was heated at 160°C for 7.5 hours. The reaction was cooled to room temperature and partitioned between saturated aqueous ethyldiamine tetra-acetic acid disodium salt solution and ethyl acetate (60ml). The organic phase was washed with brine (3x60ml), dried (MgSO₄) and the solvent removed under reduced pressure to give a gum. This material was then purified on silica gel eluting with a gradient of 100:0 dichloromethane : methanol to 95:5 dichloromethane : methanol then 95:5:0.5 dichloromethane : methanol : 0.88 ammonia. The appropriate fractions containing the N-2 linked triazole were concentrated to dryness under reduced pressure, the residue taken up in dichloromethane (5ml) and a saturated solution of hydrogen chloride in diethyl ether (2ml) added. The solvent was removed under reduced pressure and the residue triturated with diethyl ether (2x5ml). The solid was filtered and dried at 75°C for 4h to give the first title compound, cis-(1S)-N-methyl-7-(N(2)-1,2,3-triazolyl)-4-(3,4-dichlorophenyl)-1,2,3,4-tetrahydro-1-naphthalenamine hydrochloride (Example 40) as a white solid (35mg). MS *m*/*z* 373 (MH)⁺. ¹H-NMR (d₆-DMSO): δ = 2.05 (3H, m), 3.30 (1H, m), 3.70 (3H, s), 4.20 (1H, m), 4.55 (1H, s), 6.95 (1H, d), 7.40 (1H, d), 7.65 (2H, dd), 7.95 (1H, d), 8.15 (2H, s), 8.3 (1H, s), 9.30 (2H, br. s). This product was followed off the column by the N-1 linked isomer. The solvent was removed under reduced pressure and the residue taken up in dichloromethane (5ml) and a saturated solution of hydrogen chloride in diethyl ether (2ml) added. The solvent was removed under reduced pressure and the residue triturated with diethyl ether (2x5ml). The solid was filtered and dried at 75°C for 4h to give the second title compound, cis-(1S)-N-methyl-7-(N(1)-1,2,3-triazolyl)-4-(3,4-dichlorophenyl)-1,2,3,4-tetrahydro-1-naphthalenamine hydrochloride (Example 41) as a white powder (57mg). MS *m*/*z* 373 (MH)⁺. ¹H-NMR (d₆-DMSO): δ = 2.05 (3H, m), 2.21 (1H, m), 2.7 (3H, m), 4.25 (1H, m), 4.55 (1H, s), 7.0 (1H, d), 7.32 (1H, d), 7.60 (2H, m), 7.8 (1H, dd), 8.00 (1H, s), 8.18 (1H, s), 8.85 (1H, s), 9.30 (1H, br), 9.55 (1H, br).

Using the general procedure described in the preparation of Examples 40 and 41, the following N-linked heterocycles were prepared by reaction of the iodide of Example 1(a) and the appropriate heterocycle:

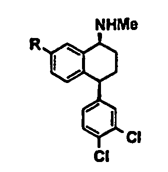

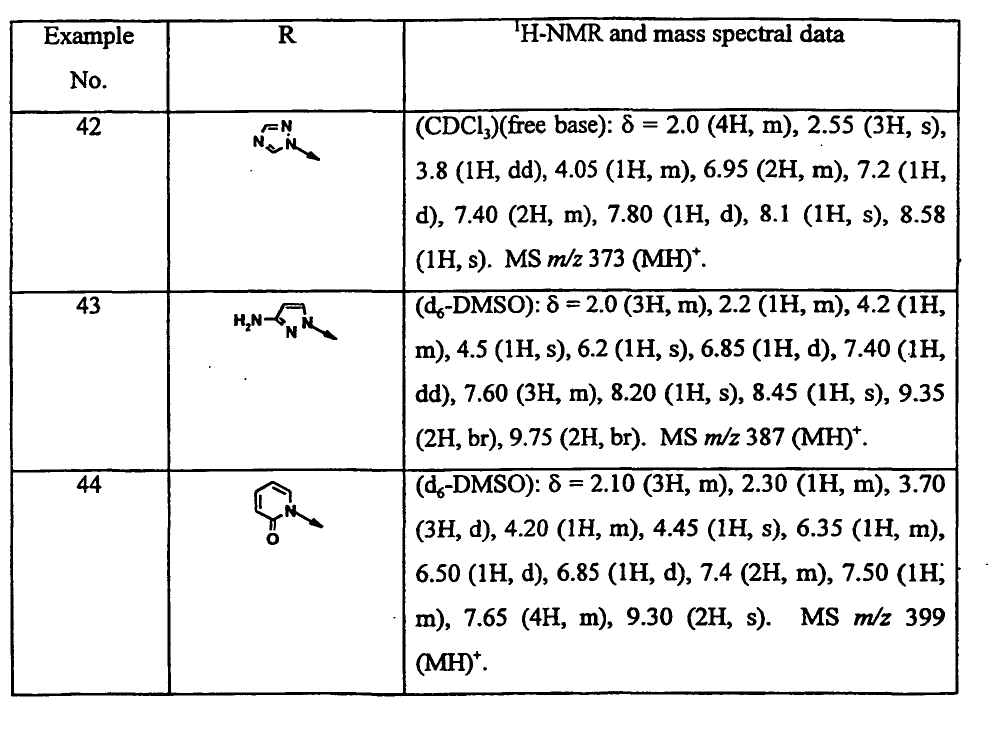

### Example 45

### Cis-(1S)-N-methyl-7-(5-(2-amino)pyridyl)-4-(3,4-dichlorophenyl)-1,2,3,4-tetrahydro-1-naphthalenamine hydrochloride

### (a) Cis-(1S)-N-methyl-7-(boronato)-4-(3,4-dichlorophenyl)-1,2,3,4-tetrahydro-1-naphthalenamine

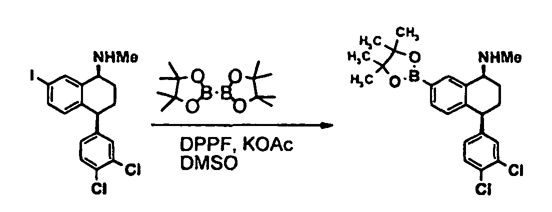

A solution of the iodide product from Example 1(a) (4.64g, 0.0107mol), bis(pinacolato)diboron (3g), DPPF [1,1'-bis(diphenylphosphino)ferrocene, available from Lancaster Synthesis Limited] (0.52g) and potassium acetate (3.16g) in dimethylsulphoxide (70ml) was heated at 70°C for 55 minutes. The reaction was cooled, ethyl acetate added and the mixture washed with water. The organic phase was dried (MgSO₄), filtered and the solvent removed under reduced pressure to give the subtitle compound which was used without further purification.

### (b) Cis-(1S)-N-methyl-7-(5-(2-amino)pyridyl)-4-(3,4-dichlorophenyl)-1,2,3,4-tetrahydro-1-naphthalenamine hydrochloride

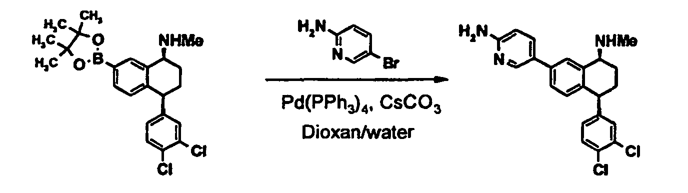

A solution of the boronate ester from step (a) (0.432g, 0.001mol), 2-amino-5-bromopyridine (0.173g), tetrakis(triphenylphosphine)palladium (0.115g) and caesium carbonate (0.65g) in dioxan (12ml) and water (4ml) was heated at 80°C for 4.5 hours. The reaction was cooled and the solvent removed under reduced pressure. Water (30ml) was added and the aqueous phase extracted with ethyl acetate (3x20ml). The combined organics were washed with brine (50ml), dried (Na₂SO₄), filtered and the solvent removed under reduced pressure. The crude product was purified on silica gel eluting with 93:7:1 dichloromethane : methanol : 0.88 ammonia. The solvent was removed under reduced pressure, dissolved in ethyl acetate (5ml) and a saturated solution of hydrogen chloride in diethyl ether (2ml) added. The solvent was removed under reduced pressure to give the title compound (0.04g). MS *m*/*z* 398 (MH)⁺. ¹H-NMR (CDCl₃): δ = 1.82 (1H, m), 2.02 (3H, m), 2.55 (3H, s), 3.66 (1H, t), 4.00 (1H, t), 4.48 (1H, s), 6.58 (1H, d), 6.82 (1H, d), 7.00 (1H, d), 7.25 (2H, m), 7.50 (1H, s), 7.65 (1H, dd), 8.30 (1H, s).

Using the general procedure described in the preparation of Example 45, the following compounds were prepared by reaction of the boronate ester from step (a) and the appropriate bromo- or iodo-heterocycle:

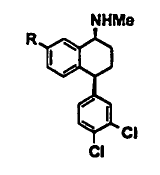

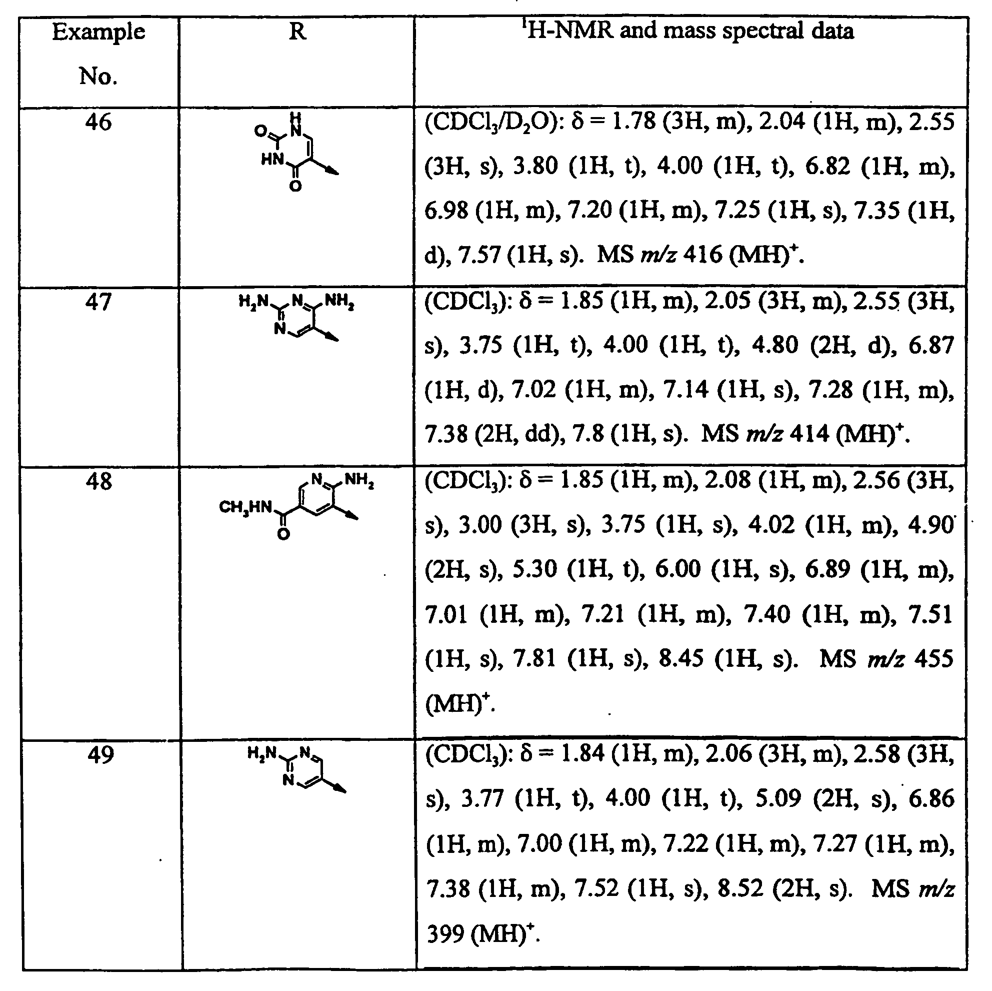

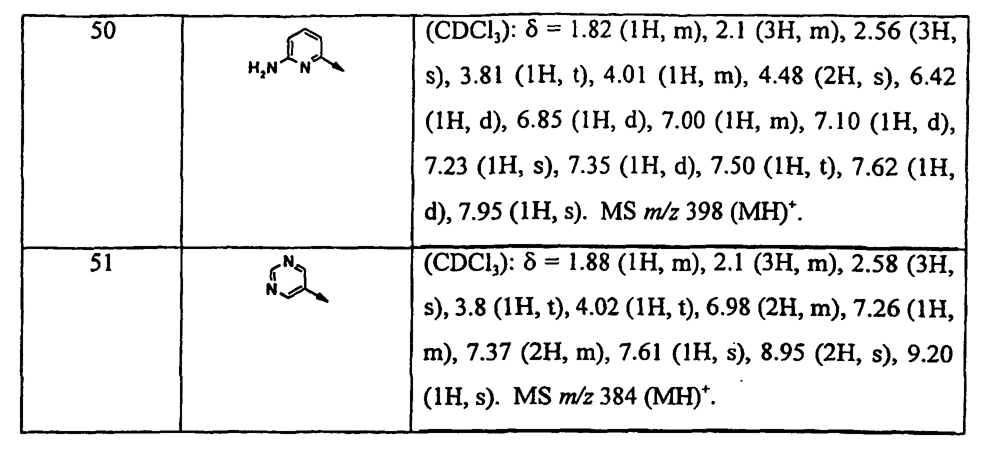

### Example 52

### Cis-(1S)-N-methyl-7-(4-pyrazolyl)-4-(3,4-dichlorophenyl)-1,2,3,4-tetrahydro-1-naphthalenamine

### (a) N-1-Ethoxymethyl-4-iodo-pyrazole

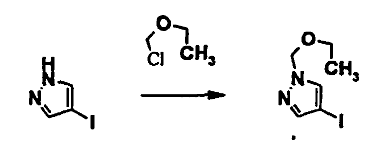

To a solution of 4-iodopyrazole (5g, 0.026mol) in acetone (40ml) was added potassium carbonate (3.92g, 0.0284mol) and the mixture cooled in an ice-water bath. Chloromethylethylether (2.66ml, 0.0284mol) was added and the reaction stirred for 16 hours. Water (100ml) was added, the mixture extracted with dichloromethane (100ml), the organics dried (MgSO₄), filtered and the solvent removed under reduced pressure. The crude product was purified on silica gel eluting with 9:1 pentane : ethyl acetate to give the subtitle compound (5.77g, 85%). MS *m*/*z* 253 (MH)⁺. ¹H-NMR (CDCl₃): δ = 1.18 (3H, t), 3.50 (2H, q), 5.42 (2H, s), 7.54 (1H, s), 7.65 (1H, s).

### (b) Cis-(1S)-N-methyl-7-(4-(N(1)-ethoxymethyl)pyrazolyl)-4-(3,4-dichlorophenyl)-1,2,3,4-tetrahydro-1-naphthalenamine

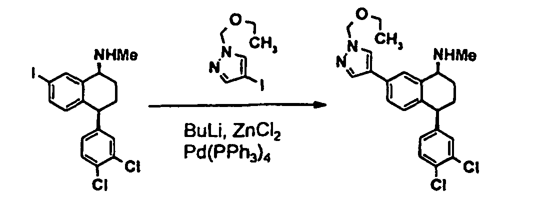

To a solution of the iodopyrazole product of step (a) (0.635g, 0.00276mol) in diethyl ether (10ml) at -95°C under nitrogen was added n-butyllithium (2.5M solution in hexane) (0.00276mol) and after 10 minutes, zinc chloride (0.00276mol) (0.5M solution in tetrahydrofuran). After 40 minutes, tetrakis(triphenylphosphine)palladium (0.106g) and a solution of the iodide product of Example 1(a) (0.4g, 0.00092mol) in diethyl ether (5ml) were added and the reaction heated at reflux for 16 hours. The reaction was cooled to room temperature, washed with saturated aqueous ethyldiamine tetra-acetic acid di-sodium salt solution and filtered. The aqueous phase was extracted with dichloromethane (50ml) and the combined organics washed with brine (100ml), dried (MgSO₄), filtered and the solvent removed under reduced pressure. The crude product was purified on silica gel eluting with 10:10:0.5 pentane : ethyl acetate : diethylamine to give the subtitle compound (0.184 g, 47%). MS *m*/*z* 430 (MH)⁺. ¹H-NMR (CDCl₃): δ = 1.18 (3H, t), 1.85 (1H, m), 2.04 (3H, m), 2.58 (3H, s), 3.58 (2H, q), 3.75 (1H, t), 3.99 (1H, q), 5.46 (2H, s), 6.80 (1H, d), 7.00 (1H, dd), 7.20 (1H, s), 7.36 (1H, d), 7.48 (2H, m), 7.68 (1H, dd), 7.84 (1H, s).

### (c) Cis-(1S)-N-methyl-7-(4-pyrazolyl)-4-(3,4-dichlorophenyl)-1,2,3,4-tetrahydro-1-naphthalenamine

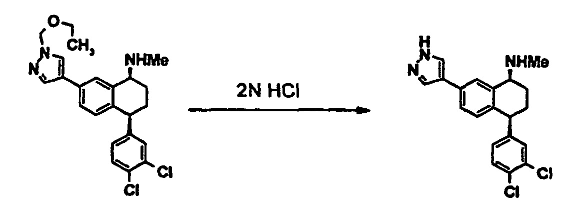

To a solution of the pyrazole product from step (b) (0.184g, 0.00043mol) in ethanol (8ml) was added aqueous 2N HCl solution (5ml) and the reaction heated at reflux for 16 hours. The reaction was cooled to room temperature and partitioned between saturated aqueous sodium carbonate and ethyl acetate. The organic layer was dried (MgSO₄), filtered and the solvent removed under reduced pressure to give the crude product which was purified on silica gel to give the title compound (0.074g, 46%). MS *m*/*z* 372 (MH)⁺. ¹H-NMR (CDCl₃): δ = 1.83 (1H, m), 2.02 (3H, m), 2.58 (3H, s), 3.54 (1H, t), 3.99 (1H, q), 6.80 (1H, d), 7.00 (1H, d), 7.24 (2H, m), 7.34 (1H, d), 7.52 (1H, s), 7.83 (2H, s).

### Example 53

### Cis-(1S)-N-methyl-7-(3-pyrazolyl)-4-(3,4-dichlorophenyl)-1,2,3,4-tetrahydro-1-naphthalenamine hydrochloride

### (a) Cis-(1S)-N-methyl-7-(5-(N(1)-ethoxymethyl)pyrazolyl)-4-(3,4-dichlorophenyl)-1,2,3,4-tetrahydro-1-naphthalenamine

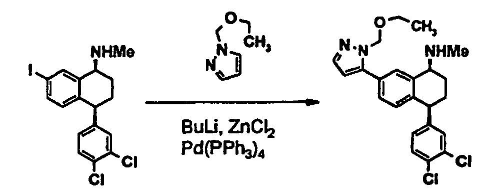

To a solution of N-ethoxymethylpyrazole (prepared using the method described in Example 52(a), but starting with pyrazole) (0.175g, 0.00139mol) in tetrahydrofuran (10ml) at -78°C under nitrogen was added n-butyllithium (2.5M solution in hexane) (0.00139mol) and after 10 minutes, zinc chloride (0.00139mol) (0.5M solution in tetrahydrofuran). After 20 minutes, the reaction was warmed to room temperature and tetrakis(triphenylphosphine) palladium (0.053g) and the iodide from Example 1(a) (0.2g, 0.00046mol) added and the reaction heated at reflux for 16 hours. The reaction was cooled to room temperature, washed with saturated aqueous ethyldiamine tetra-acetic acid di-sodium salt solution and filtered. The aqueous phase was extracted with dichloromethane (50ml) and the organic phase washed with brine (100ml), dried (MgSO₄), filtered and the solvent removed under reduced pressure. The crude product was purified on silica gel eluting with 80 : 20 : 5 pentane : ethyl acetate : diethylamine to give the subtitle compound (0.154 g, 78%). ¹H-NMR (CDCl₃): δ = 0.90 (3H, t), 1.29 (3H, m), 1.88 (1H, m), 2.04 (3H, m), 3.74 (3H, m), 4.01 (1H, m), 5.44 (2H, s), 6.40 (1H, s), 6.98 (1H, d), 7.02 (1H, d), 7.36 (1H, d), 7.40 (1H, d), 7.54 (1H, d), 7.62 (1H, d).

### (b) Cis-(1S)-N-methyl-7-(3-pyrazolyl)-4-(3,4-dichlorophenyl)-1,2,3,4-tetrahydro-1-naphthalenamine hydrochloride

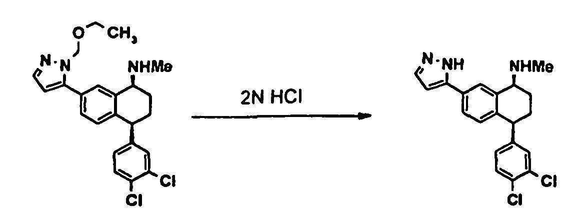

To a solution of the pyrazole from step (a) (0.15g, 0.00038mol) in ethanol (10ml) was added 2N aqueous hydrochloric acid (2ml) and the reaction heated at reflux for 48 hours. The reaction was cooled, the aqueous layer decanted off and the remaining material dissolved in ethanol and filtered. The solvent was removed under reduced pressure and the crude product purified on silica gel eluting with 80 : 20 : 5 pentane : ethyl acetate : diethylamine to give the title compound (0.045 g, 32%). The product was dissolved in dichloromethane (2ml) and a saturated solution of hydrogen chloride in diethyl ether added. The solvent was removed *in vacuo* to yield the hydrochloride salt. MS *m*/*z* 372 (MH)⁺. ¹H-NMR (d₄-MeOH): δ = 1.30 (1H, s), 2.01 (1H, m), 2.28 (3H, m), 2.90 (3H, s), 3.30 (1H, s), 4.22 (1H, m), 4.59 (1H, br), 6.98 (1H, br), 7.04 (1H, dd), 7.24 (1H, dd), 7.49 (1H, d), 7.52 (1H, d), 7.76 (1H, d), 8.00 (1H, s), 8.04 (1H, s).

### Example 54

### Cis-(1S)-N-methyl-7-(3-(N(2)-methyl)pyrazolyl)-4-(3,4-dichlorophenyl)-1,2,3,4-tetrahydro-1-naphthalenamine

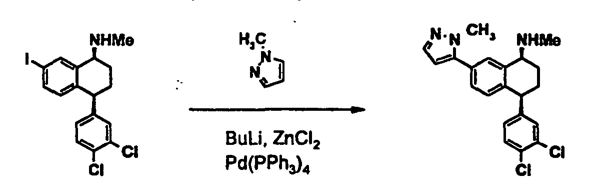

To a solution of N-methylpyrazole (0.114g, 0.00139mol) in tetrahydrofuran (5ml) at -78°C under nitrogen was added n-butyllithium (2.5M solution in hexane) (0.00139mol) and after 10 minutes, zinc chloride (0.00139mol) (0.5M solution in tetrahydrofuran). After 45 minutes, the reaction was warmed to 0°C and tetrakis(triphenylphosphine)palladium (0.053g) and a solution of the iodide product of Example 1(a) (0.2g, 0.00046mol) in tetrahydrofuran (5ml) added and the reaction heated at reflux for 16 hours. The reaction was cooled to room temperature, washed with saturated aqueous ethyldiamine tetra-acetic acid di-sodium salt solution and filtered. The aqueous phase was extracted with dichloromethane (50ml) and the combined organics washed with brine (100ml), dried (MgSO₄), filtered and the solvent removed under reduced pressure. The crude product was purified on silica gel to give the title compound (0.015g). MS *m*/*z* 386 (MH)⁺. ¹H-NMR (CDCl₃): δ = 1.88 (1H, m), 2.08 (3H, m), 2.57 (2H, s), 3.78 (1H, t), 3.90 (3H, s), 4.01 (1, m), 6.30 (1H, d), 6.88 (1H, d), 7.00 (1H, d), 7.17 (1H, d), 7.28 (1H, d), 7.3, (1H, d), 7.42 (1H, d), 7.51 (1H, d).

### Example 55

### Cis-(1S)-N-methyl-7-(3-(1,2,4-triazolyl))-4-(3,4-dichlorophenyl)-1,2,3,4-tetrahydro-1-naphthalenamine hydrochloride

### (a) Cis-(1S)-N-methyl-7-(3-((2-ethoxymethyl)-1,2,4-triazolyl))-4-(3,4-dichlorophenyl)-1,2,3,4-tetrahydro-1-naphthalenamine

To a solution of N(2)-ethoxymethyl-1,2,4-triazole (prepared by the method described in Example 52(a), but starting with 1,2,4-triazole) (0.176g, 0.00139mol) in tetrahydrofuran (10ml) at -78°C under nitrogen was added n-butyllithium (2.5M solution in hexane) (0.00139mol) and after 10 minutes, zinc chloride (0.00139mol) (0.5M solution in tetrahydrofuran). After 20 minutes, the reaction was warmed to room temperature and tetrakis(triphenylphosphine)palladium (0.053g) and the iodide product of Example 1(a) (0.2g, 0.00046mol) added and the reaction heated at reflux for 16 hours. The reaction was cooled to room temperature, washed with saturated aqueous ethyldiamine tetra-acetic acid di-sodium salt solution and filtered. The aqueous phase was extracted with dichloromethane (3x30ml) and the combined organics washed with brine (100ml), dried (MgSO₄), filtered and the solvent removed under reduced pressure. The crude product was purified on silica gel eluting with 80 : 20 : 5 pentane : ethyl acetate : diethylamine to give the subtitle compound (0.198 g). MS *m*/*z* 431 (MH)⁺. ¹H-NMR (CDCl₃): δ = 0.86 (3H, m), 1.22 (3H, m), 1.88 (1H, m), 2.08 (3H, m), 2.58 (3H, s), 3.62 (2H, q), 3.80 (1H, q), 4.03 (1H, m), 5.50, (2H, s), 6.92 (1H, d), 7.00 (1H, d), 7.35 (1H, d), 7.68 (1H, d), 7.96 (1H, d), 8.27 (1H, s).

### (b) Cis-(1S)-N-methyl-7-(3-(1,2,4-triazolyl))-4-(3,4-dichlorophenyl)-1,2,3,4-tetrahydro-1-naphthalenamine hydrochloride

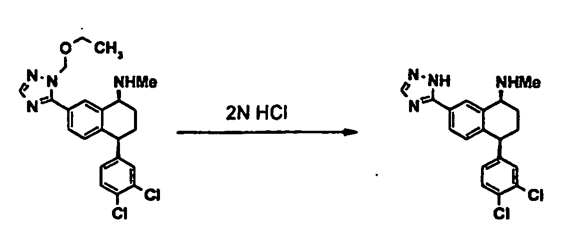

To a solution of the pyrazole product of step (a) (0.193g, 0.00044mol) in ethanol (8ml) was added 2N aqueous hydrochloric acid (2ml) and the reaction heated at reflux for 16 hours. The reaction was cooled, and concentrated under reduced pressure. The mixture was neutralised with saturated aqueous sodium carbonate and extracted with ethyl acetate. The organic phase was dried (MgSO₄), filtered and the solvent removed under reduced pressure. The crude product was purified on silica gel eluting with 97 : 3 : 1 dichloromethane : methanol: 0.88 ammonia to give the title compound. The product was dissolved in dichloromethane (2ml) and a saturated solution of hydrogen chloride in diethyl ether added. The solvent was removed *in vacuo* to yield the hydrochloride salt (0.038g). MS *m*/*z* 373 (MH)⁺. ¹H-NMR (d₄-MeOH): δ: = 2.06 (1H, m), 2.32 (3H, m), 2.92 (3H, s), 4.26 (1H, m), 4.65 (1H, br), 7.12 (1H, d), 7.2 (1H, d), 7.50 (1H, d), 7.54 (1H, d), 7.96 (1H, d), 8.28 (1H, s), 9.24 (1H, s).

### Example 56

### Cis-(1S)-N-methyl-7-(3-(N(2)methyl-1,2,4-triazolyl)-4-(3,4-dichlorophenyl)-1,2,3,4-tetrahydro-1-naphthalenamine hydrochloride

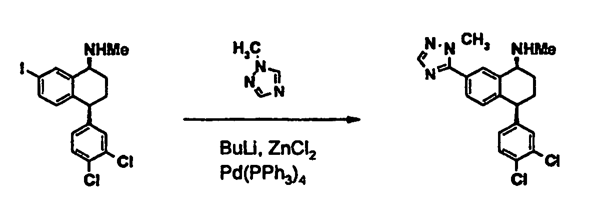

To a solution of N(1)-methyl-1,2,4-triazole (Prepared by the method described in J. Chem. Soc., Perkin Trans. I, 1973, 2506) (0.115g, 0.00139mol) in tetrahydrofuran (5ml) at -78°C under nitrogen was added n-butyllithium (2.5M solution in hexane) (0.00139mol) and after 10 minutes, zinc chloride (0.00139mol) (0.5M solution in tetrahydrofuran). After 15 minutes, the reaction was warmed to room temperature and tetrakis(triphenylphosphine) palladium (0.053g) and the iodide product of Example 1(a) (0.2g, 0.00046mol) added and the reaction heated at reflux for 16 hours. The reaction was cooled to room temperature, washed with saturated aqueous ethyldiamine tetra-acetic acid di-sodium salt solution and filtered. The aqueous phase was extracted with dichloromethane (3x30ml) and the combined organics washed with brine (100ml), dried (MgSO₄), filtered and the solvent removed under reduced pressure. The crude product was purified on silica gel eluting with 100 : 100 : 5 pentane : ethyl acetate : diethylamine to give the title compound (0.130 g). The product was dissolved in dichloromethane (2ml) and a saturated solution of hydrogen chloride in diethyl ether added. The solvent was removed *in vacuo* to yield the hydrochloride salt. MS *m*/*z* 387 (MH)⁺. ¹H-NMR (d₄-MeOH): δ = 2.08 (1H, m), 2.30 (3H, m), 2.9 (3H, s), 3.31 (1H, s), 4.10 (3H, s), 4.30 (1H, m), 4.65 (1H, s), 7.21 (1H, d), 7.28 (1H, d), 7.50 (1H, d), 7.55 (1H, d), 7.65 (1H, m), 7.74 (1H, m), 8.00 (1H, s), 8.39 (1H, s).

### Example 57

### Cis-(1S)-N-methyl-7-(5-(N(1)methyl-1,2,3-triazolyl)-4-(3,4-dichlorophenyl)-1,2,3,4-tetrahydro-1-naphthalenamine

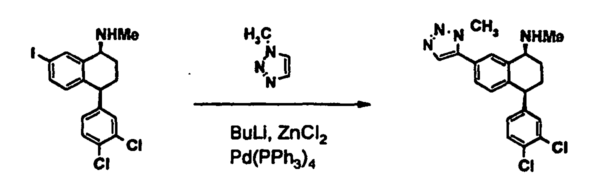

To a solution of N(1)-methyl-1,2,3-triazole (Prepared by the general method described in J. Chem. Soc., Perkin Trans. I, 1973, 2506) (0.115g, 0.00139mol) in tetrahydrofuran (2ml) at -78°C under nitrogen was added n-butyllithium (2.5M solution in hexane) (0.00139mol) and after 15 minutes, zinc chloride (0.00139mol) (0.5M solution in tetrahydrofuran). The reaction was warmed to 0°C and tetrakis(triphenylphosphine)palladium (0.053g) and a solution of the iodide product of Example 1(a) (0.2g, 0.00046mol) in tetrahydrofuran (5ml) added and the reaction heated at reflux for 16 hours. The reaction was cooled to room temperature, saturated aqueous ethyldiamine tetra-acetic acid di-sodium salt solution was added, the mixture stirred for 30 minutes, water was added and the mixture extracted with ethyl acetate. The organic phase was washed with saturated aqueous sodium bicarbonate, brine and dried (MgSO₄). The solution was filtered and the solvent removed under reduced pressure. The crude product was purified on silica gel eluting with 100 : 2.5 : 1 dichloromethane : methanol : 0.88 ammonia to give the title compound as a pale yellow foam (0.148g, 83%). MS *m*/*z* 387 (MH)^{+ 1}H-NMR (CDCl₃): δ = 1.90 (1H, m), 2.05 (3H, m), 2.55 (3H, s), 3.77 (1H, t), 4.04 (1H, t), 4.10 (3H, s), 6.93 (1H, dd), 7.00 (1H, dd), 7.16 (1H, dd), 7.26 (1H, s), 7.38 (1H, dd), 7.46 (1H, s), 7.70 (1H, s).

### Example 58

### Cis-(1S)-N-methyl-7-(4-(1,2,3-triazolyl))-4-(3,4-dichlorophenyl)-1,2,3,4-tetrahydro-1-naphthalenamine hydrochloride

### (a) N(1)-ethoxymethyl-1,2,3-triazole

To a solution of 1,2,3-triazole (10g, 0.147mol) in acetone (55ml), cooled in an ice-water bath was added potassium carbonate (20.3g, 0.147mol) followed by chloromethylethylether (13.4ml, 0.147mol), dropwise. The reaction was warmed to room temperature and stirred for 60 hours. The acetone was removed *in vacuo* and the residue suspended in dichloromethane (100ml), filtered, dried (Na₂SO₄), filtered again and the solvent removed under reduced pressure. The crude product was purified on silica eluting with a solvent gradient of 1:1 to 0:100 hexane : dichloromethane to give the subtitle compound.

### (b) Cis-(1S)-N-methyl-7-(4-(3-(ethoxymethyl)-1,2,3-triazolyl))-4-(3,4-dichlorophenyl)-1,2,3,4-tetrahydro-1-naphthalenamine

To a solution of N(1)-ethoxymethyl-1,2,3-triazole (0.353g) in tetrahydrofuran (5ml) at -78°C under nitrogen was added n-butyllithium (2.5M solution in hexane) (0.00139mol) and after 15 minutes, zinc chloride (0.00139mol) (0.5M solution in tetrahydrofuran). The reaction was warmed to 0°C and tetrakis(triphenylphosphine)palladium (0.053g) and a solution of the iodide product of Example 1(a) (0.4g, 0.00093mol) in tetrahydrofuran (5ml) added and the reaction heated at reflux for 16 hours. The reaction was cooled to room temperature, saturated aqueous ethyldiamine tetra-acetic acid di-sodium salt solution was added, the mixture stirred for 1 hour, water added and the mixture extracted with ethyl acetate. The organic phase was washed with saturated aqueous sodium bicarbonate, brine and dried (MgSO₄). The solution was filtered and the solvent removed under reduced pressure. The crude product was purified on silica gel eluting with 100 : 100 : 5 ethyl acetate : pentane : diethylamine to give the subtitle compound as a pale yellow foam (0.5g) which was used without further purification. MS *m*/*z* 431 (M)⁺. ¹H-NMR (CDCl₃): δ = 1.20 (7H, m), 2.56 (3H, s), 3.56 (2H, q), 3.77 (1H, t), 4.03 (1H, t), 5.67 (2H, s), 6.93 (1H, dd), 7.00 (1H, dd), 7.27 (1H, s), 7.40 (2H, m), 7.65 (1H, d), 7.79 (1H, s).

### (c) Cis-(1S)-N-methyl-7-(4-(1,2,3-triazolyl))-4-(3,4-dichlorophenyl)-1,2,3,4-tetrahydro-1-naphthalenamine hydrochloride

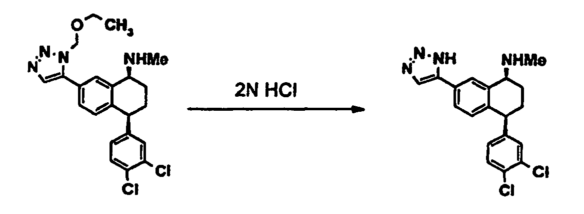

To a solution of the triazole from step (b) (0.5g, 0.00093mol) in ethanol (15ml) was added aqueous 2N HCl solution (7ml) and the reaction heated at reflux for 16 hours. The reaction was cooled to room temperature and the ethanol removed *in vacuo*. The residue was basified with 2M aqueous sodium hydroxide solution and extracted with ethyl acetate. The organic layer was dried (MgSO₄), filtered and the solvent removed under reduced pressure to give the crude product which was purified on silica gel. The product was dissolved in dichloromethane (2ml) and a saturated solution of hydrogen chloride in diethyl ether added. The solvent was removed *in vacuo* to yield the hydrochloride salt (0.24g). MS *m*/*z* 373 (M)⁺. ¹H-NMR (d₆-DMSO): δ = 2.05 (3H, t), 2.20 (1H, m), 2.70 (3H, t), 2.90 (1H, m), 4.18 (1H, t), 4.49 (1H, t), 6.85 (1H, dd), 7.33 (1H, dd), 7.60 (2H, m), 7.73 (1H, dd), 8.21 (1H, s), 8.35 (1H, s), 9.25 (1H, d), 9.35 (1H, d).

### Example 59

### Cis-(1S)-N-methyl-7-(thiomethyl)-4-(3,4-dichlorophenyl)-1,2,3,4-tetrahydro-1-naphthalenamine hydrochloride

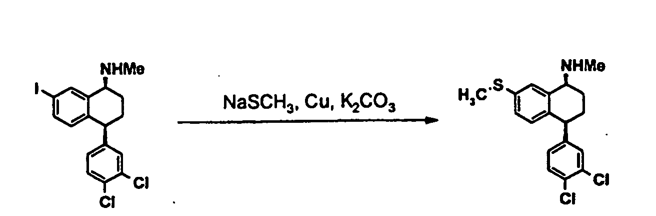

A solution of the iodide product of Example 1(a) (1.3g, 0.02mol), sodium methyl thiolate (0.147g) and copper powder (0.128g) in ethylene glycol (5ml) was warmed to 150°C over 30 minutes. After 1 hour, the reaction was cooled to room temperature and the solvent removed under reduced pressure. The residue was partitioned between ethyl acetate and saturated aqueous ethyldiamine tetra-acetic acid di-sodium salt solution, the organic phase separated and washed with brine (4x50ml), dried (MgSO₄), filtered and the solvent removed under reduced pressure. The crude product was purified on silica gel eluting with a gradient of 100 : 0 : 0 to 95 : 5 : 0.5 dichloromethane : methanol : 0.88 ammonia to yield the free base (0.22g, 32%). The product was dissolved in dichloromethane (2ml) and a saturated solution of hydrogen chloride in diethyl ether added. The solvent was removed *in vacuo* to yield the title compound. MS *m*/*z* 352 (MH)⁺. ¹H-NMR (d₆-DMSO): δ = 2.00 (3H, m), 2.20 (1H, s), 2.50 (3H, s), 2.65 (3H, d), 4.10 (1H, m), 4.4 (1H, s), 6.65 (1H, d), 7.18 (1H, d), 7.30 (1H, d), 7.60 (3H, m).

### Example 60

### Cis-(1S)-N-methyl-7-(methylsulphinyl)-4-(3,4-dichlorophenyl)-1,2,3,4-tetrahydro-1-naphthalenamine hydrochloride

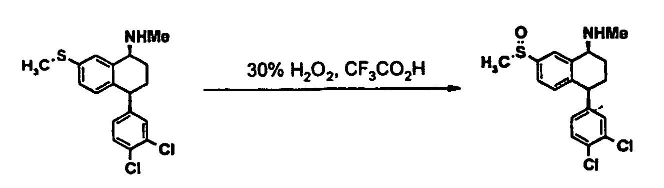

To a solution of the sulphide product of Example 59 (0.154g) in trifluoroacetic acid (2ml) was added 0.3ml of a solution of 30% H₂O₂ (8.6ml) made up to 25 ml with trifluoroacetic acid, at 0°C. After 24 hours, the solvent was removed under reduced pressure and the residue partitioned between aqueous 1N sodium hydroxide solution and ethyl acetate. The organic phase was washed with brine, dried (Na₂SO₄) and the solvent removed under reduced pressure. The residue was purified on silica gel with a gradient of 100 : 0 : 0 to 95 : 5 : 0.5 dichloromethane : methanol : 0.88 ammonia, to yield the free base. The product was dissolved in dichloromethane (2ml) and a saturated solution of hydrogen chloride in diethyl ether added. The solvent was removed *in vacuo* and triturated with diethyl ether to yield the title compound, (0.05g). MS *m*/*z* 368 (MH)⁺. ¹H-NMR (d₆-DMSO): δ = 2.05 (3H, m), 2.25 (1H, m), 2.70 (3H, d), 2.80 (3H, d), 4.20 (1H, m), 4.50 (1H, s), 6.95 (1H, m), 7.35 (1, d), 7.60 (3H, m), 8.00 (1H, d), 9.40 (2H, d).

### Example 61

### Cis-(1S)-N-methyl-7-(methylsulphonyl)-4-(3,4-dichlorophenyl)-1,2,3,4-tetrahydro-1-naphthalenamine hydrochloride

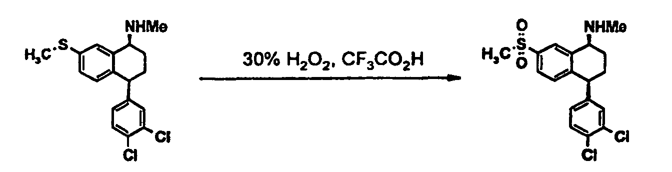

To a solution of the sulphide product of Example 59 (0.282g) in trifluoroacetic acid (4ml) was added 2.2ml of a solution of 30% H₂O₂ (8.6ml) made up to 25 ml with trifluoroacetic acid, at 0°C. After 24 hours at room temperature, the solvent was removed under reduced pressure and the residue partitioned between aqueous 1N sodium hydroxide solution and ethyl acetate. The organic phase was washed with brine, dried (Na₂SO₄) and the solvent removed under reduced pressure. The residue was purified on silica gel using a solvent gradient of 100 : 0 :0 to 95 : 5 : 0.5 dichloromethane : methanol : 0.88 ammonia to yield the free base. The product was dissolved in dichloromethane (2ml) and a saturated solution of hydrogen chloride in diethyl ether added. The solvent was removed *in vacuo* and triturated with diethyl ether to yield the title compound, (0.120g). MS *m*/*z* 384 (MH)⁺. ¹H-NMR (d₆-DMSO): δ = 2.05 (3H, m), 2.30 (1H, m), 2.7 (3H, s), 3.30 (3H, s), 4.25 (1H, t), 4.60 (1H, s), 7.00 (1H, d), 7.35 (1H, d), 7.60 (2H, dd), 7.80 (1H, d), 8.30 (1H, s).

### Example 62

### Cis-(1S)-N-methyl-7-(2-(methylsulphonyl)ethyl)-4-(3,4-dichlorophenyl)-1,2,3,4-tetrahydro-1-naphthalenamine hydrochloride

### (a) Cis-(1S)-N-methyl-7-(2-(methylsulphonyl)vinyl)-4-(3,4-dichlorophenyl)-1,2,3,4-tetrahydro-1-naphthalenamine

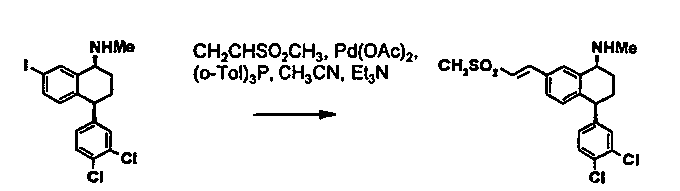

A solution of the iodide product of Example 1(a) (1.3g, 0.003mol), methyl vinyl sulphone (0.478g, 0.0045mol), palladium acetate (0.056g), tri(o-tolyl)phosphine (0.304g, 0.001mol) and triethylamine (1.25ml) in acetonitrile (15ml) was heated under reflux for 4 hours. The reaction was cooled to room temperature, the solvent removed under reduced pressure and the residue partitioned between ethyl acetate and 10% aqueous potassium carbonate solution. The organic phase was washed with brine, dried (MgSO₄), filtered and the solvent removed under reduced pressure. The residue was purified on silica gel using a solvent gradient of 100 : 0 : 0 to 95 : 5 : 0.5 dichloromethane : methanol : 0.88 ammonia to yield the subtitle compound, (0.9g, 73%). MS *m*/*z* 410 (MH)⁺. ¹H-NMR (CDCl₃): δ = 1.85 (1H, m), 2.00 (3H, m), 2.55 (3H, s), 3.05 (3H, s), 3.75 (1H, m), 4.00 (1H, t), 6.85 (3H, m), 7.25 (2H, m), 7.35 (1H, d), 7.60 (2H, d).

### (b) Cis-(1S)-N-methyl-7-(2-(methylsulphonyl)ethyl)-4-(3,4-dichlorophenyl)-1,2,3,4-tetrahydro-1-naphthalenamine hydrochloride

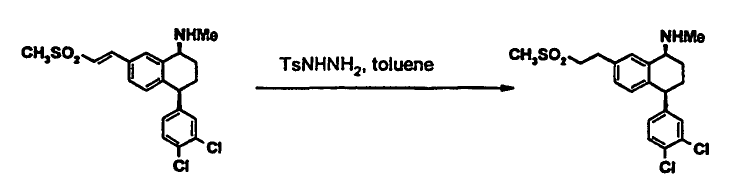

A solution of the vinyl sulphone product from Example 62(a) (0.615g, 0.0015mol) and tosylhydrazine (1.4g, 0.0075mol) in dry toluene (20ml) was heated at reflux for 7 hours. The reaction was cooled to room temperature and the residue was purified on silica gel using a solvent gradient of 100 : 0 : 0 to 95 : 5 : 0.5 dichloromethane : methanol : 0.88 ammonia to yield the free base. The product was dissolved in dichloromethane (2ml) and a saturated solution of hydrogen chloride in diethyl ether added. The solvent was removed *in vacuo* and triturated with diethyl ether to yield the title compound (0.390g). MS *m*/*z* 412 (MH)⁺. ¹H-NMR (d₆-DMSO): δ = 2.00 (3H, m), 2.20 (1H, m), 2.65 (3H, d), 3.00 (3H, s), 3.05 (3H, m), 3.40 (2H, m), 4.10 (1H, t), 4.40 (1H, s), 6.70 (1H, d), 7.10 (1H, d), 7.20 (1H, d), 7.30 (1H, d), 7.45 (1H, d), 7.60 (1H, d), 9.05 (2H, s).

Using the procedure described in of Example 62, the following ethylsulphonamides were prepared by reaction of the iodide product of Example 1(a) and the appropriate vinylsulphonamide:

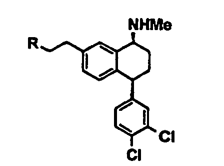

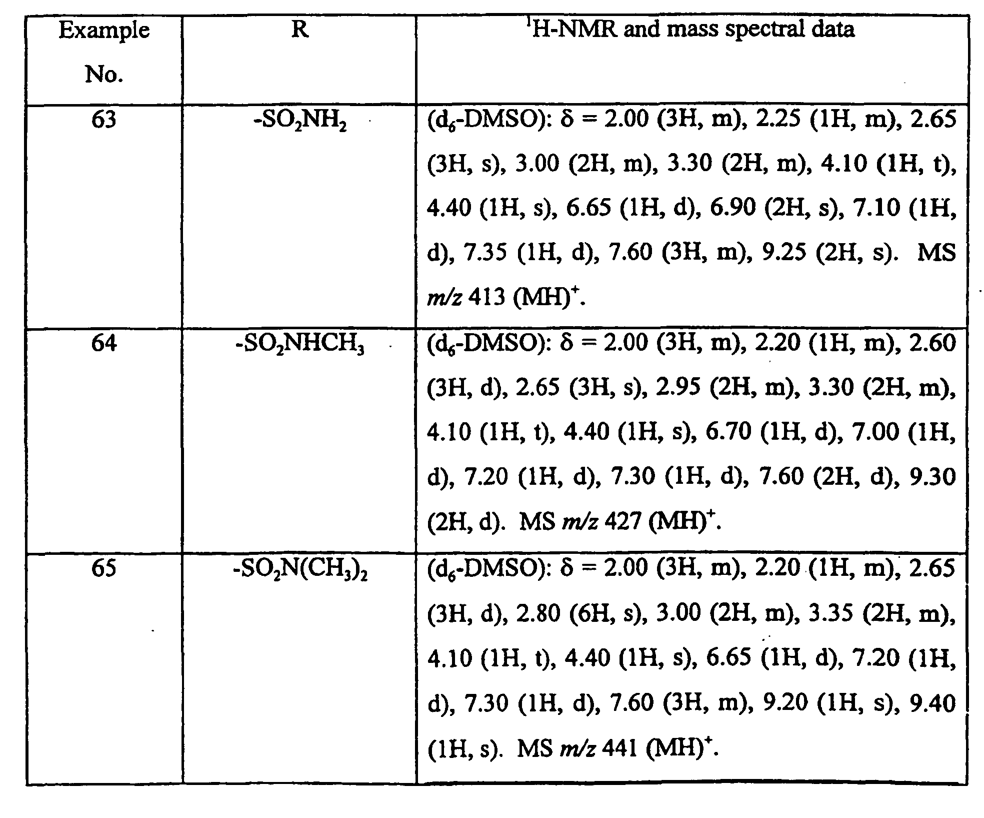

### Example 66

### Cis-(1S)-N-methyl-7-(2-methylcarbamoylethyl)-4-(3,4-dichlorophenyl)-1,2,3,4-tetrahydro-1-naphthalenamine hydrochloride

### (a) Cis-(1S)-N-methyl-7-(2-ethoxycarbonylvinyl)-4-(3,4-dichlorophenyl)-1,2,3,4-tetrahydro-1-naphthalenamine

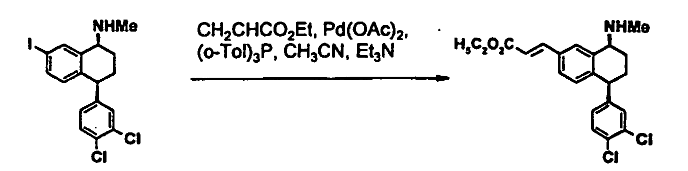

The subtitle compound was prepared using the method described in Example 62(a), using the iodide product of Example 1(a) and ethyl acrylate (yield = 84%). ¹H NMR (CDCl₃): δ = 1.33 (3H, t), 1.83 (1H, m), 2.02 (3H, m), 2.57 (3H, s), 3.71 (1H, t), 3.98 (1H, dd), 4.28 (2H, q), 6.42 (1H, d), 6.82 (1H, d), 6.97 (1H, dd), 7.23 (1H, d), 7.28 (1H, dd), 7.37 (1H, d), 7.52 (1H, d), 7.68 (1H, d). MS *m*/*z* 404 (MH)⁺.

### (b) Cis-(1S)-N-methyl-7-(2-ethoxycarbonylethyl)-4-(3,4-dichlorophenyl)-1,2,3,4-tetrahydro-1-naphthalenamine

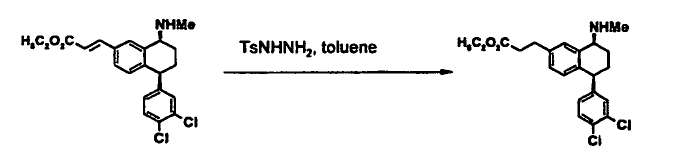

The subtitle compound was prepared using the method described in Example 62(b), using the product of step (a) and tosyl hydrazine (yield = 70.4%). ¹H NMR (CDCl₃): δ = 1.26 (3H, t), 1.82 (1H, m), 2.00 (3H, m), 2.53 (3H, s), 2.61 (2H, t), 2.92 (2H, t), 3.69 (1H, t), 3.94 (1H, dd), 4.14 (2H, q), 6.70 (1H, dd), 6.95 (2H, dt), 7.20 (1H, d), 7.25 (1H, d), 7.34 (1H, d). MS *m*/*z* 406 (MH)⁺.

### (c) Cis-(1S)-N-methyl-7-(2-methylcarbamoylethyl)-4-(3,4-dichlorophenyl)-1,2,3,4-tetrahydro-1-naphthalenamine hydrochloride

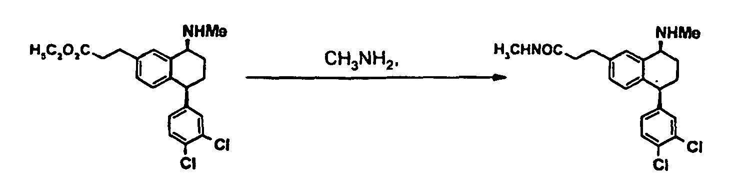

The ester product of step (b) (0.19g) and methylamine (5ml) (33% solution in ethanol) were heated at 100°C for 16 hours. The reaction was cooled and the solvent removed under reduced pressure. The residue was purified on silica gel using a solvent of 87 : 12 : 1 to 95 : 5 : 1 dichloromethane : methanol : 0.88 ammonia to yield the free base. The product was dissolved in ethyl acetate (2ml) and a saturated solution of hydrogen chloride in diethyl ether added. The solvent was removed *in vacuo* and triturated with diethyl ether to yield the title compound, (0.142g, 68%). ¹H NMR (d₆-DMSO): δ = 2.00 (3H, m), 2.21 (1H, m), 2.40 (2H, t), 2.54 (3H, d), 2.67 (3H, s), 2.80 (2H, t), 4.08 (1H, t), 4.35 (1H, br), 6.65 (1H, d), 7.10 (1H, d), 7.30 (1H, d), 7.48 (1H, s), 7.58 (1H, s), 7.60 (1H, d), 7.80 (1H, d), 9.15 (2H, br). MS *m*/*z* 391 (MH)⁺.

Using the procedure described in Example 66(c), the following amides were prepared by reaction of the ester product of Example 66(b) and the appropriate amine:

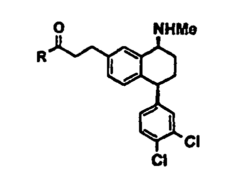

| Example No. | R | ¹H-NMR and mass spectral data |
|---|---|---|
| 67 | -NH(CH₂)₂OH | (d₆-DMSO): δ = 2.00 (3H, m), 2.22 (1H, m), 2.40 (2H, m), 2.67 (3H, s), 2.80 (2H, m), 3.09 (2H, m), 3.32 (2H, m), 4.09 (1H, t), 4.36 (1H, br), 4.65 (1H, br), 6.66 (1H, d), 7.11 1H, dd), 7.30 (1H, dd), 7.48 1H, s), 7.58 (1H, s), 7.60 (1H, d), 7.89 (1H, m), 9.13 (2H, br). MS *m*/*z* 421 (MH)⁺. |
| 68 | -NHCH₂CH(OH) CH₂OH | (d₆-DMSO): δ = 2.00 (3H, m), 2.22 (1H, m), 2.42 (2H, t), 2.68 (3H, s), 2.80 (2H, t), 2.96 (1H, m), 3.1-3.5 (4H, m), 4.08 (1H, t), 4.36 (1H, br), 6.67 (1H, d), 7.11 (1H, dd), 7.31 (1H, dd), 7.46 (1H, s), 7.58 (1H, s), 7.60 (1H, d), 7.85 (1H, br), 9.08 (2H, br). MS *m*/*z* 451 (MH)⁺. |

### Example 69

### Cis-(1S)-N-methyl-7-(2-carboxyethyl)-4-(3,4-dichlorophenyl)-1,2,3,4-tetrahydro-1-naphthalenamine

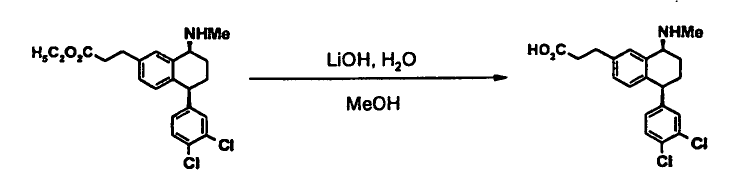

To a solution of the ester product of Example 66(b) (1.0g, 0.0025mmol) in water (15ml) and methanol (60ml) was added lithium hydroxide (0.419g) and the reaction heated at reflux for 1.25 hours. The reaction was cooled to room temperature and the solvent removed *in vacuo*. Water (30ml) was added and the pH adjusted to 5-6 by the addition 2N aqueous hydrochloric acid. The slurry was extracted with a mixture of ethyl acetate and methanol (9:1, x3). The combined organics were washed with water (x3), dried (Na₂SO₄) and the solvent removed under reduced pressure. The solid was triturated with pentane:ether (19:1), to give the title compound, (0.80g) as a cream-white solid. MS *m*/*z* 400 (MNa)⁺. ¹H NMR (Selected data) (CDCl₃): δ = 2.44 (3H, s), 2.50 (1H, m), 2.65-3.03 (3H, m), 3.94 (1H, m), 4.29 (1H, m), 6.78 (1H, d).

### Example 70

### Cis-(1S)-N-methyl-7-(2-propanamido)-4-(3,4-dichlorophenyl)-1,2,3,4-tetrahydro-1-naphthalenamine hydrochloride

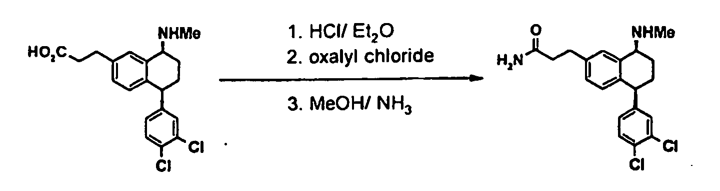

The acid product of Example 69 (0.57g, 0.0015mol) was converted to the hydrochloride salt using ethereal HCl solution and the solvent removed under reduced pressure. This was dissolved in dichloromethane (10ml) and cooled in an ice-water bath, then oxalyl chloride (0.2ml) and N,N-dimethylformamide (cat), added. The reaction was warmed to room temperature and stirred for 1 hour. The solvent was removed and the solid triturated with dichloromethane. The acid chloride was dissolved in dichloromethane (10ml) and added to a solution of saturated methanolic ammonia (5ml), cooled in an ice-water bath. After 21 hours, the solvent was removed under reduced pressure and the crude product purified on silica gel using of 93 : 7 : 1 dichloromethane : methanol : 0.88 ammonia to yield the free base, (18%). 0.1g of this product was dissolved in ethyl acetate (2ml) and a saturated solution of hydrogen chloride in diethyl ether added. The solvent was removed *in vacuo* and triturated with diethyl ether to yield the title compound. (CDCl₃): δ = 1.80 (1H, m), 1.98 (3H, m), 2.52 (5H, m), 2.92 (2H, t), 3.66 (1H, t), 3.96 (1H, q), 5.32 (1H, s), 6.71 (1H, d), 6.95 (2H, dd), 7.21 (2H, dd), 7.34 (1H, d).

### Example 71

### Cis-(1S)-N-methyl-7-(hydroxymethyl)-4-(3,4-dichlorophenyl)-1,2,3,4-tetrahydro-1-naphthalenamine

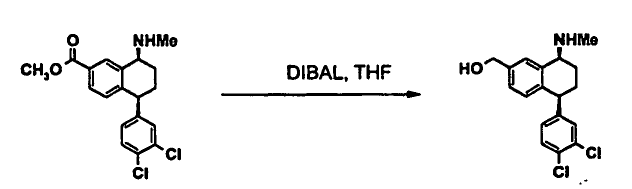

To a solution of the ester product of Example 6(a) (3.53g, 0.096mol) in tetrahydrofuran (100ml) at room temperature was added a solution of di-isobutylaluminium hydride in tetrahydrofuran (29ml, 1M solution) over 15 minutes and the reaction stirred at room temperature. A further 4ml of a solution of di-isobutylaluminium hydride in tetrahydrofuran was added and the reaction stirred at room temperature for 16 hours. Methanol (5ml) was added followed, cautiously, by saturated aqueous ammonium chloride solution, with water-cooling. Ethyl acetate (200ml) was added and the aqueous phase washed with ethyl acetate. The combined organics were washed with water, brine, dried (MgSO₄), filtered and the solvent removed under reduced pressure. The solid was triturated with diethyl ether to give the title compound (2.15g). The mother liquors were purified on silica gel using of 93 : 7 : 1 dichloromethane : methanol : 0.88 ammonia as solvent to yield a second crop of the title compound, (0.34g, total yield = 76%). MS *m*/*z* 336 (MH)⁺. ¹H NMR (CDCl₃): δ = 1.85 (1H, m), 2.02 (3H, m), 2.53 (3H, s), 3.73 (1H, t), 3.95 (1H, dd), 4.67 (2H, s), 6.79 (1H, d), 6.98 (1H, dd), 7.11 (1H, dd), 7.25 (1H, d), 7.34 (1H, d), 7.40 (1H, s).

### Example 72

### Cis-(1S)-N-methyl-7-(N(1)-1,2,3-triazolylmethyl)-4-(3,4-dichlorophenyl)-1,2,3,4-tetrahydro-1-naphthalenamine hydrochloride

### (a) Cis-(1S)-N-methyl-7-(chloromethyl)-4-(3,4-dichlorophenyl)-1,2,3,4-tetrahydro-1-naphthalenamine hydrochloride

To a solution of the alcohol product of Example 71 (0.34g) in tetrahydrofuran (10ml) at room temperature was added a saturated solution of hydrogen chloride in diethyl ether (2ml) followed by thionyl chloride (0.5ml) and the reaction stirred at room temperature for 2 hours. The solvent was removed under reduced pressure to give the subtitle compound, (0.42g, 100%). MS *m*/*z* 354 (M)⁺. ¹H NMR (d₆-DMSO): δ = 2.05 (3H, m), 2.27 (1H, m), 2.69 (3H, t), 4.14 (1H, t), 4.43 (1H, br), 4.74 (2H, s), 6.78 (1H, dd), 7.33 (2H, dd), 7.60 (1H, d), 7.62 (1H, s), 7.71 (1H, d), 9.2 (1H, br), 9.3 (1H, br).

### (b) Cis-(1S)-N-methyl-7-(N(1)-1,2,3-triazolylmethyl)-4-(3,4-dichlorophenyl)-1,2,3,4-tetrahydro-1-naphthalenamine hydrochloride

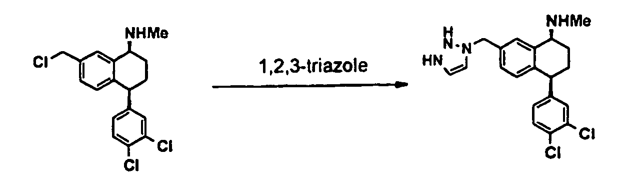

To 1,2,3-triazole (1g) at 120°C was added (72a) (0.2g) over 10 minutes and the reaction heated at 120°C for 1 hour. The reaction was cooled, water added and the mixture extracted with ethyl acetate (x3). The combined organics were washed with brine, dried (MgSO₄), filtered and the solvent removed under reduced pressure. The residue was purified on silica gel using of 93 : 7 : 1 dichloromethane : methanol : 0.88 ammonia as solvent to yield the free base, (0.049g). The product was dissolved in ethyl acetate (2ml) and a saturated solution of hydrogen chloride in diethyl ether added. The solvent was removed *in vacuo* and triturated with diethyl ether to yield the title compound (51 mg, 23%). MS *m*/*z* 387 (MH)⁺. ¹H NMR (d₆-DMSO): δ = 2.00 (3H, m), 2.25 (1H, m), 2.65 (3H, s), 4.11 (1H, t), 4.39 (1H, br), 5.60 (2H, s), 6.75 (1H, d), 7.22 (1H, d), 7.32 (1H, d), 7.60 (3H, m), 7.74 (1H, s), 8.25 (1H, s), 9.20 (2H, br).

### Example 73

### Cis-(1S)-N-methyl-7-((methylthio)methyl)-4-(3,4-dichlorophenyl)-1,2,3,4-tetrahydro-1-naphthalenamine

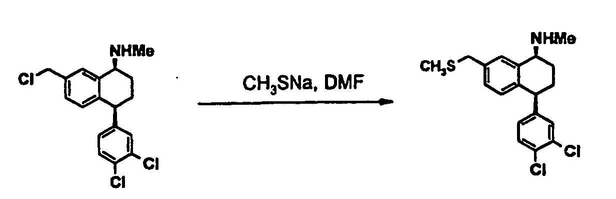

To a solution of chloride product of Example 72(a) (0.1g) in N,N-dimethylformamide (5ml) was added sodium methylthiolate (0.55g) and the reaction heated at 75°C for 3 hours. The reaction was cooled, water added and the mixture extracted with ethyl acetate (x3). The combined organics were washed with water, dried (Na₂SO₄), filtered and the solvent removed under reduced pressure to yield the title compound, (0.08g). MS *m*/*z* 366 (MH)⁺.

### Example 74

### Cis-(1S)-N-methyl-7-((methylsulphonyl)methyl)-4-(3,4-dichlorophenyl)-1,2,3,4-tetrahydro-1-naphthalenamine

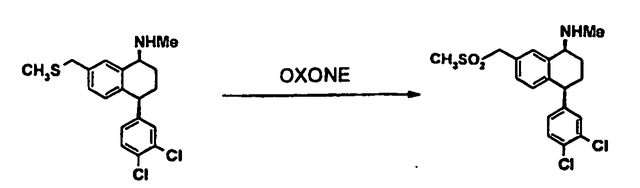

To a solution of the sulphide product of Example 73 (0.073g, 0.0002mol) in isopropyl alcohol (2ml), tetrahydrofuran (0.4ml) and water (0.2ml) cooled in an ice-water bath was added OXONE® (2KHSO₅.KHSO₄.K₂SO₄, 0.061g) and the reaction stirred at room temperature for 3 hours. A further portion of OXONE® (0.061g) was added. After 20 minutes, a few drops of 0.88 ammonia were added and the mixture extracted with ethyl acetate (x3). The combined organics were washed with water, dried (Na₂SO₄), filtered and the solvent removed under reduced pressure. The residue was purified on silica gel using 93 : 7 : 1 dichloromethane : methanol : 0.88 ammonia (diluted 1 : 1 with dichloromethane) as solvent to yield the title compound (0.06g, 75%). MS *m*/*z* 398 (MH)⁺. ¹H NMR (CDCl₃): δ = 1.82 (1H, m), 2.0 (3H, m), 2.54 (3H, m), 2.80 (3H, s), 3.73 (1H, t), 3.98 (1H, t), 4.2 (2H, t), 6.82-7.42 (6H, m, Ar).

### Example 75

### Cis-(1S)-N-methyl-7-((methylsulphinyl)methyl)-4-(3,4-dichlorophenyl)-1,2,3,4-tetrahydro-1-naphthalenamine hydrochloride

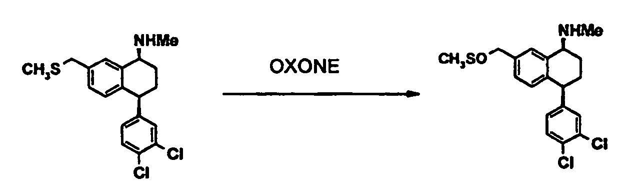

To a solution of the sulphide product of Example 73 (0.290g, 0.00079mol) in isopropyl alcohol (8ml), tetrahydrofuran (1.6ml) and water (0.8ml) cooled in an ice-water bath, was added OXONE® (0.27g) and the reaction stirred at room temperature for 6 hours. Water was added and the mixture extracted with ethyl acetate (x3). The combined organics were washed with water, brine, dried (Na₂SO₄), filtered and the solvent removed under reduced pressure. The residue was purified on silica gel using 93 : 7 : 1 dichloromethane : methanol : 0.88 ammonia (diluted 1 : 1 with dichloromethane). The product was dissolved in ethyl acetate and diethyl ether (2ml) and a saturated solution of hydrogen chloride in diethyl ether added. The solvent was removed *in vacuo* and triturated with diethyl ether to yield the title compound, (0.128g). MS *m*/*z* 382 (MH)⁺. ¹H NMR (CDCl₃): δ = 1.82 (1H, m), 2.01 (3H, m), 2.5 (3H, s), 2.55 (3H, s), 3.70 (1H, t), 3.95 (1H, m), 6.8-7.38 (6H, m, Ar).

### Example 76

### Cis-(1S)-N-methyl-7-((N-methylsulphonamido)methyl)-4-(3,4-dichlorophenyl)-1,2,3,4-tetrahydro-1-naphthalenamine hydrochloride

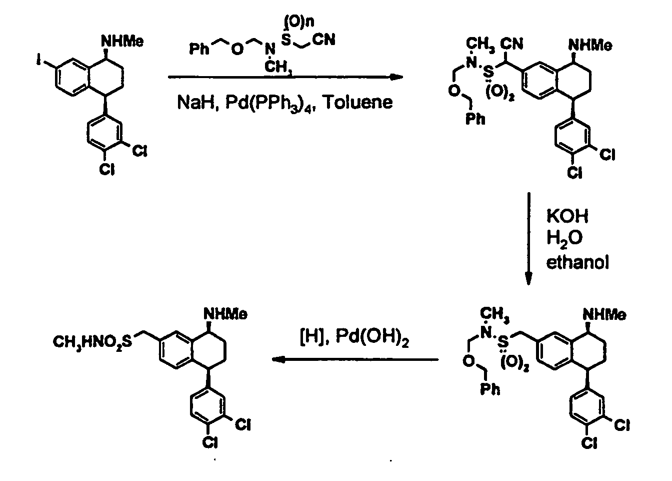

Steps (i)-(v): Preparation of N-[(benzyloxy)methyl](cyano)-N-methyl-methanesulphonamide

### (i) Methyl 2-(chlorosulphonyl)acetate

To a solution of methylthioglycolate (1.3kg, 12.25mol) in dichloromethane (9 litres) was added ice (4.5 litres). Chlorine gas was bubbled gently through the solution, maintaining the temperature below 5°C until the solution maintained a slight green colouration. The solution was degassed with nitrogen to remove excess chlorine, the organic phase collected and the solvent removed under reduced pressure to give the subtitle compound (1.758 kg, 83%) which was used without further purification. ¹H NMR (CDCl₃): 3.91 (3H, s), 4.63 (2H, s).

### (ii) Methyl 2-[(methylamino)sulphonyl]acetate

To a solution of the product of step (i) (75g) in tetrahydrofuran (150ml) at 5°C was added a solution of methylamine in tetrahydrofuran (2M solution, 435ml) over one hour. The yellow slurry was allowed to warm to room temperature and after one hour water (750ml) added. Ethyl acetate (750ml) was added and the aqueous phase extracted with ethyl acetate (500ml). The organics were combined and the solvent removed under reduced pressure to give the subtitle compound (40.2g) which was used without further purification. ¹H NMR (CDCl₃): 2.90 (3H, m), 3.75 (3H, s), 4.05 (2H, s), 4.77 (1H, br)

### (iii) Methyl 2-{[[(benzyloxy)methyl](methyl)amino]sulphonyl}acetate

To a solution of potassium-t-butoxide (14.76g) in tetrahydrofuran (100ml) at 5°C was added a solution of the product of step (ii) (20g) in tetrahydrofuran (50ml) over two hours. After a further hour, a solution of benzylchloromethyl ether (34.35g) in tetrahydrofuran (50ml) was added over 20 minutes, maintaining the temperature below 10°C. After one hour, water (200ml) was added and the mixture extracted with ethyl acetate (200ml). The organic phase was extracted with ethyl acetate (100ml) and the organics were combined. The solvent was removed under reduced pressure to give the crude product which was used without further purification.

### (iv) 2-{[[(benzyloxy)methyl](methyl)amino]sulphonyl}acetamide

To a solution of the product of step (iii) (60g) in tetrahydrofuran (60ml) was added 0.880 ammonia solution (180ml). After 18 hours, the reaction was diluted with water and extracted with ethyl acetate. The organic phase was concentrated under reduced pressure, toluene added and the solution concentrated then heated under reflux and allowed to cool to room temperature. The slurry was filtered, washed with toluene and dried under vacuum at 40°C to give the title compound (30%) which was used without further purification. ¹H NMR (d₆-DMSO): 2.94 (3H, s), 4.00 (2H, s), 4.50 (2H, s), 4.65 (2H, s), 7.22-7.42 (6H, m), 7.69 (1H, br).

### (v) N-[(benzyloxy)methyl](cyano)-N-methylmethanesulphonamide

To a solution of N,N-dimethylformamide (4.96ml) in tetrahydrofuran (100m1) at 5°C was added oxalyl chloride (5.28ml). After 30 minutes, a solution of the product of step (iv) (15g, 0.055mol) in tetrahydrofuran (50ml) was added, maintaining the temperature at 5°C. After 30 minutes, pyridine (9.36ml) was added, the reaction warmed to room temperature and water added. The mixture was extracted with ethyl acetate, the organics combined and the solvent removed under reduced pressure to yield the title compound (13.11g), which was used without further purification. ¹H NMR (CDCl₃): 3.80 (3H, s), 4.02 (2H, s), 4.60 (2H, s), 4.80 (2H, s), 7.30-7.41 (5H, m).

### (a) Cis-(1S)-N-methyl-7-(((N-methyl-N-benzyloxymethyl)sulphonamido)cyanomethyl)-4-(3,4-dichlorophenyl)-1,2,3,4-tetrahydro-1-naphthalenamine

To a solution of sodium hydride (0.24g) in toluene (12ml) and DME (2ml) under an atmosphere of nitrogen was added a solution of the product of step (v) (0.726g) in toluene (3ml), maintaining the temperature below 5°C. Tetrakis(triphenylphosphine) palladium (0.231g) was added and the reaction allowed to warm to room temperature. A solution of the iodide product of Example 1(a) (0.864g) in toluene (3ml) was added and the reaction heated at reflux for 96 hours. The reaction was cooled, water (20ml) added and the mixture extracted with ethyl acetate (x3). The combined organics were washed with brine, dried (Na₂SO₄), filtered and the solvent removed under reduced pressure. The residue was purified on silica gel using 100 : 0 to 95 :5 dichloromethane : methanol as the solvent, to give the subtitle compound, (1.1g). MS *m*/*z* 558 (MH)⁺. ¹H NMR (CDCl₃): δ = 1.80 (1H, m), 2.00 (3H, m), 2.50 (3H, s), 3.10 (3H, s), 3.15 (1H, m), 3.75 (1H, m), 4.00 (1H, t), 4.60 (4H, m), 6.90-7.70 (11H, m, Ar).

### (b) Cis-(1S)-N-methyl-7-(((N-methyl-N-benzyloxymethyl)sulphonamido)methyl)-4-(3,4-dichlorophenyl)-1,2,3,4-tetrahydro-1-naphthalenamine

To a solution of potassium hydroxide (0.605g) in water (15ml) was added a solution of the nitrile product of step (a) (1.0g) in ethanol (15ml) and the reaction heated under reflux for 68 hours. The reaction was cooled, the solvent removed under reduced pressure and the residue partitioned between water and ethyl acetate. The organic phase was dried (Na₂SO₄), filtered and the solvent removed under reduced pressure to give the subtitle compound, (0.8g), which was used without further purification. MS *m*/*z* 533 (MH)⁺. ¹H NMR (CDCl₃): δ = 1.60 (3H, m), 1.80 (1H, m), 2.50 (3H, s), 3.85 (3H, s), 3.70 (1H, m), 3.95 (1H, m), 4.20 (2H, s), 4.50 (4H, s), 6.80-7.70 (11H, m).

### (c) Cis-(1S)-N-methyl-7-((N-methylsulphonamido)methyl)-4-(3,4-dichlorophenyl)-1,2,3,4-tetrahydro-1-naphthalenamine hydrochloride

To a solution of the product of step (b) (0.28g) in methanol (20ml) and water (10ml) was added palladium hydroxide (0.05g) and methane sulphonic acid (0.2ml) and the reaction heated at 60°C under an atmosphere of hydrogen at 414 kPa (60 p.s.i.) for 20 hours. The reaction was cooled to room temperature and filtered through Arbacel™ filter aid. Water was added to the filtrate, the solution made basic with 1N aqueous sodium hydroxide solution and extracted with ethyl acetate. The organic phase was dried (Na₂SO₄), filtered and the solvent removed under reduced pressure. The residue was purified on silica gel using a solvent gradient of 100 : 0 : 0 to 95 : 5 : 0.5 dichloromethane : methanol : ammonia to yield the free base. The product was dissolved in dichloromethane (2ml) and a saturated solution of hydrogen chloride in diethyl ether added. The solvent was removed *in vacuo*, triturated with diethyl ether and dried to yield the title compound, (0.080g). MS *m*/*z* 413 (MH)⁺. ¹H NMR (d₆-DMSO): δ = 2.00 (3H, m), 2.30 (1H, m), 2.55 (2H, d), 3.30 (3H, s), 4.10 (1H, m), 4.25 (2H, q), 4.40 (1H, s), 6.75 (1H, d), 7.00 (1H, d), 7.25 (1H, d), 7.35 (1H, d), 7.60 (2H, m), 9.20 (1H, s), 9.30 (1H, s).

### Example 77

### Cis-(1S)-N-methyl-7-((carboxamido)methyl)-4-(3,4-dichlorophenyl)-1,2,3,4-tetrahydro-1-naphthalenamine hydrochloride

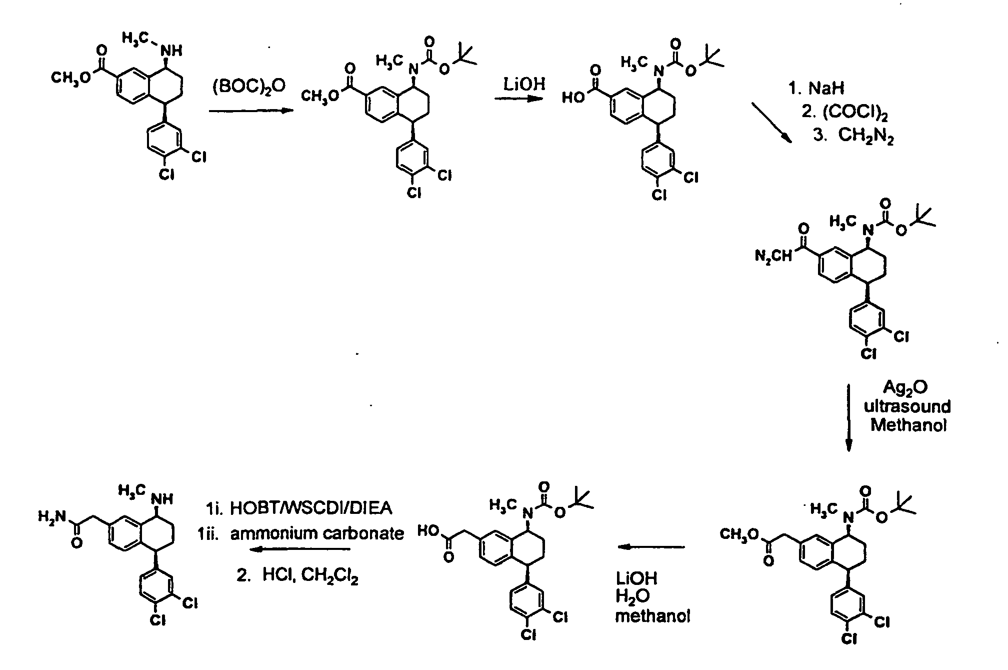

### (a) Cis-(1S)-N-methyl-N-tert-butoxycarbonyl-7-(methoxycarbonyl)-4-(3,4-dichlorophenyl)-1,2,3,4-tetrahydro-1-naphthalenamine

To a solution of the ester produced in Example 6(a) (4.3g) in tetrahydrofuran (50ml) and di-isopropylethylamine (1.68g) was added di-tert-butyl dicarbonate (2.84g) and the reaction stirred at room temperature for 16 hours. The solvent was removed under reduced pressure, toluene added and the solution washed with water (x2), brine, dried (MgSO₄), filtered and the solvent removed under reduced pressure. The residue was purified on silica gel using 9 : 1 pentane : ethyl acetate as solvent, to yield the subtitle compound, (4.4g, 80%). MS *m*/*z* 481 (MNH₄)⁺. ¹H NMR (CDCl₃) δ = 1.54 (9H, s), 1.79 (2H, m), 2.02 (1H, m), 2.29 (1H, m), 2.66 (3H, s), 3.91 (3H, s), 4.22 (1H, br), 5.30 (0.45H, br); 5.49 (0.55H, br), 6.79 (1H, d), 7.05 (1H, d), 7.07 (1H, d), 7.34 (1H, d), 7.82 (1H, d), 7.91 (1H, s).

### (b) Cis-(1S)-N-methyl-N-tert-butoxycarbonyl-7-(carboxy)-4-(3,4-dichlorophenyl)-1,2,3,4-tetrahydro-1-naphthalenamine

A solution of the carbamate produced in step (a) (2.0g) in methanol (120ml) and water (30ml), together with lithium hydroxide monohydrate (0.724g) was heated under reflux for 1.5 hours. The reaction was cooled to room temperature, the residue dissolved in water (60ml) and the pH adjusted to 3-4 using 2N aqueous hydrochloric acid solution. The solution was extracted with dichloromethane (x5), the combined organics dried (MgSO₄), filtered and the solvent removed under reduced pressure to give the subtitle compound, (1.81g), which was used without further purification. MS *m*/*z* 467 (MNH₄)⁺. ¹H NMR (CDCl₃): δ = 1.55 (9H, s), 1.80 (2H, m), 2.05 (1H, m), 2.30 (1H, m), 2.66 (3H, s), 4.22 (1H, br), 5.32 (0.45H, br), 5.50 (0.55H, br), 6.80 (1H, dd), 7.05 (1H, dd), 7.10 (1H, s), 7.34 (1H, d), 7.90 (1H, dd), 8.00 (1H, br).

### (c) Cis-(1S)-N-methyl-N-tert-butoxycarbonyl-7-(diazomethylcarbonyl)-4-(3,4-dichlorophenyl)-1,2,3,4-tetrahydro-1-naphthalenamine

To a solution of the sodium salt of (77b) (8.0g) in tetrahydrofuran (100ml) at 0°C was added N,N-dimethylformamide (cat.) followed by oxalyl chloride (3.2g, 1.5 equiv.) over 30 minutes. The reaction was warmed to room temperature and stirred for 2 hours. The solution was cooled to 0°C, a solution of diazomethane (2 equivalents) in diethyl ether (100ml) added and the reaction stirred at room temperature for 16 hours. The reaction was cooled again to 0°C, a solution of diazomethane (2 equivalents) in diethyl ether (100ml) added and the reaction stirred at room temperature for a further 16 hours. Nitrogen was bubbled through the solution for 1 hour to remove excess diazomethane and the solvent removed under reduced pressure. The residue was partitioned between diethyl ether and 10% aqueous citric acid, the organic phase separated and washed with saturated aqueous sodium bicarbonate solution and brine. The organic phase was dried (MgSO₄), filtered and the solvent removed under reduced pressure. The residue was purified on silica gel using 3 : 1 pentane : ethyl acetate as solvent, to yield the subtitle compound, (3.7g, 46%). ¹H NMR (CDCl₃): δ = 1.52 (9H, s), 1.79 (2H, m), 2.02 (1H, m), 2.28 (1H, m), 2.65 (3H, t), 4.21 (1H, br), 5.30 (0.45H, br), 5.48 (0.55H, br), 5.87 (1H, s), 6.79 (1H, d), 7.04 (1H, d), 7.08 (1H, s), 7.34 (1H, d), 7.58 (1H, d), 7.61 (1H, s).

### (d) Cis-(1S)-N-methyl-N-tert-butoxycarbonyl-7-(methoxycarbonylmethyl)-4-(3,4-dichlorophenyl)-1,2,3,4-tetrahydro-1-naphthalenamine

To a solution of the compound produced in step (c) (0.173g) in methanol (4ml) was added triethylamine (1ml) and silver (I) oxide (0.024g) and the mixture sonicated in an ultrasound bath for 35 minutes. The solvent was removed under reduced pressure and the residue purified on silica gel using 4 : 1 pentane : ethyl acetate as solvent, to yield the subtitle compound, (57%). MS *m*/*z* 495 (MNH₄)⁺. ¹H NMR (CDCl₃): δ = 1.53 (9H, s), 1.75 (2H, m), 2.00 (1H, m), 2.25 (1H, m), 2.62 (3H, s), 3.62 (2H, s), 3.73 (3H, s), 4.15 (1H, br), 5.30 (0.45H, br), 5.47 (0.55H, br), 6.80 (1H, br), 6.91 (1H, d), 7.09 (3H, m), 7.33 (1H, d).

### (e) Cis-(1S)-N-methyl-N-tert-butoxycarbonyl-7-(carboxymethyl)-4-(3,4-dichlorophenyl)-1,2,3,4-tetrahydro-1-naphthalenamine

To a solution of the ester produced in step (d) (0.1g) in methanol (4ml) and water (1ml) was added lithium hydroxide monohydrate (0.042g) and the mixture heated under reflux for 1.25 hours. The reaction was cooled to room temperature and the solvent removed under reduced pressure. Water was added and the pH adjusted to 2 using 2N aqueous hydrochloric acid. The solution was extracted with ethyl acetate and the combined organics were washed with brine, dried (MgSO₄), filtered and the solvent removed under reduced pressure to give the subtitle compound (96%), which was used without further purification. MS *m*/*z* 481 (MNH₄)⁺. ¹H NMR (CDCl₃): δ = 1.52 (9H, s), 1.75 (2H, m), 2.00 (1H, m), 2.27 (1H, m), 2.63 (3H, s), 3.65 (2H, s), 4.15 (1H, br), 5.29 (0.45H, br), 5.46 (0.55H, br), 6.80 (1H, br), 6.93 (1H, d), 7.12 (3H, m), 7.32 (1H, d).

### (f) Cis-(1S)-N-methyl-N-tert-butoxycarbonyl-7-((carboxamido)methyl)-4-(3,4-dichlorophenyl)-1,2,3,4-tetrahydro-1-naphthalenamine

To a solution of the acid produced in step (e) (0.092g), hydroxybenzotriazole (0.041g), 1-(3-dimethylaminopropyl)-3-ethyl-carbodiimide hydrochloride (0.046g) and ammonium carbonate (0.038g) in dioxan (4ml) was added diisopropylethylamine (0.087ml) and the reaction stirred at room temperature for 18 hours. The solvent was removed under reduced pressure, water added and extracted with ethyl acetate (x2). The combined organics were dried (MgSO₄), filtered and the solvent removed under reduced pressure to give the subtitle compound (98%), which was used without further purification. MS *m*/*z* 480 (MNH₄)⁺. ¹H NMR (CDCl₃): δ = 1.52 (9H, m), 1.75 (2H, m), 2.00 (1H, m), 2.25 (1H, m), 2.62 (3H, s), 3.56 (2H, s), 4.15 (1H, t), 5.29 (0.45H, br), 5.41 (0.55H, br), 5.50 (1H, br), 5.69 (1H, br), 6.81 (1H, d), 6.94 (1H, d), 7.10 (3H, m), 7.32 (1H, d).

### (g) Cis-(1S)-N-methyl-7-((carboxamido)methyl)-4-(3,4-dichlorophenyl)-1,2,3,4-tetrahydro-1-naphthalenamine hydrochloride

A solution of the compound produced in step (f) (0.091g) in dichloromethane (5ml) was cooled in an ice-water bath and the solution saturated with hydrogen chloride gas. The reaction was stirred for 1 hour, the solvent removed under reduced pressure, the residue azeotroped with dichloromethane (x3) and then triturated with diethyl ether to give the title compound, (0.065g, 91%). MS *m*/*z* 363 (MH)⁺. ¹H NMR (d₄-MeOH): δ = 1.98 (1H, m), 2.22 (3H, m), 2.88 (3H, s), 3.58 (2H, s), 4.15 (1H, dd), 4.48 (1H, t), 6.88 (1H, d), 7.24 (2H, dt), 7.45 (2H, s), 7.50 (1H, d).

### Example 78

### Cis-(1S)-N-methyl-7-((N-methylcarboxamido)methyl)-4-(3,4-dichlorophenyl)-1,2,3,4-tetrahydro-1-naphthalenamine hydrochloride

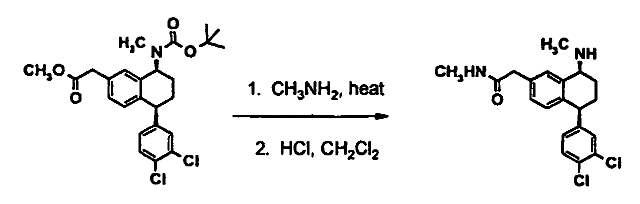

### (a) Cis-(1S)-N-methyl-N-tert-butoxycarbonyl-7-((N-methylcarboxamido)methyl)-4-(3,4-dichlorophenyl)-1,2,3,4-tetrahydro-1-naphthalenamine

A solution of the ester product of Example 77(d) (0.043g) in ethanol (0.5ml) and methylamine in ethanol (33% solution) (0.5ml) was heated at 90°C for 3 hours. The reaction was cooled, the solvent removed under reduced pressure and the residue purified on silica gel using 98 : 2 : 0.25 dichloromethane : methanol : 0.88 ammonia as solvent to give the subtitle compound (0.033g, 77%). MS *m*/*z* = 495 (MNH₄)⁺. ¹H NMR (CDCl₃): δ = 1.52 (9H, s), 1.25 (2H, m), 2.00 (1H, m), 2.25 (1H, m), 2.63 (3H, s), 2.80 (3H, d), 3.54 (2H, s), 4.15 (1H, br), 4.29 (0.45H, br), 5.42 (0.55H, br), 6.82 (1H, d), 6.94 (1H, d), 7.08 (3H, m), 7.32 (1H, d).

### (b) Cis-(1S)-N-methyl-7-((N-methylcarboxamido)methyl)-4-(3,4-dichlorophenyl)-1,2,3,4-tetrahydro-1-naphthalenamine hydrochloride

A solution of the product of step (a) (0.033g) in dichloromethane (5ml) was cooled in an ice-water bath and the solution saturated with hydrogen chloride gas. The reaction was stirred for 1 hour, the solvent removed under reduced pressure, the residue azeotroped with dichloromethane (x3) and then triturated with diethyl ether to give the title compound, (0.023g, 85%). MS *m*/*z* 377 (MH)⁺. ¹H NMR (CDCl₃): δ = 2.06 (2H, m), 2.21 (1H, m), 2.38 (1H, m), 2.55 (3H, s), 2.72 (3H, d), 3.32 (1H, d), 3.47 (1H, d), 3.95 (1H, br), 4.12 (1H, br), 6.66 (1H, br), 6.83 (1H, d), 7.18 (2H, m), 7.21 (1H, d), 7.38 (1H, d), 7.43 (1H, s), 7.70 (1H, s).

### Example 79

### Cis-(1S)-N-methyl-7-(nitro)-4-(3,4-dichlorophenyl)-1,2,3,4-tetrahydro-1-naphthalenamine

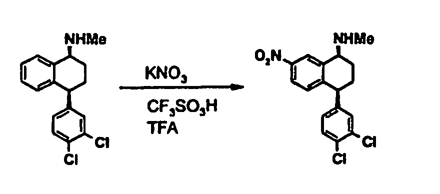

To a stirred solution of cis-(1S)-N-methyl-4-(3,4-dichlorophenyl)-1,2,3,4-tetrahydro-1-naphthalenamine hydrochloride (7.8g, 0.0228mol) in trifluoroacetic acid (70ml) in an ice-water bath was added triflic acid (7ml) followed by potassium nitrate (2.32g, 0.0228mol). After 1.5 hours, the reaction was poured into a mixture of ice and 0.88 ammonia and extracted with ethyl acetate (2x100ml). The combined organics were washed with brine (200ml), dried (MgSO₄), filtered and the solvent removed under reduced pressure. The crude product was purified on silica gel eluting with a gradient of 99.8:0.2 to 99.2:0.8 dichloromethane:methanol to give the title compound (3.8g, 44%). [containing ca., 5% of the 5-nitro isomer]. MS *m*/*z* 351 (MH)⁺. ¹H-NMR (CDCl₃): δ = 1.8-2.1 (4H, m), 2.5 (3H, s), 3.8 (1H, t), 4.1 (1H, m), 6.9 (1H, dd), 7.0 (1H, d), 7.2 (1H, s), 7.4 (1H, d), 7.9 (1H, dd), 8.3 (1H, s).

### Example 80

### Cis-(1S)-N-methyl-7-(amino)-4-(3,4-dichlorophenyl)-1,2,3,4-tetrahydro-1-naphthalenamine

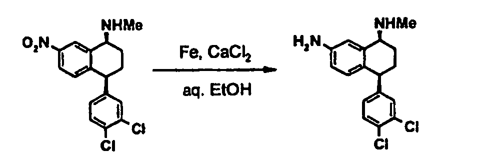

To a solution of the product of Example 79 (14.7g, 0.0419mol) in 85% aqueous ethanol (300ml) was added iron powder (21.1g, 0.376mol) and calcium chloride (2.1g, 0.019mol) and the reaction heated under reflux for 16 hours. The reaction was cooled and filtered through Arbacel™, eluting with ethyl acetate. The collected filtrate was concentrated under reduced pressure, dried (MgSO₄), filtered and the solvent removed under reduced pressure. The crude product was purified on silica gel eluting with a gradient of 98:2 dichloromethane:methanol to 90:10:1 dichloromethane:methanol:ammonia to give the title compound (9 g, 67%). MS *m*/*z* 321 (MH)⁺. ¹H-NMR (CDCl₃): δ = 1.8-2.1 (4H, m), 2.6 (3H, s), 3.7 (2H, br), 3.8 (1H, m), 3.9 (1H, m), 6.5 (1H, d), 6.5 (1H, d), 6.6 (1H, d), 6.9 (1H, s), 7.0 (1H, d), 7.2 (1H, s), 7.3 (1H, d), which was preceded on the column by the 5-amino isomer which was isolated pure following further chromatography, eluting with 99.8:0.2 dichloromethane:methanol (390mg, 3%). MS *m*/*z* 321 (MH)⁺. ¹H-NMR (CDCl₃, partial data): δ = 1.8-2.2 (4H, m), 3.8 (1H,m), 4.0 (1H, m), 6.6 (1H, d), 6.9 (1H, d), 7.0 (1H, d), 7.1 (1H, t), 7.3 (1H, d).

### Example 81

### Cis-(1S)-N-methyl-7-((methylsulphonyl)amino)-4-(3,4-dichlorophenyl)-1,2,3,4-tetrahydro-1-naphthalenamine

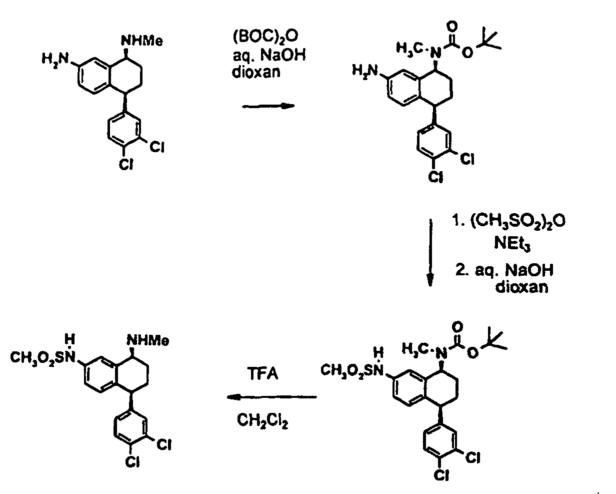

### (a) Cis-(1S)-N-methyl-N-tert-butoxycarbonyl-7-(amino)-4-(3,4-dichlorophenyl)-1,2,3,4-tetrahydro-1-naphthalenamine

To a solution of the amine product of Example 80 (0.65g, 0.00202mol) in a mixture of dioxan (8ml), water (2ml) and 1N aqueous sodium hydroxide (2ml), cooled in an ice-water bath, was added a solution of di-tert-butyl dicarbonate (0.442g, 0.00202mol). After 1 hour, the reaction was adjusted to pH 4 using 5% aqueous citric acid solution and extracted with ethyl acetate (2x20ml). The combined organics were washed with brine, dried (MgSO₄), filtered and the solvent removed under reduced pressure. The crude product was purified on silica gel eluting with 99.8:0.2 dichloromethane:methanol to give the subtitle compound, (0.33 g, 39%). MS *m*/*z* 421 (MH)⁺. ¹H-NMR (CDCl₃) (selected data): δ = 1.5 (9H, s), 1.7 (2H, m), 2.0 (1H, m), 2.26 (1H, m), 2.6 (2H, s), 4.1 (1H, t).

### (b) Cis-(1S)-N-methyl-N-tert-butoxycarbonyl-7-((methylsulphonyl)amino)-4-(3,4-dichlorophenyl)-1,2,3,4-tetrahydro-1-naphthalenamine

To a solution of the amine prepared in step (a) (0.32g) and triethylamine (0.328ml) in tetrahydrofuran at room temperature was added methane sulphonic anhydride (0.273g) and the mixture stirred for I hour. Water and triethylamine (0.328ml) were added and the mixture extracted with ethyl acetate (x2). The combined organics were dried (MgSO₄), filtered and the solvent removed under reduced pressure. The residue was stirred in a solution of 1N aqueous sodium hydroxide : dioxan (1:1) for 0.5 hours, extracted into ethyl acetate (x2), the combined organics were dried (MgSO₄), filtered and the solvent removed under reduced pressure to give the subtitle compound, (0.337g, 89%). MS *m*/*z* 518 (MNH₄)⁺. ¹H-NMR (CDCl₃): δ = 1.56 (9H, s), 1.75 (2H, m), 2.00 (1H, m), 2.24 (1H, m), 2.63 (3H, s), 3.04 (3H, s), 4.17 (1H, m), 5.41 (1H, m), 6.36 (1H, m), 6.78-7.36 (6H, m, Ar).

### (c) Cis-(1S)-N-methyl-7-((methylsulphonyl)amino)-4-(3,4-dichlorophenyl)-1,2,3,4-tetrahydro-1-naphthalenamine

To a solution of the product of step (b) (0.12g) in dichloromethane, cooled in an ice-water bath was added trifluoroacetic acid (0.275ml) and the mixture stirred at room temperature for 0.5 hours. Dichloromethane (10ml) was added and the reaction stirred at room temperature for 16 hours. The reaction was poured into a cooled solution of 0.880 ammonia : water (1:3) and extracted with ethyl acetate (x2). The combined organics were dried (MgSO₄), filtered and the solvent removed under reduced pressure. The crude product was purified on silica gel eluting with 95:5:0.5 dichloromethane: methanol:ammonia to give the title compound (0.06 g, 63%). MS *m*/*z* 416 (MNH₄)⁺. ¹H-NMR (CDCl₃): δ = 1.8-2.0 (4H, m), 2.6 (3H, s), 3.0 (3H, s), 3.8 (1H, m), 4.0 (1H, m), 6.78-7.38 (6H, m, Ar)

### Example 82

### Cis-(1S)-N-methyl-7-(N-methyl(methylsulphonyl)amino)-4-(3,4-dichlorophenyl)-1,2,3,4-tetrahydro-1-naphthalenamine

### (a) Cis-(1S)-N-methyl-N-tert-butoxycarbonyl-7-(N-methyl(methylsulphonyl)amino)-4-(3,4-dichlorophenyl)-1,2,3,4-tetrahydro-1-naphthalenamine

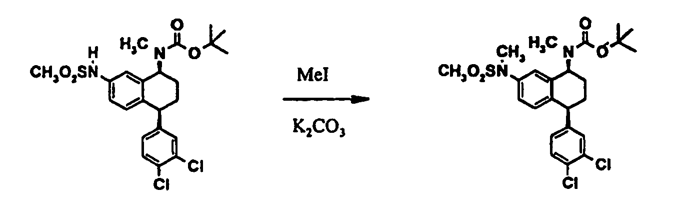

To a solution of the compound of Example 81(b) (0.23g) in acetone (2ml) was added potassium carbonate (0.067g) and methyl iodide (0.09ml) and the reaction heated at reflux for 16 hours. The reaction was cooled to room temperature, the solvent removed under reduced pressure and the residue partitioned between 10% aqueous potassium carbonate and ethyl acetate. The aqueous phase was extracted with ethyl acetate and the combined organics dried (MgSO₄), filtered and the solvent removed under reduced pressure. The crude product was purified on silica gel eluting with 99.5:0.5 dichloromethane:methanol to give the subtitle compound, (0.15 g, 63%). MS *m*/*z* 530 (MNH₄)⁺. ¹H-NMR (CDCl₃): δ = 1.5 (9H, s), 1.8 (2H, m), 2.0 (1H, m), 2.3 (1H, m), 2.6 (3H, s), 2.9 (3H, s), 3.3 (3H, s), 4.2 (1H, s), 5.5 (1H, m), 6.8-7.3 (6H, m, Ar).

### (b) Cis-(1S)-N-methyl-7-(N-methyl(methylsulphonyl)amino)-4-(3,4-dichlorophenyl)-1,2,3,4-tetrahydro-1-naphthalenamine

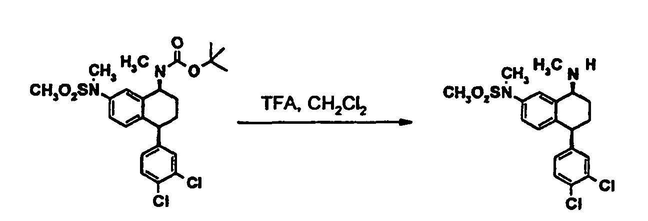

To a solution of the product of step (a) (0.15g) in dichloromethane, cooled in an ice-water bath was added trifluoroacetic acid (0.23ml) and the mixture stirred at room temperature for 0.5 hours. Dichloromethane (10ml) was added and the reaction stirred at room temperature for 16 hours. The reaction was poured into a cooled solution of 0.88 ammonia : water (1:1) and extracted with ethyl acetate (x2). The combined organics were dried (MgSO₄), filtered and the solvent removed under reduced pressure. The crude product was purified on silica gel eluting with 98:2 dichloromethane:methanol to give the title compound, (0.082 g, 67%). ¹H-NMR (CDCl₃): δ = 1.8-2.0 (4H, m), 2.7 (3H, s), 2.9 (3H, s), 3.2 (3H, s), 3.3 (1H, m), 4.0 (1H, m), 6.8-7.4 (6H, m).

Using the method of Example 82, the following sulphonamides were prepared using the sulphonamide product of Example 81(b) and the appropriate alkylating agent:

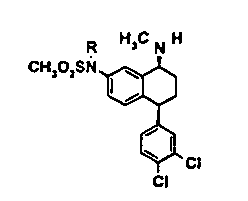

| Example No. | R | ¹H-NMR and mass spectral data |
|---|---|---|
| 83¹ | -CH₂CH₂OH | (d₆-DMSO): δ = 1.92-2.29 (4H, m), 2.64 (3H, m), 3.08 (3H, s), 3.38 (2H, m), 3.66 (2H, m), 4.12 (1H, m), 4.43 (1H, m), 6.78-7.79 (6H, Ar), 9.38 (1H, br), 9.59 (1H, br). MS *m*/*z* 443 (MH)⁺. |
| 84¹ | -CH₂CH₂OCH₃ | (d₆-DMSO): δ = 1.92-2.28 (4H, m), 2.66 (3H, m), 3.08 (3H, s), 3.19 (3H, s), 3.34 (2H, m), 3.80 (2H, m), 4.16 (1H, m), 4.44 (1H, br), 6.72-7.80 (6H, Ar), 9.47 (1H, br), 9.70 (1H, br). MS *m*/*z* 457 (MH)⁺. |

| | | |
|---|---|---|
| 1. Hydrochloride salt | | |

### Example 85

### Cis-(1S)-N-methyl-7-(N(4)-1,2,4-triazolyl)-4-(3,4-dichlorophenyl)-1,2,3,4-tetrahydro-1-naphthalenamine

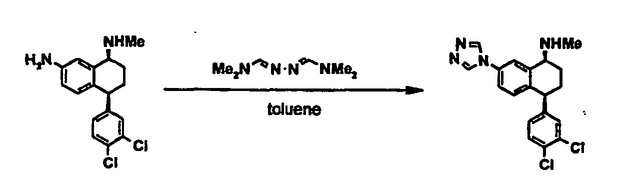

To a solution of the product of Example 80 (0.4g, 0.00125mol) in toluene (10ml) was added N'-[(E)-(dimethylamino)methylidene]-N,N-dimethylhydrazonoformamide (J.Chem. Soc. (C)., 1967, 1664) (0.212g) and p-toluene sulphonic acid (0.284g) and the reaction heated at reflux for 16 hours. The reaction was cooled, diluted with ethyl acetate and made basic with saturated aqueous sodium bicarbonate solution. The organic layer was dried (MgSO₄), filtered and the solvent removed under reduced pressure. The crude product was purified on silica gel, eluting with a solvent gradient of 100 : 100 : 5 ethyl acetate : pentane : diethylamine to 100 : 5 : 5 ethyl acetate : methanol : diethylamine to yield the title compound, (0.194g, 42%). MS *m*/*z* 373 (MH)⁺. ¹H-NMR (CDCl₃): δ = 1.93 (1H, m), 2.07 (3H, m), 2.57 (3H, s), 3.78 (1H, t), 4.06 (1H, t), 6.97 (1H, dd), 6.99 (1H, dd), 7.13 (1H, dd), 7.22 (1H, d), 7.39 (1H, dd), 7.50 (1H, d), 8.46 (2H, s).

### Example 86

### Cis-(1S)-N-methyl-7-hydroxy-4-(3,4-dichlorophenyl)-1,2,3,4-tetrahydro-1-naphthalenamine

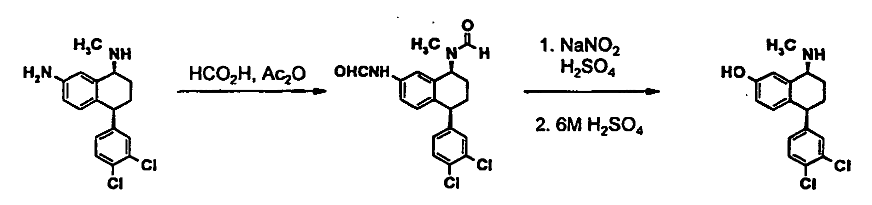

### (a) Cis-(1S)-N-methyl-N-formyl-7-(N-formylamino)-4-(3,4-dichlorophenyl)-1,2,3,4-tetrahydro-1-naphthalenamine

Formic acid (2.45ml) was added to a stirred solution of acetic anhydride (4.9ml), cooled in an ice-water bath. This was heated to 50°C for 0.25h, cooled to 5°C and added to a solution of the product of Example 80 (1.37g) in formic acid (2.6ml). The reaction was stirred at room temperature for 2 hours, ice-water added and the mixture extracted with dichloromethane (x2). The combined organics were washed with 1N aqueous sodium hydroxide solution, brine, dried (Na₂SO₄), filtered and the solvent removed under reduced pressure. The crude product was purified on silica gel, eluting with a solvent gradient of 100 : 0 to 95 : 5 dichloromethane : methanol to yield the subtitle compound, (1.1g, 74%). MS *m*/*z* 377 (MH)⁺. ¹H-NMR (CDCl₃): δ =1.95 (3H, m), 2.30 (1H, m), 2.75 (2H, d), 2.80 (1H, d), 4.20 (1H, m), [4.70 (m), 5.70 (t), 1H], 6.80-8.70 (6H, Ar).

### (b) Cis-(1S)-N-methyl-N-formyl-7-(hydroxy)-4-(3,4-dichlorophenyl)-1,2,3,4-tetrahydro-1-naphthalenamine

To a solution of the product of step (a) (0.995g) in acetone (28.5ml) was added a mixture of concentrated sulphuric acid (5.7ml) and water (28.5ml), in an ice-water bath. Sodium nitrite (0.217g) was added, stirred in the ice-water bath for 0.5 hours and then allowed to warm to room temperature. The reaction was heated at 75°C for 2 hours, cooled in an ice-water bath and made basic with concentrated aqueous ammonium hydroxide solution. The reaction was extracted with ethyl acetate (x3), the combined organics washed with water, dried (Na₂SO₄), filtered and the solvent removed under reduced pressure. The crude product was purified on silica gel, eluting with a solvent gradient of 100 : 0 dichloromethane : methanol to 95 : 5 dichloromethane : methanol to yield the subtitle compound, (0.66g, 66%). MS *m*/*z* 350 (MH)⁺. ¹H-NMR (CDCl₃): δ = 2.00 (4H, m), 2.8 (3H, m), 4.15 (1H, m), [4.70 (m), 5.70 (m), 1H], 6.60-8.3 (6H, Ar).

### (c) Cis-(1S)-N-methyl-7-hydroxy-4-(3,4-dichlorophenyl)-1,2,3,4-tetrahydro-1-naphthalenamine

To a solution of the product of step (b) (0.64g) in dioxan (10ml) was added 6M aqueous hydrochloric acid (15ml) and the reaction heated at reflux for 3.5 hours. The reaction was cooled to room temperature, the solvent removed under reduced pressure and water added. The solution was made basic using concentrated aqueous ammonium hydroxide solution, extracted with ethyl acetate (x3) and the combined organics washed with brine, dried (Na₂SO₄), filtered and the solvent removed under reduced pressure. The crude product was purified on silica gel, eluting with a solvent gradient of 100 : 0 : 0 to 95 : 5 : 0.5 dichloromethane : methanol : 0.88 ammonia to yield the title compound. MS *m*/*z* 322 (MH)⁺. ¹H-NMR (CDCl₃): δ = 1.80 (1H, m), 2.00 (3H, m), 2.50 (3H, s), 3.70 (1H, t), 3.95 (1H, m), 6.60 (1H, m), 6.65 (1H, d), 6.85 (1H, d), 6.95 (1H, dd), 7.15 (1H, d), 7.30 (1H, d).

### Example 87

### Cis-(1S)-N-methyl-7-(carboxamidomethyloxy)-4-(3,4-dichlorophenyl)-1,2,3,4-tetrahydro-1-naphthalenamine hydrochloride

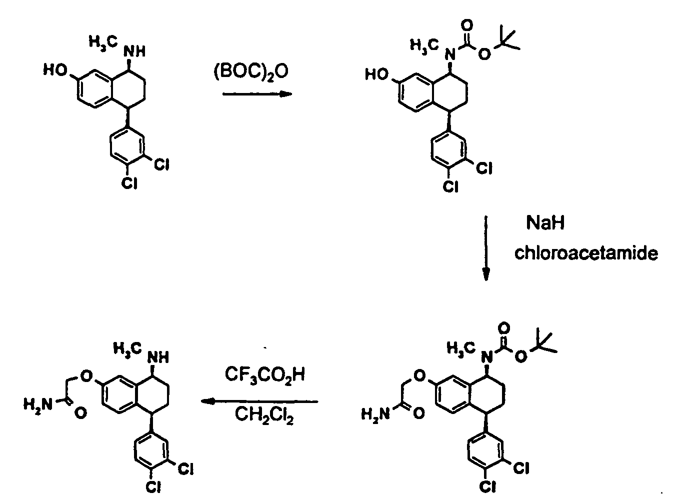

### (a) Cis-(1S)-N-methyl-N-tert-butoxycarbonyl-7-(hydroxy)-4-(3,4-dichlorophenyl)-1,2,3,4-tetrahydro-1-naphthalenamine

To a solution of the product of Example 86 (0.4g, 0.00124mol) in dichloromethane (15ml) and di-isopropylethylamine (0.23ml), was added di-tert-butyl dicarbonate (0.297g, 0.00136mol). After 22 hours, the reaction was washed aqueous citric acid solution (x3) and water (x3). The organic phase was dried (Na₂SO₄), filtered and the solvent removed under reduced pressure. The crude product was purified on silica gel eluting with 9 : 1 pentane : ethyl acetate to 5 : 1 pentane : ethyl acetate to give the subtitle compound (0.41 g). MS *m*/*z* 422 (MH)⁺. ¹H-NMR (CDCl₃): δ = 1.53 (9H, s), 1.7 (2H,m), 1.96 (1H, m), 2.21 (1H, m), 2.6 (3H, d), 4.08 (1H, s), 5.38 (1H, m), 6.51 (1H, s), 6.70 (2H, dd), 6.82 (2H, m), 7.04 (1H, d), 7.30 (1H, d).

### (b) Cis-(1S)-N-methyl-N-tert-butoxycarbonyl-7-(carboxamidomethyloxy)-4-(3,4-dichlorophenyl)-1,2,3,4-tetrahydro-1-naphthalenamine

To a solution of the product of step (a) (0.03g) in tetrahydrofuran (2ml) at room temperature was added sodium hydride (0.0025g, 80% dispersion in oil) and stirred for 10 minutes. Chloroacetamide (0.0072g) was added and the reaction heated at 50-60°C for 2 hours and then stirred at room temperature for 21 hours. Water (15ml) was added and the mixture extracted with ethyl acetate (x3). The combined organics were washed with water, dried (Na₂SO₄), filtered and the solvent removed under reduced pressure to give the subtitle compound, which was used without further purification. MS *m*/*z* 496 (MNH₄)⁺. ¹H-NMR (partial data) (CDCl₃): δ = 1.50 (9H, s), 1.74 (2H, m), 2.0 (1H, m), 2.25 (1H, m), 2.62 (3H, s), 4.14 (1H, m), 5.50 (1H, d), 6.78 (2H, m), 6.90 (1H, d), 7.07 (1H, s), 7.23 (1H, s), 7.35 (1H, d).

### (c) Cis-(1S)-N-methyl-7-(carboxamidomethyloxy)-4-(3,4-dichlorophenyl)-1,2,3,4-tetrahydro-1-naphthalenamine hydrochloride

To a solution of the product of step (b) (0.034g) in dichloromethane (0.4ml) was added trifluoroacetic acid (0.054ml). After 1.5 hours, saturated aqueous sodium bicarbonate solution was added. The aqueous layer was made basic (pH 10) with ammonia solution and extracted with dichloromethane (x2). The combined organics were washed with water, dried (Na₂SO₄), filtered and the solvent removed under reduced pressure. The crude product was purified on silica gel eluting with 93:7:1 dichloromethane:methanol:0.88 ammonia to give the free base, (0.01g). The product was dissolved in ethyl acetate (2ml) and a saturated solution of hydrogen chloride in diethyl ether added. The solvent was removed *in vacuo*, triturated with diethyl ether and dried to yield the title compound (16 mg). MS m/z 379 (MH)⁺. ¹H-NMR (CDCl₃): δ = 1.82 (1H, m), 1.9 (1H, m), 2.00 (2H, t), 2.52 (3H, s), 3.67 (1H, t), 3.95 (1H, t), 4.51 (2H, s), 5.59 (1H, s), 6.53 (1H, s), 6.74 (3H, m), 6.95 (2H, dt), 7.20 (1H, s), 7.35 (1H, d).

### Example 88

### Cis-(1S)-N-methyl-6-(carboxymethyl)-7-((methylsulphonyl)amino)-4-(3,4-dichlorophenyl)-1,2,3,4-tetrahydro-1-naphthalenamine hydrochloride

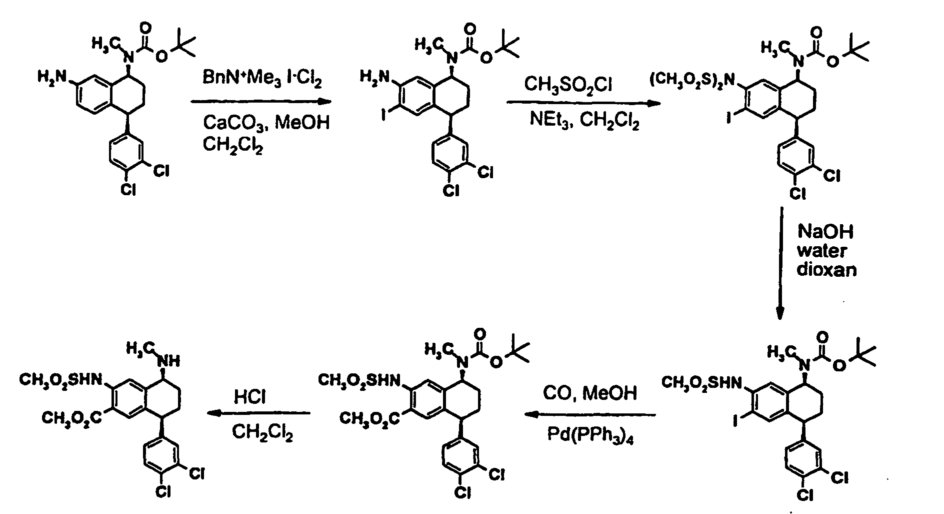

### (a) Cis-(1S)-N-methyl-N-tert-butoxycarbonyl-6-iodo-7-amino-4-(3,4-dichlorophenyl)-1,2,3,4-tetrahydro-1-naphthalenamine

To a solution of the product of Example 81(a) (1.55g, 0.00368mol) in dichloromethane (35ml) and methanol (14ml) was added calcium carbonate (0.52g, 0.00519mol) and benzyltrimethylammonium dichloroiodate (1.4g, 0.00402mol) and the reaction stirred for 4 hours. The mixture was poured into saturated aqueous sodium thiosulphate solution (50ml) and extracted with dichloromethane (x2). The organic phases were combined, dried (MgSO₄), filtered and the solvent removed under reduced pressure. The residue was purified on silica eluting with 95 : 5 to 9 : 1 pentane : ethyl acetate to give the subtitle compound, (0.867g, 43%). MS *m*/*z* 564 (MNH₄)⁺. ¹H-NMR (CDCl₃): δ = 1.50 (9H, s), 1.65 (2H, m), 1.93 (1H, m), 2.19 (1H, m), 2.62 (1.8H, s), 2.64 (1.2H, s), 4.06 (3H, m), 5.16 (0.4H, br), 5.36 (0.6H, br), 6.57 (1H, s), 6.83 (1H, m), 7.09 (1H, s), 7.12 (1H, s), 7.34 (1H, d).

### (b) Cis-(1S)-N-methyl-N-tert-butoxycarbonyl-6-(iodo)-7-((bis-methylsulphonyl)amino)-4-(3,4-dichlorophenyl)-1,2,3,4-tetrahydro-1-naphthalenamine

To a solution of the compound of step (a) (0.627g, 0.00115mol) in dichloromethane (10ml), cooled in an ice-water bath was added triethylamine (0.4ml, 0.00287mol) and methane sulphonyl chloride (0.195ml, 0.00252mol) and the reaction stirred for 17 hours.

The mixture was partitioned between dichloromethane (25ml) and water (25ml), the aqueous phase extracted with dichloromethane (15ml) and the combined organics dried (MgSO₄), filtered and the solvent removed under reduced pressure. The residue was purified on silica eluting with 85 : 15 to 4 : 1 pentane : ethyl acetate to give the subtitle compound, (0.705g, 87%). MS *m*/*z* 720 (MNH₄)⁺. ¹H-NMR (CDCl₃): δ = 1.50 (9H, s), 1.77 (2H, br), 1.99 (1H, m), 2.24 (1H, m), 2.64 (3H, s), 3.42 (2.1H, s), 3.49 (0.9H, br), 3.58 (0.9H, br), 3.63 (2.1H, s), 4.15 (1H, m), 5.23 (0.3H, br), 5.39 (0.7H, br), 6.73 (1H, br), 7.16 (1H, s), 7.37 (1H, d), 7.55 (1H, s).

### (c) Cis-(1S)-N-methyl-N-tert-butoxycarbonyl-6-(iodo)-7-((methylsulphonyl)amino)-4-(3,4-dichlorophenyl)-1,2,3,4-tetrahydro-1-naphthalenamine

To a solution of the compound of step (b) (0.705g, 0.001 mol) in dioxan (9ml) was added 2M aqueous sodium hydroxide (5ml) and the reaction stirred at room temperature for 3 hours. The dioxan was removed under reduced pressure and the residue was partitioned between water (40ml) and ethyl acetate (30ml). The aqueous phase was made acid (pH4) by the addition of 10% aqueous citric acid and extracted with ethyl acetate. The organics were washed with brine, dried (MgSO₄) and the solvent removed under reduced pressure to give the subtitle compound (0.62g, 99%). MS *m*/*z* 642 (MNH₄)⁺. ¹H-NMR (CDCl₃): δ = 1.53 (9H, s), 1.75 (2H, m), 1.96 (1H, m), 2.23 (1H, m), 2.66 (3H, s), 3.04 (3H, s), 4.12 (1H, m), 5.25 (0.35H, br), 5.36 (0.65H, br), 6.60 (1H, s), 6.80 (1H, d), 7.09 (1H, s), 7.40 (3H, m).

### (d) Cis-(1S)-N-methyl-N-tert-butoxycarbonyl-6-(methoxycarbonyl)-7-((methylsulphonyl)amino)-4-(3,4-dichlorophenyl)-1,2,3,4-tetrahydro-1-naphthalenamine

To a solution of the product of step (c) (0.283g, 0.00045mol) in methanol (10ml) was added triethylamine (0.095ml), and tetrakis(triphenylphosphine) palladium (0.026g) and the reaction heated at 80°C under an atmosphere of carbon monoxide at 690 kPa (100p.s.i.) for 4 hours. The reaction was cooled to room temperature, the solvent removed *in vacuo* and the residue purified on silica using 3 : 1 pentane : ethyl acetate to give the subtitle compound, (0.235g, 93%). MS *m*/*z* 574 (MNH₄)⁺. ¹H-NMR (CDCl₃): δ = 1.54 (9H, s), 1.77 (2H, m), 2.01 (1H, m), 2.26 (1H, m), 2.66 (3H, s), 3.06 (3H, s), 3.84 (3H, s), 4.19 (1H, m), 5.27 (0.25H, br), 5.39 (0.75H, br), 6.79 (1H, d), 7.09 (1H, s), 7.36 (1H, d), 7.55 (1H, s), 7.63 (1H, s), 10.3 (1H, s).

### (e) Cis-(1S)-N-methyl-6-(methoxycarbonyl)-7-((methylsulphonyl)amino)-4-(3,4-dichlorophenyl)-1,2,3,4-tetrahydro-1-naphthalenamine hydrochloride

Hydrogen chloride gas was bubbled through a solution of the product of step (d) (0.037g) in dichloromethane (5ml), cooled in an ice-water bath, for 10 minutes. The reaction was stirred for 40 minutes and the solvent removed under reduced pressure to yield the title compound (0.032g). MS *m*/*z* 455 (MH)⁺. ¹H-NMR (d₄-MeOH): δ = 1.99 (1H, m), 2.17 (3H, m), 2.88 (3H, s), 3.18 (3H, s), 3.83 (3H, s), 4.19 (1H, dd), 4.56 (1H, t), 7.24 (1H, dd), 7.47 (1H, d), 7.53 (1H, d), 7.61 (1H, s), 7.80 (1H, s).

### Example 89

### Cis-(1S)-N-methyl-6-(carboxy)-7-((methylsulphony)amino)-4-(3,4-dichlorophenyl)-1,2,3,4-tetrahydro-1-naphthalenamine hydrochloride

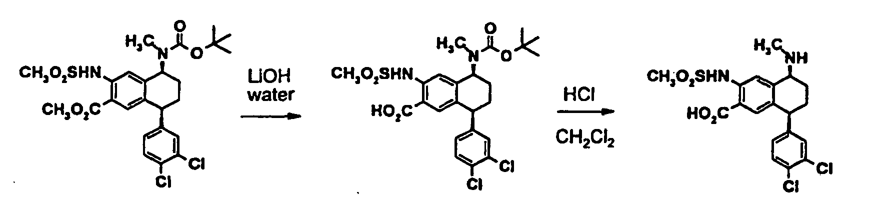

### (a) Cis-(1S)-N-methyl-N-tert-butoxycarbonyl-6-(carboxy)-7-((methylsulphonyl)amino)-4-(3,4-dichlorophenyl)-1,2,3,4-tetrahydro-1-naphthalenamine

To a solution of the product of Example 88(d) (0.185g, 0.00033mol) in tetrahydrofuran (5ml) was added water (5ml) and lithium hydroxide monohydrate (0.03g) and the reaction stirred at room temperature for 16 hours, heated at 50°C for 7 hours and then stirred at room temperature for a further 16 hours. The tetrahydrofuran was removed under reduced pressure, the residue partitioned between dichloromethane (30ml) and water (30ml) and acidified with 10% aqueous citric acid solution. The aqueous layer was washed with dichloromethane and the combined organics dried (MgSO₄), filtered and the solvent removed under reduced pressure to give the subtitle compound, (0.177g, 98%). MS *m*/*z* 560 (MNH₄)⁺. ¹H-NMR (CDCl₃): δ = 1.55 (9H, s), 1.77 (2H, m), 2.01 (1H, m), 2.26 (1H, m), 2.67 (0.6H, br), 2.72 (2.4H, s), 3.07 (3H, s), 4.18 (1H, m), 5.34 (1H, br), 6.81 (1H, d), 7.08 (1H, s), 7.36 (1H, d), 7.53 (1H, br), 7.70 (1H, br), 10.15 (0.2H, br), 10.18 (0.8H, s).

### (b) Cis-(1S)-N-methyl-6-(carboxy)-7-((methylsulphonyl)amino)-4-(3,4-dichlorophenyl)-1,2,3,4-tetrahydro-1-naphthalenamine hydrochloride

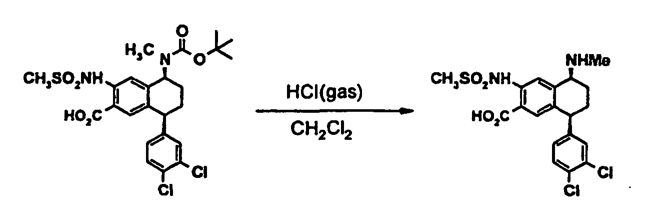

Hydrogen chloride gas was bubbled through a solution of the compound of step (a) (0.021g) in dichloromethane (5ml), cooled in an ice-water bath, for 20 minutes. The reaction was stirred for 17 hours and the solvent removed under reduced pressure to yield the title compound (0.017g, 92%). MS *m*/*z* 443 (MH)⁺. ¹H-NMR (d₄-MeOH): δ = 1.99 (1H, m), 2.26 (3H, m), 2.89 (3H, s), 3.17 (3H, s), 4.18 (1H, m), 4.55 (1H, br), 7.22 (1H, d), 7.50 (2H, m), 7.64 (1H, s), 7.79 (1H, s).

### Example 90

### Cis-(1S)-N-methyl-6-(2-(sulphonamido)ethyl)-4-(3,4-dichlorophenyl)-1,2,3,4-tetrahydro-1-naphthalenamine hydrochloride

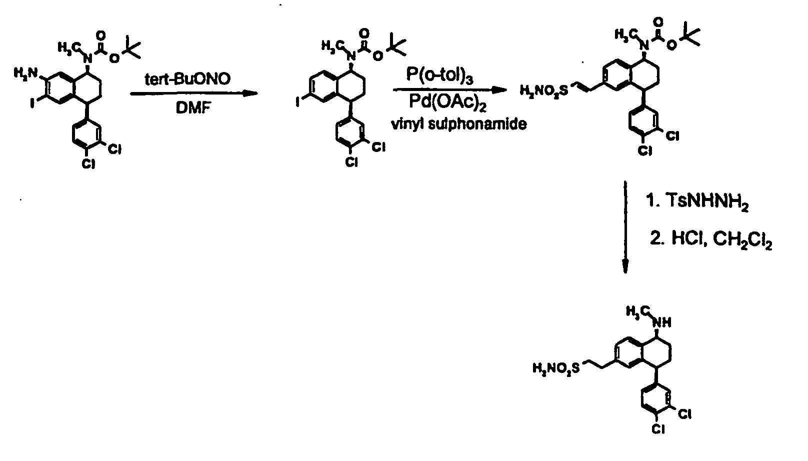

### (a) Cis-(1S)-N-methyl-N-tert-butoxycarbonyl-6-iodo-4-(3,4-dichlorophenyl)-1,2,3,4-tetrahydro-1-naphthalenamine

To N,N-dimethylformamide (1ml) at 60°C was added tert-butylnitrite (0.07ml), followed by a solution of the product of Example 88(a) (0.24g, 0.00044mol) in N,N-dimethylformamide (1ml). After 10 minutes, the reaction was cooled to room temperature, and the reaction mixture partitioned between ethyl acetate (50ml) and water (50ml). The organic phase was washed with brine, dried (MgSO₄), filtered and the solvent removed under reduced pressure. The residue was purified on silica eluting with a solvent gradient of 95 : 5 to 9: 1 pentane : ethyl acetate to yield the subtitle compound (0.134g, 57%). MS *m*/*z* 549 (MNH₄)⁺. ¹H-NMR (CDCl₃): δ = 1.51 (9H, s), 1.74 (2H, br), 1.96 (1H, m), 2.23 (1H, m), 2.62 (3H, s), 4.13 (1H, m), 5.21 (0.35H, br), 5.40 (0.65H, br), 6.79 (1H, br), 6.95 (1H, br), 7.10 (1H, s), 7.30 (1H, s), 7.36 (1H, d), 7.58 (1H, d).

### (b) Cis-(1S)-N-methyl-N-tert-butoxycarbonyl-6-(2-(sulphonamido)vinyl)-4-(3,4-dichlorophenyl)-1,2,3,4-tetrahydro-1-naphthalenamine

The subtitle compound was prepared by the general method described in Example 62(a), using the product of step (a) and vinylsulphonamide. MS *m*/*z* 528 (MNH₄)⁺. ¹H-NMR (CDCl₃): δ = 1.53 (9H, s), 1.75 (2H, br), 2.01 (1H, m), 2.27 (1H, m), 2.63 (3H, s), 4.19 (1H, m), 4.72 (2H, s), 5.28 (0.4H, br), 5.45 (0.6H, br), 6.83 (2H, m), 7.11 (3H, m), 7.35 (3H, m).

### (c) Cis-(1S)-N-methyl-N-tert-butoxycarbonyl-6-(2-(sulphonamido)ethyl)-4-(3,4-dichlorophenyl)-1,2,3,4-tetrahydro-1-naphthalenamine

The subtitle compound was prepared by the general method described in Example 62(b), using the compound of step (b). MS *m*/*z* 530 (MNH₄)⁺. ¹H-NMR (CDCl₃): δ = 1.51 (9H, s), 1.73 (2H, m), 1.98 (1H, m), 2.25 (1H, m), 2.62 (3H, s), 3.07 (2H, m), 3.34 (2H, m), 4.16 (1H, br), 4.54 (2H, br), 5.27 (0.3H, br), 5.42 (0.7H, br), 6.80 (2H, br), 7.11 (3H, m), 7.36 (1H, m).

### (d) Cis-(1S)-N-methyl-6-(2-(sulphonamido)ethyl)-4-(3,4-dichlorophenyl)-1,2,3,4-tetrahydro-1-naphthalenamine hydrochloride

The title compound was prepared by the method of Example 89(b), starting with the compound of step (c). MS *m*/*z* 413 (MH)⁺. ¹H-NMR (d₄-MeOH): δ = 1.99 (1H, m), 2.23 (3H, m), 2.83 (3H, s), 3.01 (2H, m), 3.22 (2H, m), 4.17 (1H, dd), 4.46 (1H, br), 6.83 (1H, s), 7.18 (1H, d), 7.3 (1H, d), 7.43 (1H, s), 7.53 (2H, m).

### Example 91

### Cis-(1S)-N-methyl-6-(carboxy)-4-(3,4-dichlorophenyl)-1,2,3,4-tetrahydro-1-naphthalenamine hydrochloride

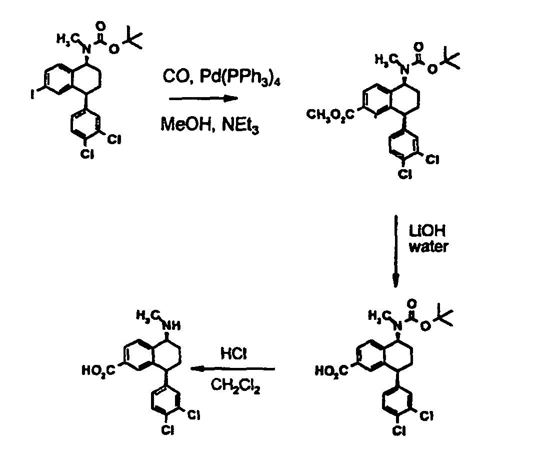

### (a) Cis-(1S)-N-methyl-N-tert-butoxycarbonyl-6-(methoxycarbonyl)-4-(3,4-dichlorophenyl)-1,2,3,4-tetrahydro-1-naphthalenamine

The subtitle compound was prepared by the method of Example 6(a), starting with the product of Example 90(a). MS *m*/*z* 481 (MNH₄)⁺. ¹H-NMR (CDCl₃): δ = 1.50 (9H, s), 1.74 (2H, br), 2.01 (1H, m), 2.27 (1H, m), 2.62 (3H, s), 3.85 (3H, s), 4.24 (1H, m), 5.29 (0.4H, br), 5.46 (0.6H, br), 6.78 (1H, br), 7.05 (1H, s), 7.30 (2H, m), 7.62 (1H, s), 7.92 (1H, d).

### (b) Cis-(1S)-N-methyl-N-tert-butoxycarbonyl-6-(carboxy)-4-(3,4-dichlorophenyl)-1,2,3,4-tetrahydro-1-naphthalenamine

The subtitle compound was prepared by the method of Example 89(a), starting with the compound of step (a). MS *m*/*z* 467 (MNH₄)⁺. ¹H-NMR (CDCl₃): δ = 1:54 (9H, s), 1.76 (2H, m), 2.02 (1H, m), 2.28 (1H, m), 2.64 (3H, s), 4.24 (1H, m), 5.31 (0.4H, br), 5.50 (0.6H, br), 6.79 (1H, m), 7.04 (1H, s), 7.33 (2H, m), 7.67 (1H, s), 7,97 (1H, d).

### (c) Cis-(1S)-N-methyl-6-(carboxy)-4-(3,4-dichlorophenyl)-1,2,3,4-tetrahydro-1-naphthalenamine hydrochloride

The title compound was prepared by the method of Example 89(b), starting with the product of Example 91(b). MS *m*/*z* 350 (MH)⁺. ¹H-NMR (d₄-MeOH): δ = 1.99 (1H, m), 2.25 (3H, m), 2.86 (3H, s), 4.24 (1H, dd), 4.56 (1H, t), 7.20 (1H, d), 7.44 (1H, s), 7.52 (1H, d), 7.59 (1H, s), 7.62 (1H, d), 7.99 (1H, d).

### Example 92

### Cis-(1S)-N-methyl-6-(methoxycarbonyl)-4-(3,4-dichlorophenyl)-1,2,3,4-tetrahydro-1-naphthalenamine hydrochloride

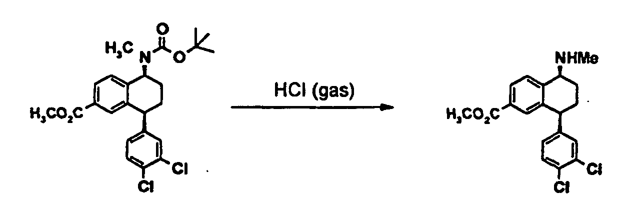

The title compound was prepared by the method of Example 89(b), starting with the product of Example 91 (a). MS *m*/*z* 364 (MH)⁺. ¹H-NMR (d₄-MeOH): δ = 2.01 (1H, m), 2.24 (3H, m), 2.86 (3H, s), 3.82 (3H, s), 4.23 (1H, dd), 4.57 (1H, br), 7.20 (1H, d), 7.43 (1H, s), 7.50 (1H, d), 7.57 (1H, s), 7.66 (1H, d), 7.98 (1H, d).

### Example 93

### Cis-(1S)-N-methyl-6-sulphonamido-4-(3,4-dichlorophenyl)-1,2,3,4-tetrahydro-1-naphthalenamine

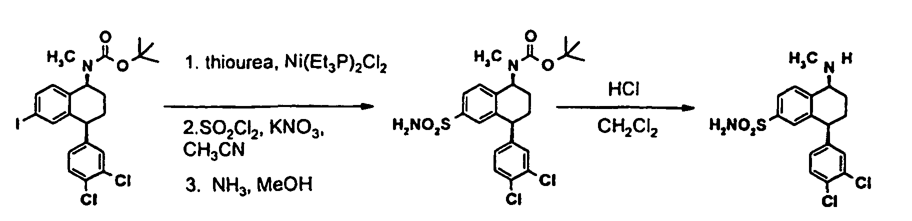

### (a) Cis-(1S)-N-methyl-N-tert-butoxycarbonyl-6-(sulphonamido)-4-(3,4-dichlorophenyl)-1,2,3,4-tetrahydro-1-naphthalenamine

To a solution of the product of Example 90(a) (0.522g, 0.001mol) and thiourea (0.114g, 0.0015mol) in N,N-dimethylformamide (2ml) was added bis(triethylphosphine)nickel-dichloride (0.0366g) and sodium cyanoborohydride (1M solution in tetrahydrofuran) (0.15ml). The reaction was heated at 60°C for 4 hours and then stirred at room temperature for 16 hours. A solution of aqueous sodium hydroxide (IN, 5ml) was added, the reaction stirred for 1 hour and made acid using 10% aqueous citric acid solution. The reaction was extracted with ethyl acetate, the organic phase washed with brine, dried (MgSO₄), filtered and the solvent removed under reduced pressure to give a crude thiol (0.49g), which was used without further purification. A solution of the crude thiol (0.1g) in acetonitrile (2ml) was cooled in an ice-water bath and sulphuryl chloride (0.08g) and powdered potassium nitrate (0.06g) added. After 1 hour, saturated aqueous sodium bicarbonate solution was added and the mixture extracted with diethyl ether. The organic layer was dried (MgSO₄), filtered and the solvent removed under reduced pressure. The crude product was dissolved in saturated ammoniacal methanol solution and allowed to stand for 16 hours. The solvent was removed under reduced pressure and the residue purified on silica eluting with 97.5 : 2.5 dichloromethane : methanol to give the subtitle compound, (0.031g). MS *m*/*z* 501 (MNH₄)⁺. ¹H-NMR (CDCl₃): δ = 1.54 (9H, s), 1.80 (2H, m), 2.06 (1H, m), 2.29 (1H, m), 2.65 (3H, s), 4.25 (1H, br), 4.80 (2H, br), 5.30 (0.45H, br), 5.47 (0.55H, br), 6.79 (1H, d), 7.08 (1H, s), 7.35 (1H, d), 7.39 (1H, d), 7.54 (1H, s), 7.80 (1H, d).

### (b) Cis-(1S)-N-methyl-6-sulphonamido-4-(3,4-dichlorophenyl)-1,2,3,4-tetrahydro-1-naphthalenamine

To a solution of the product of step (a) (9.03 1g) in dichloromethane (5ml) cooled in an ice-water bath, hydrogen chloride gas was added until the solution was saturated. After stirring for 1 hour, the solvent was removed *in vacuo* and the residue azeotroped with dichloromethane (x2) and triturated with diethyl ether. The solvent was removed under reduced pressure and the residue purified on silica eluting with 93:7:1 dichloromethane:methanol:0.88 ammonia, to give the title compound (0.15g). MS *m*/*z* 385 (MH)⁺. ¹H-NMR (CDCl₃): δ = 1.86 (1H, m), 2.00 (1H, m), 2.07 (2H, m), 2.54 (3H, s), 3.75 (1H, t), 4.04 (1H, t), 4.70 (2H, br), 6.94 (1H, dd), 7.21 (1H, d), 7.39 (1H, d), 7.40 (1H, s), 7.55 (1H, d), 7.74 (1H, dd).

### Example 94

### (5S,8S)-8-(3,4-Dichlorophenyl)-5-(methylamino)-5,6,7,8-tetrahydro-2-naphthalenecarboxamide hydrochloride

### (a) tert-Butyl (1S,4S)-6-[(1H-1,2,3-benzotriazol-1-yloxy)carbonyl]-4-(3,4-dichlorophenyl)-1,2,3,4-tetrahydro-1-naphthalenyl(methyl)carbamate

To a solution of the compound of Example 91 (b) (0.155g, 0.00034mol) in N,N-dimethylformamide (2.5ml) at 0°C was added triethylamine (0.055ml, 0.00039mol) and TBTU (0.12g, 0.00037mol) and the reaction stirred for 1 hour.

### (b) tert-Butyl (1S,4S)-6-(aminocarbonyl)-4-(3,4-dichlorophenyl)-1,2,3,4-tetrahydro-1-naphthalenyl(methyl)carbamate

A portion of the solution prepared in step (a) (0.6 ml) was added to a stirred solution of 0.88 ammonia (1ml) and the reaction stirred for 16 hours. The reaction mixture was diluted with ethyl acetate (20ml) and washed with 10% aqueous citric acid solution (20ml) and brine, dried (MgSO₄), filtered and the solvent removed under reduced pressure. The residue was purified on silica gel, eluting with ethyl acetate : pentane (1:1) to give the subtitle compound, (0.037g). MS *m*/*z* 466 (MNH₄)⁺. ¹H NMR (CDCl₃); δ = 1.50 (9H, s), 1.77 (2H, m), 2.02 (1H, m), 2.29 (1H, m), 2.64 (3H, s), 4.24 (1H, m), 5.30-5.47 (1H, br), 5.63 (1H, br), 5.87 (1H, br), 6.79 (1H, m), 7.06 (1H, s), 7.33 (2H, m), 7.43 (1H, s), 7.67 (1H, d).

### (c) (5S,8S)-8-(3,4-Dichlorophenyl)-5-(methylamino)-5,6,7,8-tetrahydro-2-naphthalenecarboxamide hydrochloride

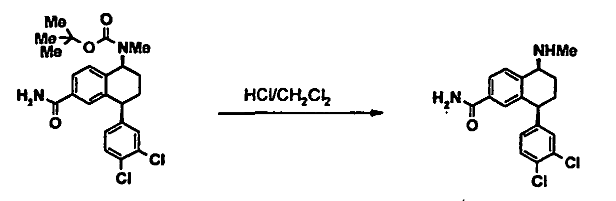

To the product of step (b) (0.037g) was added a saturated solution of HCl in dichloromethane at 0°C. After 70 minutes, the solvent was removed *in vacuo* to yield the title compound (0.028g). MS *m*/*z* 349 (MH)⁺. ¹H NMR (CD₃OD); δ = 2.02 (1H, m), 2.24 (3H, m), 2.87 (3H, s), 4.24 (1H, m), 4.55 (1H, m), 7.19 (1H, d), 7.43 (1H, s), 7.50 (2H, m), 7.64 (1H, d), 7.83 (1H, d).

### Example 95

### (5S,8S)-8-(3,4-Dichlorophenyl)-N-(2-hydroxyethyl)-5-(methylamino)-5,6,7,8-tetrahydro-2-naphthalenecarboxamide hydrochloride

### (a) tert-Butyl (1S,4S)-4-(3,4-dichlorophenyl)-6-{[(2-hydroxyethyl)amino}carbonyl}-1,2,3,4-tetrahydro-1-naphthalenyl(methyl)carbamate

The subtitle compound was prepared from a portion of the solution produced in Example 94(a), using the method of Example 94(b), but using ethanolamine as the amine in place of ammonia. MS *m*/*z* 514 (MNa)⁺. ¹H NMR (CDCl₃); δ = 1.52 (9H, s), 1.76 (1H, m), 2.10 (1H, m), 2.27 (2H, m), 2.62 (3H, s), 3.58 (2H, m), 3.80 (2H, t), 4.23 (1H, m), 5.30-5.46 (1H, br), 6.58 (1H, br, t), 6.79 (1H, d), 7.04 (1H, s), 7.33 (3H, m), 7.65 (1H, d).

### (b) (5S,8S)-8-(3,4-dichlorophenyl)-N-(2-hydroxyethyl)-5-(methylamino)-5,6,7,8-tetrahydro-2-naphthalenecarboxamide hydrochloride

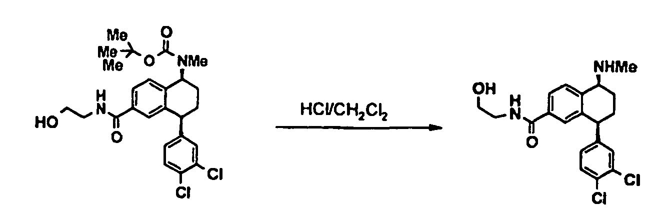

The title compound was prepared from the product of step (a), using the method of Example 94(c). MS *m*/*z* 393 (MH)⁺. ¹H NMR (CD₃OD); δ = 2.01 (1H, m), 2.25 (3H, m), 2.87 (3H, s), 3.43 (2H, m), 3.64 (2H, t), 4.25 (1H, dd), 4.55 (1H, m), 7.19 (1H, dd), 7.41 (1H, d), 7.46 (1H, s), 7.50 (1H, d), 7.63 (1H, d), 7.82 (1H, d).

### Example 96

### 3-[(5S,8S)-8-(3,4-Dichlorophenyl)-5-(methylamino)-5,6,7,8-tetrahydro-2-naphthalenyl]propanoic acid hydrochloride

### (a) Ethyl (E)-3-[(5S,8S)-5-[(tert-butoxycarbonyl)(methyl)amino]-8-(3,4-dichlorophenyl)-5,6,7,8-tetrahydro-2-naphthalenyl]-2-propenoate

To a solution of the compound of Example 90(a) (0.217g, 0.00041mol) in acetonitrile (10ml) was added triethylamine (0.17ml, 0.00122mol), tri-o-tolylphosphine (0.05g, 0.00016mol), palladium acetate (0.0018g, 0.00008mol) and ethyl acrylate (0.05ml) and the mixture heated under reflux for 75 minutes. The reaction was allowed to cool to room temperature and poured into a mixture of ethyl acetate (30ml) and water (30ml). The organic layer was washed with brine, dried (MgSO₄), filtered and the solvent removed under reduced pressure. The residue was purified on silica eluting with a solvent gradient of ethyl acetate/pentane [5:95] to ethyl acetate/pentane [7.5:92.5] to give the subtitle compound (0.163g). MS *m*/*z* 522 (MNH₄)⁺. ¹H NMR (CDCl₃); δ = 1.32 (3H, t), 1.54 (9H, s), 1.76 (2H, m), 2.10 (1H, m), 2.26 (1H, m), 2.63 (3H, s), 4.12 (3H, m), 5.28-5.46 (1H, br), 6.34 (1H, d), 6.79 (1H, br), 7.10 (2H, s), 7.23 (1H, d), 7.78 (1H, d).

### (b) Ethyl 3-[(5S,8S)-5-[(tert-butoxycarbonyl)(methyl)amino]-8-(3,4-dichlorophenyl)-5,6,7,8-tetrahydro-2-naphthalenyl]propanoate

To a solution of the product of step (a) (0.163g, 0.00032mol) in toluene (10ml) was added tosyl hydrazine (0.3g, 0.00161mol) and the reaction heated at reflux for 5 hours. The reaction was cooled, the solution decanted and concentrated *in vacuo* to give a residue which was purified on silica eluting with ethyl acetate/pentane [7.5:92.5] solution to give the subtitle compound (0.125g). MS *m*/*z* 523 (MNH₄)⁺. ¹H NMR (CDCl₃); δ = 1.20 (3H, t), 1.50 (9H, s), 1.71 (2H, m), 1.95 (1H, m), 2.24 (1H, m), 2.55 (2H, t), 2.60 (3H, s), 2.85 (2H, t), 4.08 (2H, q), 4.15 (1H, m), 5.27-5.43 (1H, m), 6.78 (2H, m), 7.08 (3H, m), 7.33 (1H, d).

### (c) 3-[(5S,8S)-5-[(tert-Butoxycarbonyl)(methyl)amino]-8-(3,4-dichlorophenyl)-5,6,7,8-tetrahydro-2-naphthalenyl]propanoic acid

To a solution of the product of step (b) (0.125g, 0.00025mol) in tetrahydrofuran (5ml) was added water (5ml) and lithium hydroxide (0.025g, 0.0006mol) and the reaction stirred at room temperature for 2.5 hours and then at 50°C for 2 hours. The reaction was cooled to room temperature, acidified with 10% aqueous citric acid and extracted with ethyl acetate. The organic layer was washed with brine, dried (MgSO₄), filtered and the solvent removed under reduced pressure to give the subtitle compound (0.118g). MS *m*/*z* 495 (MNH₄)⁺. ¹H NMR (CDCl₃); δ = 1.51 (9H, s), 1.72 (2H, m), 2.00 (1H, m), 2.27 (1H, m), 2.62 (5H, m), 2.87 (2H, t), 4.16 (1H, m), 5.28-5.43 (1H, m), 6.80 (2H, m), 7.10 (2H, m), 7.33 (1H, d).

### (d) 3-[(5S,8S)-8-(3,4-Dichlorophenyl)-5-(methylamino)-5,6,7,8-tetrahydro-2-naphthalenyl]propanoic acid hydrochloride

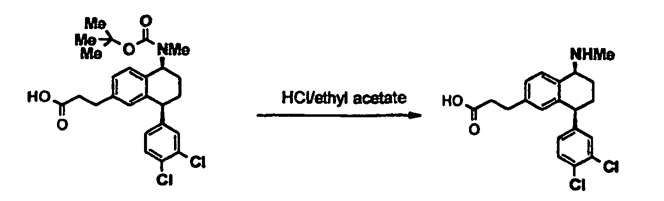

HCl gas was bubbled through a solution of the product of step (c) (0.02g) in ethyl acetate (5ml) at 0°C for 15 minutes. After 45 minutes, the reaction was concentrated *in vacuo* to give the title compound. MS *m*/*z* 378 (MH)⁺. ¹H NMR (CD₃OD); δ = 1.96 (1H, m), 2.20 (3H, m), 2.48 (2H, t), 2.81 (5H, m), 4.16 (1H, m), 4.44 (1H, m), 6.80 (1H, s), 7.17 (1H, d), 7.24 (1H, s), 7.41 (2H, m), 7.48 (1H, d).

### Example 97

### (5S,8S)-8-(3,4-Dichlorophenyl)-N-methyl-5-(methylamino)-5,6,7,8-tetrahydro-2-naphthalenecarboxamide hydrochloride

### (a) tert-Butyl (1S,4S)-4-(3,4-dichlorophenyl)-6-[(methylamino)carbonyl]-1,2,3,4-tetrahydronaphthalenyl(methyl)carbamate

The subtitle compound was prepared from a portion of the solution prepared in Example 94(a) (0.6ml, 0.000083mol), using the method of Example 94(b), but using methylamine (1ml, 2M solution in tetrahydrofuran) as the amine in place of ammonia. Yield = 0.035g. MS *m*/*z* 480 (MNH₄)⁺. ¹H NMR (CDCl₃); δ = 1.51 (9H, s), 1.76 (2H, m), 2.01 (1H, m), 2.28 (1H, m), 2.61 (3H, s), 2.96 (3H, d), 4.22 (1H, m), 5.30-5.48 (1H, m), 6.03 (1H, s), 6.79 (1H, m), 7.04 (1H, s), 7.27 (1H, m), 7.36 (2H, m), 7.64 (1H, d).

### (b) (5S,8S)-8-(3,4-Dichlorophenyl)-N-methyl-5-(methylamino)-5,6,7,8-tetrahydro-2-naphthalenecarboxamide hydrochloride

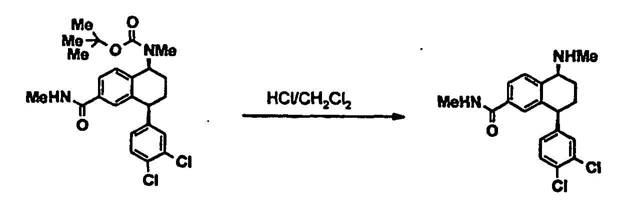

The title compound was prepared from the product of step (a), using the method of Example 94(c). MS *m*/*z* 363 (MH)⁺. ¹H NMR (CD₃OD); δ = 2.02 (1H, m), 2.24 (3H, m), 2.83 (3H, s), 2.87 (3H, s), 4.24 (1H, m), 4.56 (1H, m), 7.20 (1H, d), 7.41 (1H, s), 7.43 (1H, s), 7.50 (1H, d), 7.65 (1H, d), 7.79 (1H, d).

### Example 98

### (5S,8S)-8-(3,4-Dichlorophenyl)-N,N-dimethyl-5-(methylamino)-5,6,7,8-tetrahydro-2-naphthalenecarboxamide hydrochloride

### (a) tert-Butyl (1S,4S)-4-(3,4-dichlorophenyl)-6-[(dimethylamino)carbonyl]-1,2,3,4-tetrahydro-1-naphthalenyl(methyl)carbamate

The subtitle compound was prepared from a portion of the solution produced in Example 94(a) (0.6ml, 0.000083mol), using the method of Example 94(b), but using dimethylamine (1ml, 2M solution in tetrahydrofuran) as the amine in place of ammonia. Yield = 0.033g. MS *m*/*z* 494 (MNH₄)⁺. ¹H NMR (CDCl₃); δ = 1.50 (9H, m), 2.01 (1H, m), 2.27 (1H, m), 2.63 (3H, s), 2.93 (3H, s), 3.05 (3H, s), 4.19 (1H, m), 5.28-5.47 (1H, m), 6.79 (1H, d), 7.00 (1H, s), 7.08 (1H, s), 7.23 (1H, m), 7.33 (2H, m).

### (b) (5S,8S)-8-(3,4-Dichlorophenyl)-N,N-dimethyl-5-(methylamino)-5,6,7,8-tetrahydro-2-naphthalenecarboxamide hydrochloride

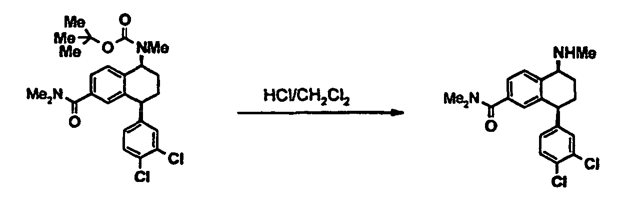

The title compound was prepared from the product of step (a), using the method of Example 94(c). MS *m*/*z* 377 (MH)⁺. ¹H NMR (CD₃OD); δ = 2.02 (1H, m), 2.26 (3H, m), 2.86 (6H, s), 3.00 (3H, s), 4.22 (1H, m), 4.54 (1H, m), 6.92 (1H, s), 7.22 (1H, d), 7.40 (1H, d), 7.45 (1H, s), 7.65 (1H, d).

### Example 99

### 2-{(5S,8S)-8-(3,4-Dichlorophenyl)-5-(methylamino)-3-[(methylsulphonyl)amino]-5,6,7,8-tetrahydro-2-naphthalenyl}-1-ethanesulphonamide

### (a) tert-Butyl (1S,4S)-6-[(E)-2-(aminosulphonyl)ethenyl]-4-(3,4-dichlorophenyl)-7-[(methylsulphonyl)amino]-1,2,3,4-tetrahydro-1-naphthalenyl(methyl)carbamate

To a solution of the product of Example 88(c) (0.112g, 0.00018mol) in acetonitrile (5ml) was added triethylamine (0.075ml, 0.00054mol), tri-o-tolylphosphine (0.022g, 0.000072mol), palladium acetate (0.0008g, 0.0000036mol) and vinylsulphonamide (0.024g, 0.00022mol) and the mixture heated under reflux for 3.5 hours. The reaction was allowed to cool to room temperature and poured into a mixture of ethyl acetate (30ml) and water (30ml). The organic layer was washed with brine, dried (MgSO₄), filtered and the solvent removed under reduced pressure. The residue was purified on silica eluting with ethyl acetate/pentane (1:1) to give the subtitle compound (0.057g). MS *m*/*z* 626 (MNa)⁺. ¹H NMR (CDCl₃); δ = 1.52 (9H, s), 1.77 (2H, m), 2.10 (1H, m), 2.25 (1H, m), 2.66 (3H, s), 3.07 (3H, s), 4.18 (1H, m), 5.25 (2H, br), 5.40 (1H, m), 6.79 (2H, m), 7.10 (1H, s), 7.14 (1H, s), 7.30 (1H, s), 7.37 (1H, d), 7.76 (1H, d).

### (b) tert-Butyl (1S,4S)-6-[2-(aminosulphonyl)ethyl]-4-(3,4-dichlorophenyl)-7-[(methylsulphonyl)amino]-1,2,3,4-tetrahydro-1-naphthalenyl(methyl)carbamate

To a solution of the product of step (a) (0.05 1g, 0.000084mol) in toluene (5ml) was added tosyl hydrazine (0.078g, 0.00042mol) and the reaction heated at reflux for 17 hours. The reaction was cooled, the solution decanted and concentrated in vacuo to give a residue which was purified on silica eluting with ethyl acetate/dichloromethane [40:60] solution to give the title compound (0.01g). MS *m*/*z* 628 (MNa)⁺. ¹H NMR (CDCl₃); δ = 1.51 (9H, s), 1.73 (2H, m), 1.99 (1H, m), 2.24 (1H, m), 2.64 (3H, s), 3.08 (3H, s), 3.18 (2H, m), 3.36 (2H, t), 4.15 (1H, m), 4.94 (2H, br), 5.26-5.38 (1H, m), 6.76 (1H, m), 6.87 (1H, s), 6.93 (1H, br), 7.08 (1H, s), 7.17 (1H, s), 7.36 (1H, d).

### (c) 2-{(5S,8S)-8-(3,4-Dichlorophenyl)-5-(methylamino)-3-[(methylsulphonyl)amino]-5,6,7,8-tetrahydro-2-naphthalenyl}-1-ethanesulphonamide

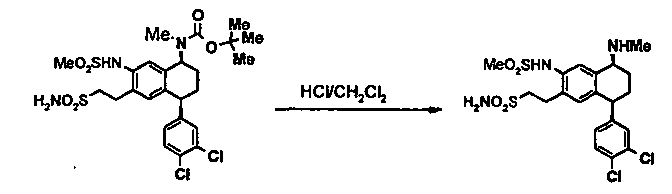

The title compound was prepared from the product of step (b), using the method described in Example 94(c). MS *m*/*z* 506 (MH)⁺. ¹H NMR (CD₃OD); δ = 1.98 (1H, m), 2.22 (3H, m), 2.84 (3H, s), 3.13 (5H, m), 3.22 (2H, m), 4.19 (1H, m), 4.48 (1H, m), 6.93 (1H, s), 7.20 (1H, d), 7.45 (1H, s), 7.50 (1H, d), 7.57 (1H, s).

### Example 100

### (5S,8S)-8-(3,4-Dichlorophenyl)-5-(methylamino)-3-[(methylsulphonyl)amino]-5,6,7,8-tetrahydro-2-naphthalenecarboxamide hydrochloride

### (a) tert-Butyl (1S,4S)-6-(aminocarbonyl)-4-(3,4-dichlorophenyl)-7-[(methylsulphonyl)amino]-1,2,3,4-tetrahydro-1-naphthalenyl(methyl)carbamate

To a solution of the compound of Example 89(a) (0.065g, 0.00012mol) in dioxan (5ml) at room temperature was added triethylamine (0.025ml, 0.00018mol), HOBT (0.022g, 0.00014mol), EDC hydrochloride (0.034g, 0.00018mol) and ammonium carbonate (0.023g, 0.00024mol). After 16 hours, the solvent was removed *in vacuo* and the residue partitioned between ethyl acetate (30ml) and water (15ml). The organic phase was washed with water, brine, dried (MgSO₄), filtered and the solvent removed *in vacuo* to yield a residue which was purified on silica using a solvent gradient of ethyl acetate/pentane [1:1] to ethyl acetate/pentane [3:2] to yield the subtitle compound (0.05g). MS *m*/*z* 559 (MNH₄)⁺. ¹H NMR (CDCl₃); δ = 1.53 (9H, s), 1.74 (2H, m), 1.99 (1H, m), 2.17 (1H, m), 2.68 (3H, s), 3.05 (3H, s), 4.08 (1H, m), 5.30 (1H, m), 5.77 (1H, m), 6.19 (1H, br), 6.78 (1H, d), 7.09 (1H, s), 7.15 (1H, s), 7.35 (1H, d), 7.48 (1H, s), 10.63 (1H, s).

### (b) (5S,8S)-8-(3,4-Dichlorophenyl)-5-(methylamino)-3-[(methylsulphonyl)amino]-5,6,7,8-tetrahydro-2-naphthalenecarboxamide hydrochloride

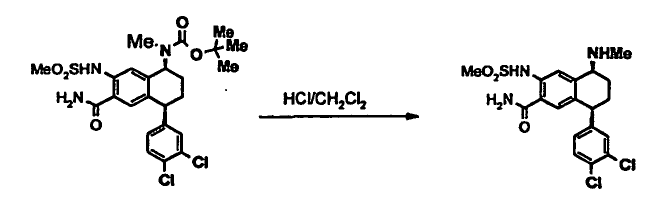

The title compound was prepared from the product of step (a), using the method of Example 94(c). MS *m*/*z* 422 (MH)⁺. ¹H NMR (CD₃OD); δ = 1.97 (1H, m), 2.21 (3H, m), 2.87 (3H, s), 3.09 (3H, s), 4.21 (1H, dd), 4.53 (1H, m), 7.18 (1H, dd), 7.40 (1H, s), 7.44 (1H, d), 7.50 (1H, d), 7.77 (1H, s).

### Example 101

### (5S,8S)-8-(3,4-Dichlorophenyl)-N-(2-hydroxyethyl)-5-(methylamino)-3-[(methylsulphonyl)amino]-5,6,7,8-tetrahydro-2-naphthalenecarboxamide

### (a) tert-Butyl (1S,4S)-4-(3,4-dichlorophenyl)-6-{[(2-hydroxyethyl)amino]carbonyl}-7-[(methylsulphonyl)amino]-1,2,3,4-tetrahydro-1-naphthalenyl(methyl)carbamate

The subtitle compound was prepared from the product of Example 89(a), using the method of Example 100(a), but using ethanolamine in place of ammonium carbonate. MS *m*/*z* 603 (MNH₄)⁺. ¹H NMR (CDCl₃); δ = 1.52 (9H, s), 1.74 (2H, m), 2.00 (1H, m), 2.10 (1H, br), 2.15 (1H, m), 2.66 (3H, s), 3.04 (3H, s), 3.53 (2H, m), 3.77 (2H, m), 4.18 (1H, m), 5.27-5.37 (1H, m), 6.58 (1H, t), 6.79 (1H, d), 7.10 (2H, m), 7.36 (1H, d), 7.49 (1H, s), 10.42 (0.3H, br), 10.53 (0.7H, br).

### (b) (5S,8S)-8-(3,4-dichlorophenyl)-N-(2-hydroxyethyl)-5-(methylamino)-3-[(methylsulphonyl)amino]-5,6,7,8-tetrahydro-2-naphthalenecarboxamide

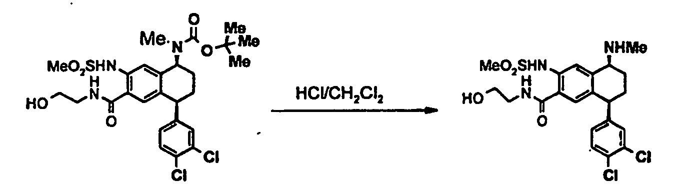

The title compound was prepared from the product of step (a), using the method of Example 94(c). MS *m*/*z* 486 (MH)⁺. ¹H NMR (CD₃OD); δ = 1.98 (1H, m), 2.20 (3H, m), 2.87 (3H, s), 3.40 (2H, m), 3.61 (2H, t), 4.21 (1H, m), 4.52 (1H,m), 7.16 (1H, d), 7.33 (1H, s), 7.42 (1H, s), 7.50 (1H, d), 7.75 (1H, s).

### Example 102

### (5S,8S)-8-(3,4-dichlorophenyl)-N,N-dimethyl-5-(methylamino)-3-[(methylsulphonyl)amino]-5,6,7,8-tetrahydro-2-naphthalenecarboxamide

### (a) tert-Butyl (1S,4S)-4-(3,4-dichlorophenyl)-6-[(dimethylamino)carbonyl]-7-[(methylsulphonyl)amino]-1,2,3,4-tetrahydro-1-naphthalenyl(methyl)carbamate

The subtitle compound was prepared from the product of Example 89(a), using the method of Example 100(a), but using a solution of dimethylamine in ethanol in place of ammonium carbonate. MS *m*/*z* 587 (MNH₄)⁺. ¹H NMR (CDCl₃); δ = 1.53 (9H, s), 1.75 (2H, m), 2.00 (1H, m), 2.28 (1H, m), 2.67 (3H, s), 2.98 (6H, br), 3.07 (3H, s), 4.14 (1H, m), 5.28-5.41 (1H, m), 6.78 (1H, d), 6.85 (1H, s), 7.08 (1H, s), 7.32 (1H, d), 7.40 (1H, s), 8.04 (1H, br).

### (b) (5S,8S)-8-(3,4-Dichlorophenyl)-N,N-dimethyl-5-(methylamino)-3-[(methylsulphonyl)amino]-5,6,7,8-tetrahydro-2-naphthalenecarboxamide

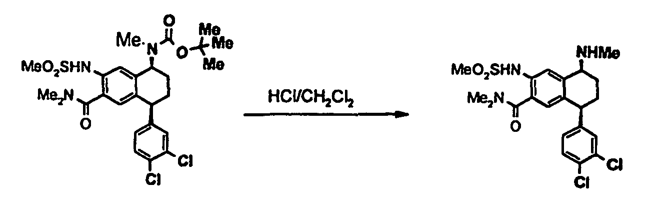

The title compound was prepared from the product of step (a), using the method of Example 94(c). MS *m*/*z* 470 (MH)⁺. ¹H NMR (CD₃OD); δ = 1.96 (1H, m), 2.26 (3H, m), 2.79 (3H, s), 2.88 (3H, s), 3.01 (3H, s), 3.08 (3H, s), 4.19 (1H, dd), 4.50 (1H, dd), 6.82 (1H, s), 7.20 (1H, dd), 7.44 (1H, d), 7.51 (1H, d), 7.67 (1H, s).

### Example 103

### 3-[(5S,8S)-8-(3,4-Dichlorophenyl)-5-(methylamino)-5,6,7,8-tetrahydro-2-naphthalenyl]propanamide hydrochloride

### (a) tert-Butyl (1S,4S)-6-[3(1H-1,2,3-benzotriazol-1-yloxy)-3-oxopropyl]-4-(3,4-dichlorophenyl)-1,2,3,4-tetrahydro-1-naphthalenyl(methyl)carbamate

To a solution of the compound of Example 96(c) (0.098g, 0.0002mol) in acetonitrile (3ml) in an ice-water bath was added EDC hydrochloride (0.045g, 0.00023mol) and HOBT (0.035g, 0.00023mol). After 5 minutes, the reaction was warmed to room temperature and stirred for 0.5 hours.

### (b) tert-Butyl (1S,4S)-6-(3-amino-3-oxopropyl)-4-(3,4-dichlorophenyl)-1,2,3,4-tetrahydro-1-naphthalenyl(methyl)carbamate

To a portion of the solution produced in step (a) (1ml) was added a solution of 0.88 ammonia (1ml) and EDC hydrochloride (0.02g, 0.0001mol) and the reaction stirred for 3 hours. The reaction mixture was diluted with ethyl acetate (20ml) and washed with 10% aqueous citric acid solution (20ml) and brine, dried (MgSO₄), filtered and the solvent removed under reduced pressure. The residue was purified on silica gel, eluting with a gradient of ethyl acetate/pentane [3:1] to neat ethyl acetate to give the title compound, (0.03g). MS *m*/*z* 494 (MNH₄)⁺. ¹H NMR (CDCl₃); δ = 1.50 (9H, s), 1.70 (2H, m), 1.98 (1H, m), 2.23 (1H, m), 2.45 (2H, t), 2.60 (3H, s), 2.87 (2H, t), 4.13 (1H, m), 5.27-5.40 (2H, m), 5.53 (1H, br), 6.80 (2H, m), 7.02 (1H, s), 7.10 (2H, m), 7.32 (1H, d).

### (c) 3-[(5S,8S)-8-(3,4-Dichlorophenyl)-5-(methylamino)-5,6,7,8-tetrahydro-2-naphthalenyl]propanamide hydrochloride

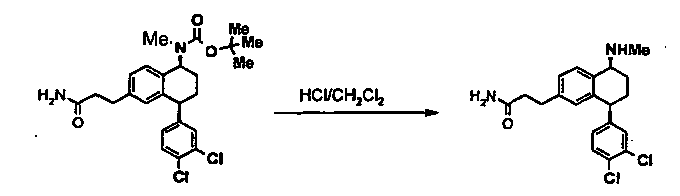

The title compound was prepared from the product of step (b), using the method of Example 94(c). MS *m*/*z* 377 (MH)⁺. ¹H NMR (CD₃OD); δ = 1.95 (1H, m), 2.20 (3H, m), 2.40 (2H, t), 2.81 (5H, m), 4.16 (1H, dd), 4.44 (1H, m), 6.80 (1H, s), 7.16 (1H, dd), 7.25 (1H, d), 7.43 (2H, m), 7.50 (1H, d).

### Example 104

### 3-[(5S,8S)-8-(3,4-Dichlorophenyl)-5-(methylamino)-5,6,7,8-tetrahydro-2-naphthalenyl]-N-methylpropanamide hydrochloride

### (a) tert-Butyl (1S,4S)-4-(3,4-dichlorophenyl)-6-[3-(methylamino)-3-oxopropyl]-1,2,3,4-tetrahydro-1-naphthalenyl(methyl)carbamate

To a solution of the compound of Example 103(a) (1ml) was added a 2M solution of methylamine in tetrahydrofuran (1ml) and the reaction stirred for 3 hours. The reaction mixture was diluted with ethyl acetate (20ml) and washed with 10% aqueous citric acid solution (20ml) and brine, dried (MgSO₄), filtered and the solvent removed under reduced pressure. The residue was purified on silica gel, eluting with gradient of ethyl acetate : pentane (1:1) to (3:1) ethyl acetate : pentane to give the title compound, (0.017g). MS *m*/*z* 509 (MNH₄)⁺. ¹H NMR (CDCl₃); δ = 1.50 (9H, s), 1.70 (2H, m), 1.97 (1H, m), 2.24 (1H, m), 2.39 (2H, m), 2.61 (3H, s), 2.71 (3H, d), 2.87 (2H, m), 4.13 (1H, m), 5.27-5.42 (2H, m), 6.78 (2H, m), 7.02 (1H, s), 7.11 (2H, m), 7.33 (1H, d).

### (b) 3-[(5S,8S)-8-(3,4-Dichlorophenyl)-5-(methylamino)-5,6,7,8-tetrahydro-2-naphthalenyl]-N-methylpropanamide hydrochloride

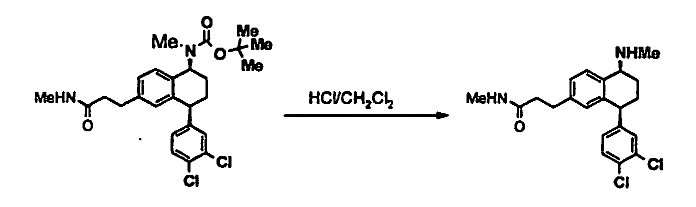

The title compound was prepared from the product of step (a), using the method of Example 94(c). MS *m*/*z* 391 (MH)⁺. ¹H NMR (CD₃OD); δ = 1.97 (1H, m), 2.20 (3H, m), 2.35 (2H, m), 2.60 (3H, s), 2.80 (5H, m), 4.16 (1H, m), 4.43 (1H, m), 6.75 (1H, s), 7.18 (1H, d), 7.22 (1H, d), 7.47 (3H, m).

### Example 105

### 3-[(5S,8S)-8-(3,4-Dichlorophenyl)-5-(methylamino)-5,6,7,8-tetrahydro-2-naphthalenyl]-N-(2-hydroxyethyl)propanamide

### (a) tert-Butyl (1S,4S)-4-(3,4-dichlorophenyl)-60{3-[(2-hydroxyethyl)amino]-3-oxopropyl}-1,2,3,4-tetrahydro-1-naphthalenyl(methyl)carbamate

To a solution of the compound of Example 103(a) (1ml) was added tetrahydrofuran (1ml) and ethanolamine (0.1ml, 0.00166mol) and the reaction stirred for 3 hours. The reaction mixture was diluted with ethyl acetate (20ml) and washed with 10% aqueous citric acid solution (20ml) and brine, dried (MgSO₄), filtered and the solvent removed under reduced pressure. The residue was purified on silica gel, eluting with a gradient of ethyl acetate : pentane (9:1) to 100% ethyl acetate to give the subtitle compound, (0.022g). MS *m*/*z* 521 (MNH₄)⁺. ¹H NMR (CDCl₃); δ = 1.49 (9H, s), 1.70 (2H, m), 1.96 (1H, m), 2.23 (2H, m), 2.41 (2H, m), 2.61 (3H, s), 2.87 (2H, m), 3.32 (2H, m), 3.61 (2H, m), 4.12 (1H, m), 5.25-5.38 (1H, m), 5.78 (1H, br), 6.78 (2H, m), 7.07 (3H, m), 7.32 (1H, d).

### (b) 3-[(5S,8S)-8-(3,4-Dichlorophenyl)-5-(methylamino)-5,6,7,8-tetrahydro-2-naphthalenyl]-N-(2-hydroxyethyl)propanamide

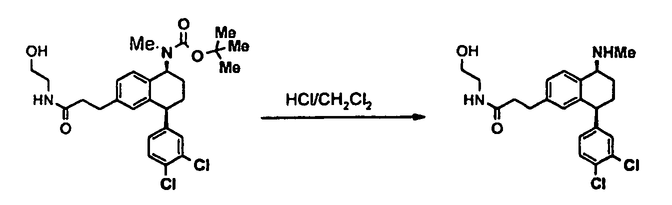

The title compound was prepared from the product of step (a), using the method of Example 94(c). MS *m*/*z* 421 (MH)⁺. ¹H NMR (CD₃OD); δ = 1.93 (1H, m), 2.20 (3H, m), 2.36 (2H, t), 2.80 (5H, m), 3.18 (2H, t), 3.50 (2H, t), 4.13 (1H, dd), 4.44 (1H, m), 6.79 (1H, s), 7.17 (1H, dd), 7.24 (1H, d), 7.41 (2H, m), 7.49 (1H, d).

### Example 106

### 3-{(5S,8S)-8-(3,4-Dichlorophenyl)-5-(methylamino)-3-[(methylsulphonyl)amino]-5,6,7,8-tetrahydro-2-naphthalenyl}propanamide hydrochloride

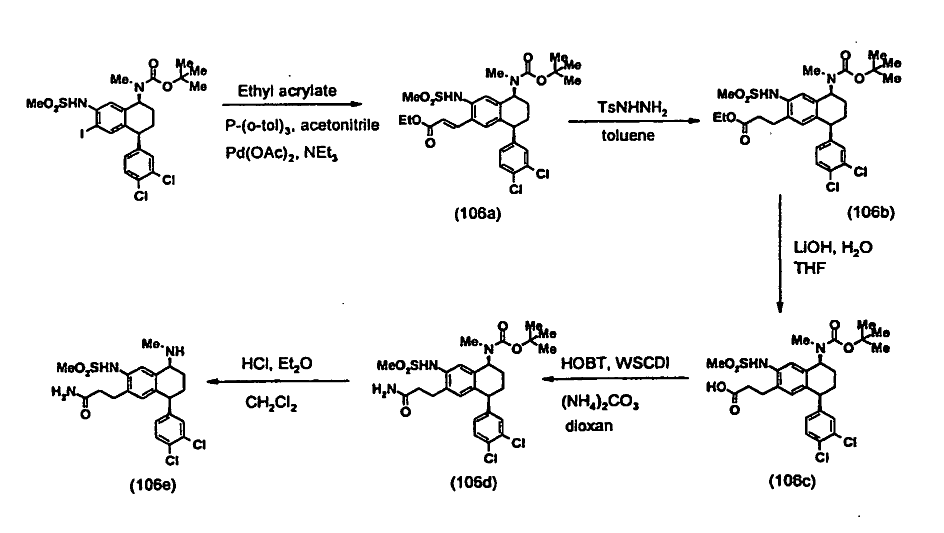

### (a) Ethyl (E)-3-{(5S,8S)-5-[(tert-butoxycarbonyl)(methyl)amino]-8-(3,4-dichlorophenyl)-3-[(methylsulphonyl)amino]-5,6,7,8-tetrahydro-2-naphthalenyl}-2-propenoate

To a solution of the product of Example 88(c) (0.2g, 0.32 mmol) in acetonitrile (10ml) was added triethylamine (135µl), tri-o-tolyl phosphine (38mg), palladium acetate (14mg) and ethyl acrylate (40µl, 0.37 mmol) and the reaction heated under reflux for 2.5 hours. The reaction was cooled to room temperature and poured into ethyl acetate (50ml) washed with 10% aqueous citric acid solution, water and brine. The organic phase was dried (MgSO₄), filtered and the solvent removed under reduced pressure. The crude product was purified on silica gel, eluting with a solvent gradient of ethyl acetate/pentane [25:75] to [35:65] to yield the subtitle compound (172mg, 90%). MS *m*/*z* 614 (MNH₄)⁺. ¹H NMR (CDCl₃); δ = 1.25 (3H, t), 1.51 (9H, s), 1.77 (2H, m), 2.00 (1H, m), 2.25 (1H, m), 2.65 (3H, s), 3.03 (3H, s), 4.21 (3H, m), 5.27-5.40 (1H, m), 6.26 (1H, d), 6.80 (2H, m), 7.10 (2H, m), 7.20 (1H, s), 7.33 (1H, m), 7.84 (1H, m).

### (b) Ethyl 3-{(5S,8S)-5-[(tert-butoxycarbonyl)(methyl)amino]-8-(3,4-dichlorophenyl)-3-[(methylsulphonyl)amino]-5,6,7,8-tetrahydro-2-naphthalenyl}propanoate

To a solution of the product of step (a) (172mg, 0.29mmol) in toluene (10ml) was added tosylhydrazine (300mg) and the reaction heated under reflux for 5 hours. The reaction was cooled to room temperature and then placed in a freezer for 16 hours. The solution was decanted from the precipitate and concentrated under reduced pressure. The crude product was purified on silica gel, eluting with a solvent gradient of ethyl acetate/dichloromethane [5:95] to [7.5:92.5]. MS *m*/*z* 616 (MNH₄)⁺. ¹H NMR (CDCl₃); δ = 1.20 (3H, t), 1.72 (2H, m), 1.96 (1H, m), 2.23 (1H, m), 2.61 (2H, m), 2.63 (3H, s), 2.84 (2H, m), 3.05 (3H, s), 4.10 (3H, m), 5.23-5.40 (1H, m), 6.78 (2H, m), 7.06 (1H, s), 7.24 (1H, m), 7.34 (1H, m), 7.85 (1H, m).

### (c) 3-{(5S,8S)-5-[(tert-Butoxycarbonyl)(methyl)amino]-8-(3,4-dichlorophenyl)-3-[(methylsulphonyl)amino]-,6,7,8-tetrahydro-2-naphthalenyl}propanoic acid

To a solution of the product of step (b) (124mg, 0.21 mmol) in tetrahydrofuran (5ml) was added aqueous lithium hydroxide solution (1M solution, 2ml) and the reaction stirred at 45°C for 22 hours. The reaction was cooled to room temperature, the tetrahydrofuran removed under reduced pressure and the residue partitioned between dichloromethane and water and acidified with 10% aqueous citric acid solution. The organic layer was dried (MgSO₄), filtered and the solvent removed under reduced pressure to give the subtitle compound (105mg, 89%). ¹H NMR (CDCl₃); δ = 1.48 (9H, s), 1.97 (1H, m), 2.23 (1H, m), 2.62 (5H, m), 2.76-3.03 (5H, m), 4.12 (1H, m), 5.14-5.40 (1H, m), 6.78 (2H, m), 7.08 (1H, s), 7.22 (1H, s), 7.34 (1H, d), 7.83 (1H, br).

### (d) tert-Butyl (1S,4S)-6-(3-amino-3-oxopropyl)-4-(3,4-dichlorophenyl)-7-[(methylsulphonyl)amino]-1,2,3,4-tetrahydro-1-naphthalenyl(methyl)carbamate

To a solution of the product of step (c) (37mg, 0.065mmol) in dioxan (3ml) was added hydroxybenzotriazole (11mg), EDC hydrochloride (19mg) and ammonium carbonate 19mg) and the reaction stirred for 3 hours. The reaction mixture was diluted with ethyl acetate, washed with 10% aqueous citric acid solution and brine, dried (MgSO₄), filtered and the solvent removed under reduced pressure. The residue was purified on silica gel, eluting with a solvent gradient of ethyl acetate/pentane [1:1] to neat ethyl acetate to give the subtitle compound (62%). MS *m*/*z* 592 (MNa)⁺. ¹H NMR (CDCl₃); δ = 1.50 (9H, s), 1.71 (2H, m), 1.97 (1H, m), 2.22 (1H, m), 2.54 (2H, m), 2.64 (3H, s), 2.76-3.95 (2H, m), 3.04 (3H, s), 4.10 (1H, m), 5.23-5.40 (1H, m), 5.56 (1H, br), 5.63 (1H, br), 6.75 (1H, s), 6.80 (1H, dd), 7.06 (1H, s), 7.25 (1H, m), 7.35 (1H, d), 8.79 (1H, br).

### (e) {(5S,8S)-8-(3,4-Dichlorophenyl)-5-(methylamino)-3-[(methylsulphonyl)amino]-5,6,7,8-tetrahydro-2-naphthalenyl}propanamide hydrochloride

The title compound was prepared from the product of step (d), using the method of Example 94(c). MS *m*/*z* 470. ¹H NMR (CD₃OD); δ = 1.94 (1H, m), 2.22 (3H, m), 2.43 (2H, t), 2.85 (5H, m), 3.07 (3H, s), 4.16 (1H, dd), 4.44 (1H,m), 6.85 (1H, s), 7.19 (1H, dd), 7.43 (1H, d), 7.50 (1H, d), 7.55 (1H, s).

### Example 107

### 3-{(5S,8S)-5-(3,4-Dichlorophenyl)-8-(methylamino)-2-[(methylsulphonyl)amino]-5,6,7,8-tetrahydro-1-naphthalenyl}-propanamide hydrochloride

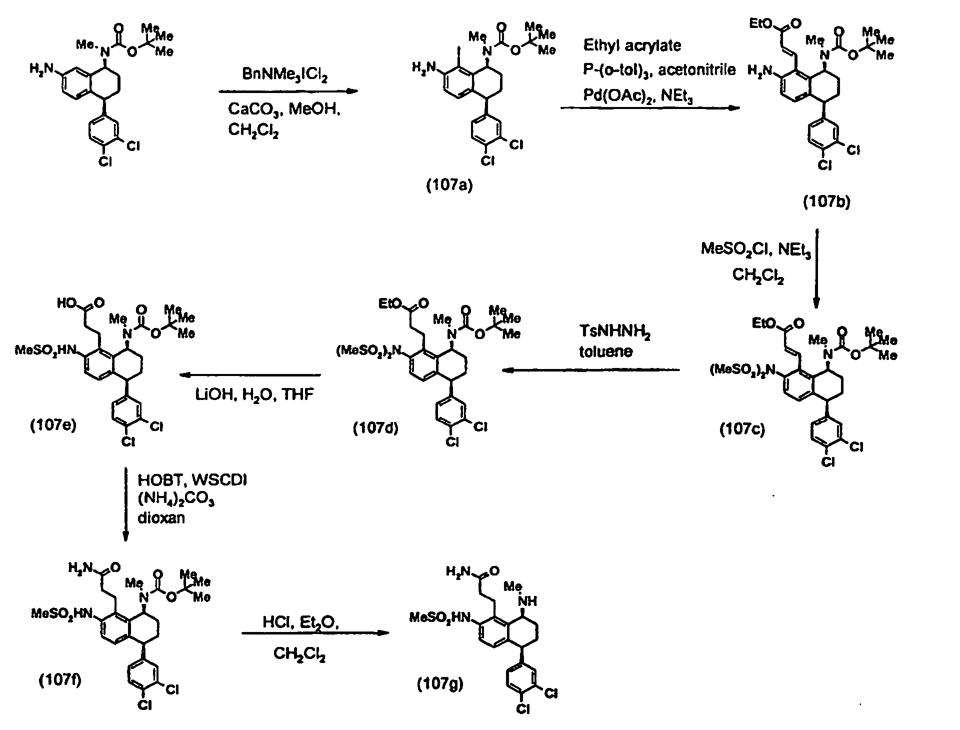

### (a) tert-Butyl (1S,4S)-7-amino-4-(3,4-dichlorophenyl)-8-iodo-1,2,3,4-tetrahydro-1-naphthalenyl(methyl)carbamate

To a solution of the product of Example 81(a) (24.85g, 59mmol) in dichloromethane (500ml) and methanol (200ml), cooled in an ice-water bath, was added calcium carbonate (11.4g, 114 mmol) and benzyltrimethylammonium dichloroiodate (30.8g, 88.5mmol). After 6h, the reaction was poured into 5% aqueous sodium thiosulphate solution (800ml). The organic phase was washed with 5% aqueous thiosulphate solution, dried (MgSO₄), filtered and the solvent removed under reduced pressure. The residue was purified on silica gel, eluting with diethyl ether:pentane to give the subtitle compound. MS *m*/*z* 547 (MH)⁺. ¹H NMR (CDCl₃); δ = 1.50-1.57 (9H, m), 1.77 (1H, m), 1.88 (1H, m), 1.98 (1H, m), 2.61 (3H, m), 3.90 (1H,m), 4.24 (2H, s), 5.00-5.14 (1H,m), 6.62 (2H, m), 6.95 (1H, m), 7.21 (1H, d), 7.38 (1H, dd).

### (b) Ethyl (E)-3-[(5S,8S)-2-amino-8-[(tert-butoxycarbonyl)(methyl)amino]-5-(3,4-dichlorophenyl)-5,6,7,8-tetrahydro-1-naphthalenyl]-2-propenoate

The subtitle compound was prepared by the method of Example 106(a), starting with the product of step (a) above. Yield = 69%. MS *m*/*z* 519 (MH)⁺. ¹H NMR (CDCl₃); δ = 1.32 (3H, m), 1.43-1.51 (9H, m), 1.84 (2H, m), 1.97 (1H, m), 2.03 (1H, m), 2.53-2.56 (3H, m), 3.94 (3H, m), 4.22 (2H, m), 5.19-5.37 (1H, m), 6.23 (1H, m), 6.60 (1H, m), 6.67 (1H, m), 6.94 (1H, m), 7.20 (1H, m), 7.35 (1H, m), 7.62 (1H, m).

### (c) Ethyl (E)-3-[(5S,8S)-2-[bis(methylsulphonyl)amino]-8-[(tert-butoxycarbonyl)(methyl)amino]-5-(3,4-dichlorophenyl)-5,6,7,8-tetrahydro-1-naphthalenyl]-2-propenoate

The subtitle compound was prepared from the product of step (b) above, using the method of Example 88(b). MS *m*/*z* 692 (MH)⁺.

### (d) Ethyl 3-[(5S,8S)-2-[(bis(methylsulphonyl)amino]-8-[(tert-butoxycarbonyl)(methyl)amino]-5-(3,4-dichlorophenyl)-5,6,7,8-tetrahydro-1-naphthalenyl]propanoate

The subtitle compound was prepared from the product of step (c) above, using the method of Example 106(b) using above compound (c). Yield = 40%. MS *m*/*z* 699 (MNa)⁺. ¹H NMR (CDCl₃); δ = 1.25 (3H, m), 1.52 (9H, m), 1.95 (2H, m), 2.05 (1H, m), 2.19 (1H, m), 2.43 (1H, m), 2.58 (3H, s), 2.62-2.77 (1H, m), 2.93 (1H, m), 3.23 (1H, m), 3.40 (3H, s), 3.61 (3H, s), 4.01 (1H, m), 4.17 (2H, m), 5.30-5.47 (1H, m), 6.84 (1H, d), 6.96 (1H, d), 7.17 (1H, d), 7.23 (1H, m), 7.41 (1H, d).

### (e) 3-{(5S,8S)-8-[(tert-Butoxycarbonyl)(methyl)amino]-5-(3,4-dichlorophenyl)-2-[(methylsulphonyl)amino]-5,6,7,8-tetrahydro-1-naphthalenyl}propanoic acid

The subtitle compound was prepared from the product of step (d) above, using the method of Example 106(c). Yield = 61%. MS *m*/*z* 593 (MNa)⁺. ¹H NMR (CDCl₃); δ = 1.49-1.55 (9H, m), 1.90 (2H, m), 2.03 (1H, m), 2.13 (1H, m), 2.62 (5H, m), 2.87 (1H, m), 3.03 (4H, m), 3.97 (1H, m), 5.17-5.43 (1H, m), 6.79 (1H, d), 6.96 (1H, m), 7.23 (1H, m), 7.37 (2H, m).

### (f) tert-Butyl (1S,4S)-8-(3-amino-3-oxopropyl)-4-(3,4-dichlorophenyl)-7-[(methylsulphonyl)amino]-1,2,3,4-tetrahydro-1-naphthalenyl(methyl)carbamate

The subtitle compound was prepared from the product of step (e) above, using the method of Example 106(d). Yield = 22%. MS *m*/*z* 592 (MNa)⁺. ¹H NMR (CDCl₃); δ = 1.49-1.56 (9H, m), 1.92 (2H, m), 2.03 (1H, m), 2.13 (1H, m), 2.37-3.14 (10H, m), 3.97 (1H, m), 5.28-5.41 (1H, m), 5.49-5.72 (2H, m), 6.79 (1H, m), 6.97 (1H, d), 7.23 (1H, m), 7.38 (2H, m), 9.50-9.66 (1H, m).

### (g) 3-{(5S,8S)-5-(3,4-Dichlorophenyl)-8-(methylamino)-2-[(methylsulphonyl)amino]-5,6,7,8-tetrahydro-1-naphthalenyl}-propanamide hydrochloride

The title compound was prepared from the product of step (f), using the method of Example 94(c). Yield = 100%. MS *m*/*z* 470 (MH)⁺. ¹H NMR (CD₃OD); δ = 1.93 (1H, m), 2.14 (2H, m), 2.53 (1H, m), 2.70 (1H, m), 2.94 (3H, s), 3.04 (3H, s), 3.17 (3H, m), 4.17 (1H, dd), 4.78 (1H, m), 6.81 (1H, d), 7.22 (1H, dd), 7.30 (1H, d), 7.46 (1H, d), 7.52 (1H, d).

### Example 108

### (1S,4S)-4-(3,4-Dichlorophenyl)-N-methyl-6-(methylsulphanyl)-1,2,3,4-tetrahydro-1-naphthalenamine

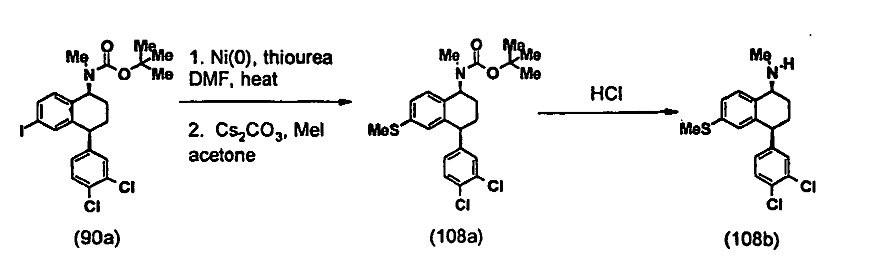

### (a) tert-Butyl (1S,4S)-4-(3,4-dichlorophenyl)-6-(methylsulphanyl)-1,2,3,4-tetrahydro-1-naphthalenyl(methyl)carbamate

To a solution of the product of Example 90(a) (532mg, 1mmol) in N,N-dimethylformamide (2ml) was added thiourea (114mg), (Et₃P)₂NiCl₂ (37mg) followed by sodium cyanoborohydride (0.15ml, 1M solution in tetrahydrofuran) and the reaction heated at 60°C for 4h. The reaction was allowed to cool to room temperature and 1N aqueous sodium hydroxide solution (5ml) added. After 1h, 10% aqueous citric acid solution was added and the mixture extracted with ethyl acetate, the organic phase washed with brine, dried (MgSO₄), filtered and the solvent removed under reduced pressure to give the crude thiol (490mg).

To a solution of the above thiol (343mg) in acetone (15ml) was added caesium carbonate (325mg) followed by methyl iodide (142 mg). After 1h, water was added and the mixture extracted with ethyl acetate. The organic phase was washed with water, dried (MgSO₄), filtered and the solvent removed under reduced pressure. The residue was purified on silica gel, eluting with ethyl acetate:pentane, to give the subtitle compound (181mg, 51%). MS *m*/*z* 453 (MH)⁺. ¹H NMR (CDCl₃); δ = 1.50 (9H, s), 1.70 (2H, m), 1.98 (1H, m), 2.23 (1H, m), 2.40 (3H, s), 2.62 (3H, s), 4.12 (1H, s), 5.26-5.41 (1H, m), 6.80 (2H, s), 7.13 (3H, m), 7.33 (1H, d).

### (b) (1S,4S)-4-(3,4-Dichlorophenyl)-N-methyl-6-(methylsulphanyl)-1,2,3,4-tetrahydro-1-naphthalenamine

The subtitle compound was prepared from the product of step (a), using the method of Example 94(c). MS *m*/*z* 351 (MH)⁺. ¹H NMR (CDCl₃); δ = 1.81 (1H, m), 2.00 (3H, m), 2.35 (3H, s), 2.53 (3H, s), 3.70 (1H, s), 3.96 (1H, t), 6.70 (1H, s), 6.96 (1H, d), 7.11 (1H, d), 7.28 (1H, m), 7.32 (1H, d), 7.37 (1H, d).

### Example 109

### (1S,4S)-4-(3,4-Dichlorophenyl)-N-methyl-6-(methylsulphinyl)-1,2,3,4-tetrahydro-1-naphthalenamine

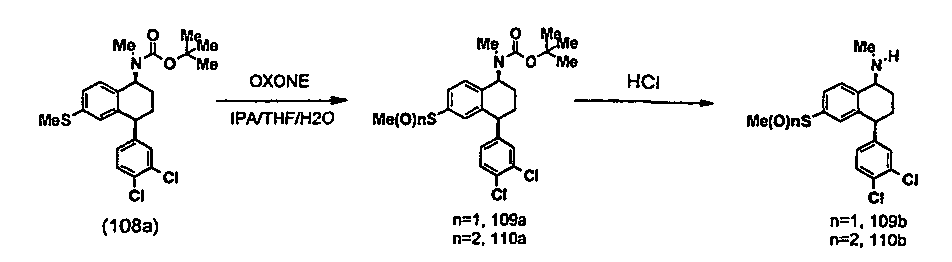

### (a) tert-Butyl (1S,4S)-4-(3,4-dichlorophenyl)-6-(methylsulphinyl)-1,2,3,4-tetrahydro-1-naphthalenyl(methyl)carbamate

To a solution of the product of Example 108(a) (60mg, 0.13mmol) in isopropanol : tetrahydrofuran : water (10:2:1) (3ml), cooled in an ice-water bath, was added OXONE™ (potassium peroxymonosulphate, 50mg) in portions. After 2 hours, water was added and the solution made basic with 2N NaOH. The mixture was extracted with ethyl acetate, the organic phase washed with brine, dried (MgSO₄), filtered and the solvent removed under reduced pressure. The crude product was purified on silica gel, eluting with ethyl acetate to give the subtitle compound (55mg, 89%). MS *m*/*z* 485 (MNH₄)⁺. ¹H NMR (CDCl₃); δ = 1.51 (9H, s), 1.79 (2H, m), 2.02 (1H, m), 2.30 (1H, m), 2.63 (3H, s), 2.68 (3H, s), 4.26 (1H, m), 5.30-5.50 (1H, m), 6.79 (1H, d), 7.05 (1H, d), 7.2-7.6 (4H, m).

### (b) (1S,4S)-4-(3,4-Dichlorophenyl)-N-methyl-6-(methylsulphinyl)-1,2,3,4-tetrahydro-1-naphthalenamine

The title compound was prepared from the product of step (a), using the method of Example 94(c). MS *m*/*z* 368 (MH)⁺. ¹H NMR (CDCl₃); δ = 1.87 (1H, m), 2.06 (3H, m), 2.54 (3H, s), 2.63 (3H, s), 3.78 (1H, d), 4.05 (1H, q), 6.95 (1H, m), 7.09 (1H, d), 7.23 (1H, s), 7.37 (1H, dd), 7.50 (1H, d), 7.59 (1H, m).

### Example 110

### (1S,4S)-4-(3,4-Dichlorophenyl)-N-methyl-6-(methylsulphonyl)-1,2,3,4-tetrahydro-1-naphthalenamine

### (a) tert-Butyl (1S,4S)-4-(3,4-dichlorophenyl)-6-(methylsulphonyl)-1,2,3,4-tetrahydro-1-naphthalenyl(methyl)carbamate

The subtitle compound was prepared from the product of Example 108(a), using the method of Example 109(a), but using 2 equivalents of OXONE™, and allowing the reaction to warm to room temperature. MS *m*/*z* 501 (MNH₄)⁺. ¹H NMR (CDCl₃); δ = 1.53 (9H, s), 1.80 (2H, m), 2.06 (1H, m), 2.31 (1H, m), 2.67 (3H, s), 3.03 (3H, s), 4.29 (1H, s), 5.32.-5.49 (1H, br), 6.78 (1H, d), 7.07 (1H, s), 7.37 (1H, d), 7.43 (1H, d), 7.58 (1H, s), 7.82 (1H, d).

### (b) (1S,4S)-4-(3,4-Dichlorophenyl)-N-methyl-6-(methylsulphonyl)-1,2,3,4-tetrahydro-1-naphthalenamine

The title compound was prepared from the product of step (a), using the method of Example 94(c). MS *m*/*z* 384 (MH)⁺. ¹H NMR (CDCl₃); δ = 1.89 (1H, m), 2.08 (3H, m), 2.56 (3H, s), 2.96 (3H, s), 3.80 (1H, t), 4.08 (1H, t), 6.94 (1H, d), 7.22 (1H, s), 7.39 (1H, d), 7.43 (1H, s), 7.65 (1H, d), 7.77 (1H, d).

### Example 111

### 2-[(5S,8S)-5-(3,4-Dichlorophenyl)-8-(methylamino)-5,6,7,8-tetrahydro-2-naphthalenyl]acetic acid

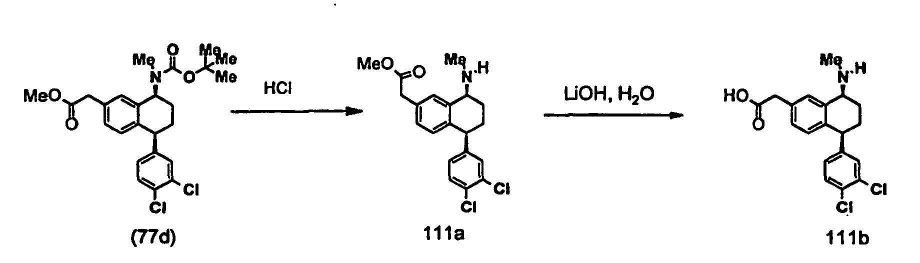

### (a) Methyl 2-[(5S,8S)-5-(3,4-dichlorophenyl)-8-(methylamino)-5,6,7,8-tetrahydro-2-naphthalenyl]acetate

The subtitle compound was prepared from the product of Example 77(d), using the method of Example 94(c). MS *m*/*z* 379 (MH)⁺. ¹H NMR (CDCl₃); δ = 1.81 (1H, m), 2.00 (3H, m), 2.53 (3H, s), 3.60 (2H, s), 3.70 (4H, s), 3.94 (1H, dd), 6.75 (1H, d), 6.95 (1H, d), 7.05 (1H, d), 7.26 (2H, m), 7.34 (1H, d).

### (b) 2-[(5S,8S)-5-(3,4-Dichlorophenyl)-8-(methylamino)-5,6,7,8-tetrahydro-2-naphthalenyl]acetic acid

The title compound was prepared from the product of step (a), using the method of Example 77(e). MS *m*/*z* 365 (MH)⁺. ¹H NMR (d₆-DMSO); δ = 1.73 (1H, m), 1.92 (3H, m), 2.39 (3H, s), 3.43 (2H, s), 3.68 (1H, d), 4.03 (1H, dd), 6.60 (1H, d), 6.99 (1H, d), 7.14 (1H, d), 7.29 (1H, s), 7.42 (1H, s), 7.52 (1H, d).

### Example 112

### 3-[(5S,8S)-5-(3,4-Dichlorophenyl)-8-(methylamino)-5,6,7,8-tetrahydro-1-naphthalenyl]-N-methyl propanamide

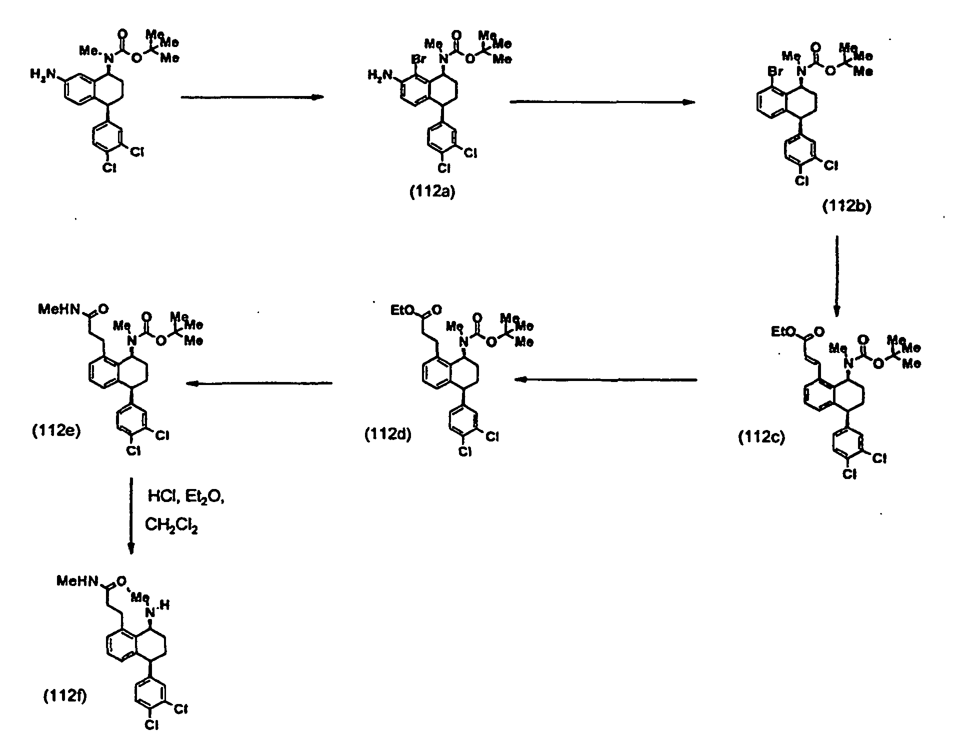

### (a) tert-Butyl (1S,4S)-7-amino-8-bromo-4-(3,4-dichlorophenyl)-1,2,3,4-tetrahydro-1-naphthalenyl(methyl)carbamate

To a solution of the product of Example 81(a) (632 mg, 1.5 mmol) in N,N-dimethylformamide (3 ml) cooled in an ice-water bath was added a solution of *N*-bromosuccinimide (280 mg, 1.05 equiv) in N,N-dimethylformamide (3 ml) and the reaction stirred at room temperature for 2 hours. The mixture was diluted with Et₂O (10 ml) and EtOAc (10 ml), washed with H₂O and the organic phase dried (MgSO₄), filtered and the solvent removed under reduced pressure. The residue was purified on silica gel, eluting with hexane : EtOAc to give the subtitle compound (300 mg, 40%). MS *m*/*z* 500 (MH)⁺. ¹H-NMR (CDCl₃): δ = 1.53, (9H, s), 1.75-2.02 (3H, m), 2.18 (1H, br), 2.62 (3H, d), 3.91 (1H, m), 4.19 (2H, s), 5.19-5.30 (1H, br), 6.61 (2H, m), 6.98 (1H, t), 7.22 (1H, d), 7.37 (1H, d).

### (b) tert-Butyl (1S,4S)-8-bromo-4-(3,4-dichlorophenyl)-1,2,3,4-tetrahydro-1-naphthalenyl(methyl)carbamate

To a solution of t-butyl nitrite (35µl) in N,N-dimethylformamide (0.5 ml) at 60°C was added a solution of the compound of step (a) (100 mg, 0.2 mmol) and the mixture stirred for 10 min. The reaction was cooled and partitioned between ethyl acetate and water. The organic phase was dried (MgSO₄), filtered and the solvent removed under reduced pressure. The residue was purified on silica gel eluting with ethyl acetate:hexane to give the subtitle compound. This material, contaminated with about 30% of the 3,4-dehydro elimination product, could be used without further purification.

To obtain a pure sample, HCl gas was bubbled through a solution of the above crude bromide (269 mg) in dichloromethane, cooled in an ice-water bath, until the solution was saturated. The reaction was stirred for 30 min, the solvent removed under reduced pressure and the residue azeotroped with dichloromethane. This product was purified on silica gel, eluting with dichloromethane:methanol:ammonia to give 8-bromosertraline (107 mg, 50%). MS *m*/*z* 383 (MH)⁺. ¹H-NMR (d₆-DMSO): δ = 1.55 (1H, t), 1.72 (1H, br), 1.84 (1H, br), 2.11 (2H, br), 2.41 (3H, s), 3.70 (1H, s), 4.08 (1H, m), 6.66 (1H, d), 7.01 (1H, t), 7.18 (1H, d), 7.45 (2H, m), 7.57 (1H, d).
The above amine was then converted to 112(b) using (BOC)₂O, (1.5 equiv), DMAP (0.1 equiv) and di-isopropylethylamine(1.5 equiv), in tetrahydrofuran (75%). ¹H-NMR (CDCl₃): δ = 1.54 (9H, s), 1.90 (2H, m), 2.04 (1H, br), 2.19 (1H, br) 2.60 (3H, s), 3.99 (1H, br), 5.25-5.35 (1H, br), 6.83 (1H, d), 6.96 (1H, m), 7.02 (1H, t), 7.23 (1H, m), 7.39 (1H, d), 7.44 (1H, d).

### (c) Ethyl (E)-3-[(5S,8S)-8-[(tert-butoxycarbonyl)(methyl)amino]-5-(3,4-dichlorophenyl)-5,6,7,8-tetrahydro-1-naphthalenyl]-2-propenoate and Ethyl (Z)-3-[(5S,8S)-8-[(tert-butoxycarbonyl)(methyl)amino]-5-(3,4-dichlorophenyl)-5,6,7,8-tetrahydro-1-naphthalenyl]-2-propenoate

The subtitle compounds were prepared from the product of step (b), using the method of Example 106(a). MS *m*/*z* 503 (MH)⁺.

### (d) Ethyl 3-[(5S,8S)-8-[(tert-butoxycarbonyl)(methyl)amino]-5-(3,4-dichlorophenyl)-5,6,7,8-tetrahydro-1-naphthalenyl]propanoate

The subtitle compound was prepared from the products of step (c), using the method of Example 106(b). ¹H-NMR (CDCl₃): δ = 1.26 (3H, t), 1.55 (9H, s), 1.92 (2H, m), 2.08 (2H, br), 2.55 (2H, m), 2.60 (3H, s), 2.84-3.00 (2H, m), 4.01 (1H, br), 4.13 (2H, q), 5.32-5.46 (1H, m), 6.75 (1H, d), 6.96 (1H, t), 7.11 (2H, m), 7.23 (1H, m), 7.37 (1H, d).

### (e) tert-Butyl (1S,4S)-4-(3,4-dichlorophenyl)-8-[3-(methylamino)-3-oxopropyl]-1,2,3,4-tetrahydro-1-naphthalenyl(methyl)carbamate

A solution of the compound of Example 112(d) (110mg) in 33% methylamine in ethanol solution (2.5ml) was heated at 110°C for 4h. The reaction was cooled to room temperature, the solvent removed under reduced pressure and the crude product purified on silica gel, eluting with ethyl acetate:hexane to give the subtitle compound (73mg). MS *m*/*z* 490 (MH)⁺.

### (f) 3-[(5S,8S)-5-(3,4-Dichlorophenyl)-8-(methylamino)-5,6,7,8-tetrahydro-1-naphthalenyl]-N-methyl propanamide

The title compound was prepared from the product of step (e), using the method of Example 94(c) using above compound (e). ¹H-NMR (CDCl₃): δ = 1.66 (1H, t), 1.95 (1H, m), 2.01 (1H, m), 2.28 (1H, d), 2.56 (3H, s), 2.62 (2H, q), 2.77 (3H, s), 3.04 (2H, t), 3.86 (1H, s), 3.95 (1H, m), 6.20 (1H, br), 7.00 (1H, d), 7.07 (2H, m), 7.27 (1H, s), 7.35 (12H, d).

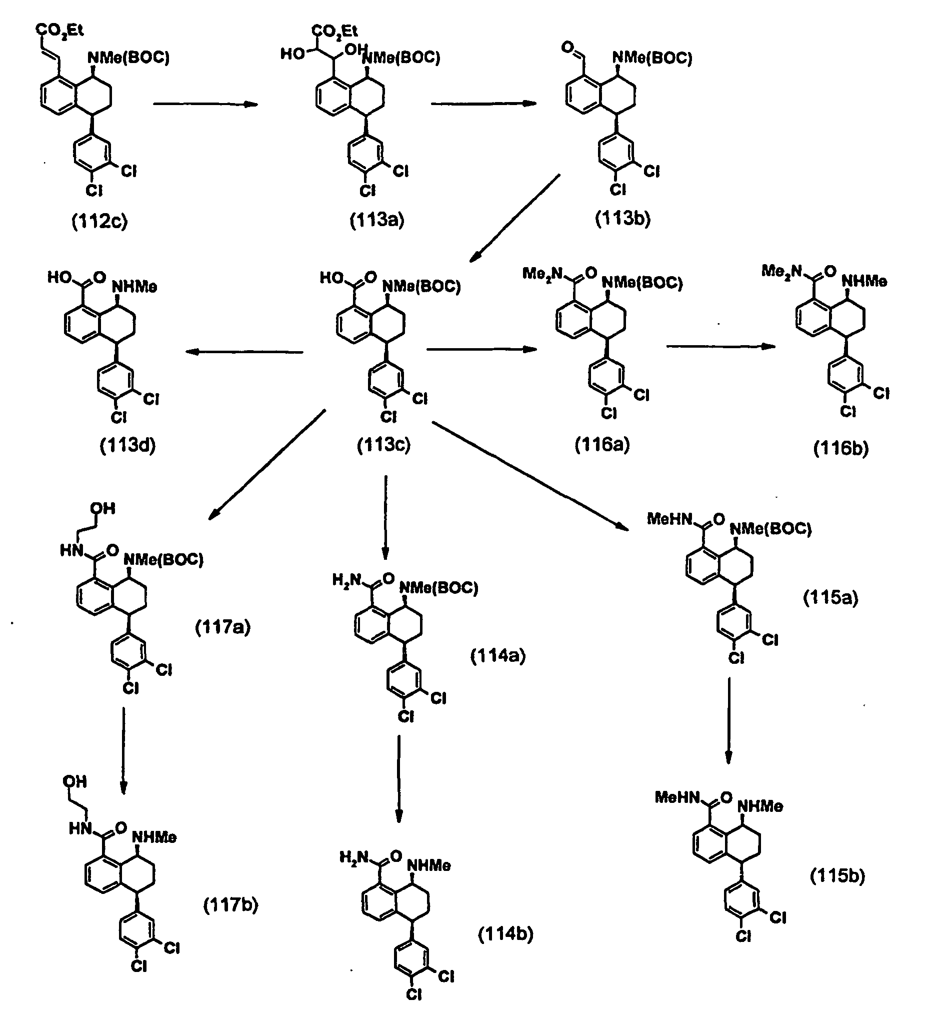

### Example 113

### (5S,8S)-5-(3,4-Dichlorophenyl)-8-(methylamino)-5,6,7,8-tetrahydro-1-naphthalenecarboxylic acid

### (a) Ethyl 3-[(5S,8S)-8-[(tert-butoxycarbonyl)(methyl)amino]-5-(3,4-dichlorophenyl)-5,6,7,8-tetrahydro-1-naphthalenyl}-2,3-dihydroxypropanoate

To a solution of the product of Example 112(c) (745 mg, 1.48 mmol) in acetone (21 ml) at room temperature was added a solution of N-methylmorpholine-N-oxide (520mg, 10% wt solution) and then osmium tetroxide (15 mg, 4% wt in H₂O) and the reaction stirred for 48 hours. The solvent was removed under reduced pressure and partitioned between ethyl acetate and water. The organic phase was dried (MgSO₄), filtered and the solvent removed under reduced pressure to give the intermediate diol (774 mg, 97%) which was used without further purification. MS *m*/*z* 539 (M)⁺. ¹H-NMR (CDCl₃): δ = 1.08-1.40 (3H, m), 1.40-1.65 (9H, m), 1.80-2.25 (4H, m), 2.63 (3H, d), 3.22-3.34 (1H, m), 3.95-4.43 (4H, m), 5.05-5.60 (2H, m), 6.87 (1H, t), 6.98 (1H, m), 7.26 (2H, m), 7.37 (1H, d), 7.52-7.68 (1H, m).

### (b) tert-Butyl (1S,4S)-4-(3,4-dichlorophenyl)-8-formyl-1,2,3,4-tetrahydro-1-naphthalenyl(methyl)carbamate

To a solution of the product of step (a) (774 mg, 1.44 mmol) in dioxan (12.7 ml) at room temperature was added a solution of sodium periodate (338 mg, 1.58 mmol) in water (4.3 ml) and the reaction stirred for 16 hours. The reaction was partitioned between ethyl acetate and water and the organic phase dried (MgSO₄), filtered and the solvent removed under reduced pressure. The residue was purified on silica gel, eluting with ethyl acetate/pentane to give the subtitle compound (503 mg, 81%). MS *m*/*z* 437 (MH)⁺. ¹H-NMR (CDCl₃): δ = 1.50 (9H, s), 1.80-2.02 (3H, m), 2.18 (1H, br), 2.52 (3H, s), 4.19 (1H, s), 5.78-5.98 (1H, br), 6.90 (1H, d), 7.16 (2H, m), 7.37 (1H, t), 7.38 (1H, d), 7.75 (1H, d), 10.16 (1H, s).

### (c) (5S,8S)-8-[(tert-Butoxycarbonyl)(methyl)amino]-5-(3,4-dichlorophenyl)-5,6,7,8-tetrahydro-1-naphthalenecarboxylic acid.

To a solution of the product of step (b) (382 mg, 0.88 mmol) in tert-butanol (22 ml) and 2-methyl-2-butene (6 ml) at room temperature was added a solution of NaClO₂ (856 mg, 10.75 equiv) and KH₂PO₄ (970 mg, 8.1 equiv) in H₂O (9 ml), dropwise, over 10 min. The reaction was then stirred for 16 h. The solvent was then removed under reduced pressure, partitioned between EtOAc and H₂O and the organic phase dried (MgSO₄), filtered and the solvent removed under reduced pressure. The residue was purified on silica gel, eluting with CH₂Cl₂:MeOH to give the subtitle compound (423 mg, 94%). MS *m*/*z* 450. ¹H-NMR (CDCl₃): δ = 1.49 (9H, s), 1.78-2.00 (3H, br), 2.14 (1H, br), 2.58 (3H, s), 4.12 (1H, m), 5.50-5.82 (1H, br), 6.94 (1H, br), 7.06 (1H, d), 7.34 (2H, br), 7.35 (1H, d), 7.60 (1H, s).

### (d) (5S,8S)-5-(3,4-Dichlorophenyl)-8-(methylamino)-5,6,7,8-tetrahydro-1-naphthalenecarboxylic acid

The title compound was prepared from the product of step (c), using the method of Example 94(c). MS *m*/*z* 350 (MH)⁺. ¹H-NMR (d₆-DMSO): δ = 1.94 (2H, m), 2.25 (1H, q), 2.41 (1H, d), 2.76 (3H, s), 4.20 (1H, m), 4.95 (1H, br), 6.97 (1H, d), 7.37 (1H, d), 7.40 (1H, t), 7.59 (1H, s), 7.65 (1H, d), 7.94 (1H, d), 8.42 (0.5H, br), 8.76 (0.5H, br).

### Example 114

### (5S,8S)-5-(3,4-Dichlorophenyl)-8-(methylamino)-5,6,7,8-tetrahydro-1-naphthalene carboxamide hydrochloride

### (a) tert-Butyl (1S,4S)-8-(aminocarbonyl)-4-(3,4-dichlorophenyl)-1,2,3,4-tetrahydro-1-naphthalenyl(methyl)carbamate

To a solution of the product of Example 113(c) (88 mg, 0.2 mmol) in dioxan (1.5 ml) at room temperature was added HOBT (41 mg, 1.5 equiv), N,N-diisopropylethylamine (87 µl, 2.5 equiv), EDC hydrochloride (46 mg, 1.2 equiv) and ammonium carbonate (39 mg, 1.2 equiv) and the reaction stirred overnight. The mixture was partitioned between CH₂Cl₂ and H₂O, the organic phase dried (MgSO₄), filtered and the solvent removed under reduced pressure. The residue was purified on silica gel, eluting with CH₂Cl₂:MeOH to give the subtitle compound (71 mg, 81%). MS *m*/*z* 449 (MH)⁺. ¹H-NMR (CDCl₃): δ = 1.42 (9H, s), 1.81 (2H, br), 1.96 (1H, br), 2.17 (1H, br), 2.69 (3H, br), 4.13 (1H, m), 5.38-5.80 (3H, br), 6.87-7.02 (2H, m), 7.22 (2H, m), 7.35 (2H, m).

### (b) (5S,8S)-5-(3,4-dichlorophenyl)-8-(methylamino)-5,6,7,8-tetrahydro-1-naphthalene carboxamide hydrochloride

HCl gas was bubbled through a solution of the product of step (a) (69 mg) in dichloromethane (5 ml), and cooled in an ice-water bath until the solution was saturated. After 1.5 h, the solvent was removed under reduced pressure to give the title compound (54 mg, 91%). MS *m*/*z* 340 (MH)⁺. ¹H-NMR (d₆-DMSO): δ = 1.90 (2H, br), 2.12 (1H, q), 2.40 (1H, d), 2.93 (3H, s), 4.16 (1H, m), 4.42 (1H, br), 6.86 (1H, d), 7.38 (2H, m), 7.52 (1H, d), 7.64 (2H, m), 7.38 (2H, m), 7.52 (1H, d), 7.64 (2H, m), 8.08 (1H, s), 8.44 (1H, s), 9.02 (1H, br), 9.32 (1H, br).

### Example 115

### (5S,8S)-5-(3,4-dichlorophenyl)-N-methyl-8-(methylamino)-5,6,7,8-tetrahydro-1-naphthalenecarboxamide hydrochloride

### (a) tert-Butyl (1S,4S)-4-(3,4-dichlorophenyl)-8-[(methylamino)carbonyl]-1,2,3,4-tetrahydro-1-naphthalenyl(methyl)carbamate

The subtitle compound was prepared from the product of Example 113(c), using the method of Example 114(a), but using methylamine in THF in place of ammonium carbonate and a 1:0.5 CH₂Cl₂:THF solvent mixture to give the subtitle compound. (52%). MS *m*/*z* 463 (MH)⁺. ¹H-NMR (CDCl₃): δ = 1.44 (9H, s), 1.83 (3H, br), 1.96 (1H, br), 2.18 (1H, br), 2.70 (3H, br), 2.95 (3H, d), 4.12 (1H, m), 5.73 (2H, m), 6.90 (2H, m), 7.19 (3H, m), 7.35 (1H, m).

### (b) (5S,8S)-5-(3,4-Dichlorophenyl)-N-methyl-8-(methylamino)-5,6,7,8-tetrahydro-1-naphthalenecarboxamide hydrochloride

The title compound was prepared from the product of step (a), using the method of Example 94(c) (yield =100%). MS *m*/*z* 353 (MH)⁺. ¹H-NMR (d₆-DMSO): δ = 1.93 (2H, m), 2.16 (1H, q), 2.40 (1H, d), 2.70 (3H, s), 2.92 (3H, s), 4.17 (1H,m), 4.39 (1H, br), 6.87 (1H, d), 7.38 (2H,m), 7.49 (1H, d), 7.62 (1H, d), 7.68 (1H, s), 8.96 (1H, m), 9.04 (1H, br), 9.12 (1H, br)

### Example 116

### (5S,8S)-5-(3,4-Dichlorophenyl)-N,N-dimethyl-8-(methylamino)-5,6,7,8-tetrahydro-1-naphthalenecarboxamide hydrochloride

### (a) tert-Butyl (1S,4S)-4-(3,4-dichlorophenyl)-8-[(dimethylamino)carbonyl]-1,2,3,4-tetrahydro-1-naphthalenyl(methyl)carbamate

The subtitle compound was prepared from the product of Example 113(c), using the method of Example 115(a), but using dimethylamine hydrochloride in place of methylamine (yield = 80%). MS *m*/*z* 477 (MH)⁺. ¹H-NMR (CDCl₃): δ = 1.48 (9H, s), 1.63-2.13 (4H, m), 2.58-2.83 (5H, br), 2.99 (3H, m), 3.10 (1H, m), 4.11 (1H, m), 5.42 (1H, m), 6.90 (2H, m), 7.15 (3H, m), 7.35 (1H, d).

### (b) (5S,8S)-5-(3,4-Dichlorophenyl)-N,N-dimethyl-8-(methylamino)-5,6,7,8-tetrahydro-1-naphthalenecarboxamide hydrochloride

The subtitle compound was prepared from the product of step (a), using the method of Example 94(c) (yield = 57%). MS *m*/*z* 378 (MH)⁺. ¹H-NUR (d₆-DMSO): δ = 1.96 (2H, m), 2.12 (1H, q), 2.39 (1H, d), 2.70 (3H, s), 2.87 (3H, s), 3.09 (3H, s), 4.16 (1H, m), 4.30 (1H, br), 6.79 (1H, d), 7.33 (2H, m), 7.42 (1H, d), 7.64 (1H, d), 7.72 (1H, s), 8.78 (1H, br), 9.21 (1H, br).

### Example 117

### (5S,8S)-5-(3,4-dichlorophenyl)-N-(2-hydroxyethyl)-8-(methylamino)-5,6,7,8-tetrahydro-1-naphthalenecarboxamide

### (a) tert-Butyl (1S,4S)-4-(3,4-dichlorophenyl)-8-{[(2-hydroxyethyl)amino]carbonyl}-1,2,3,4-tetrahydro-1-naphthalenyl(methyl)carbamate

The subtitle compound was prepared from the product of Example 113(c), using the method of Example 115(a), but using ethanolamine in place of methylamine (yield = 80%). MS *m*/*z* 493 (MH)⁺. ¹H-NMR (d₆-DMSO): δ = 1.37 (9H, m), 1.64 (1H, br), 1.73 (1H, br), 1.87 (1H, br), 2.05 (1H, m), 3.29 (2H, m), 3.47 (2H, m), 4.25 (1H, m), 4.56 (1H, m), 5.55 (1H, br), 6.92 (1H, d), 7.00-7.40 (4H, m), 7.57 (1H, t), 8.06 (1H, d).

### (b) (5S,8S)-5-(3,4-Dichlorophenyl)-N-(2-hydroxyethyl)-8-(methylamino)-5,6,7,8-tetrahydro-1-naphthalenecarboxamide

The title compound was prepared from the product of step (a), using the method of Example 94(c) (yield = 100%). MS *m*/*z* 393 (MH)⁺. ¹H-NMR (d₆-DMSO): δ = 1.92 (2H, m), 2.16 (1H, q), 2.40 (1H, d), 2.71 (3H, s), 3.37 (2H, m), 3.56 (2H, m), 4.15 (1H, m), 4.36 (1H, br), 4.84 (1H, m), 6.88 (1H, d), 7.37 (2H, m), 7.50 (1H, d), 7.62 (1H, d), 7.68 (1H, s), 8.96 (1H, s), 9.10 (1H, br), 9.24 (1H, br).

### Example 118

### 2-[(5S,8S)-5-(3,4-Dichlorophenyl)-8-(methylamino)-5,6,7,8-tetrahydro-1-naphthalenyl]-1-ethanesulphonamide hydrochloride

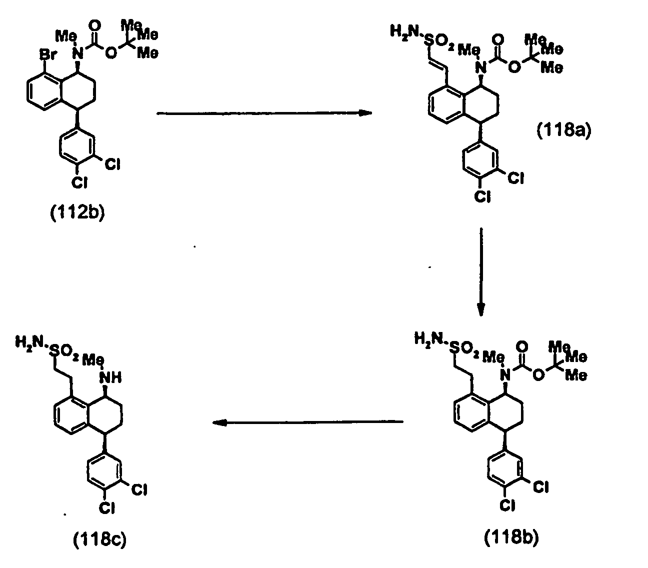

### (a) tert-Butyl (1S,4S)-8-[(E)-2-(aminosulphonyl)ethenyl]-4-(3,4-dichlorophenyl)-1,2,3,4-tetrahydro-1-naphthalenyl(methyl) carbamate

The subtitle compound was prepared from the product of Example 112(b), using the method of Example 106(a), but using vinylsulphonamide in place of ethyl acrylate (yield = 227 mg, 89%). MS *m*/*z* 528 (MH)⁺. ¹H-NMR (CDCl₃): δ = 1.58 (9H, s), 1.73 (1H, m), 1.88 (1H, m), 1.98 (1H, m), 2.19 (1H, m), 2.40 (3H, s), 4.12 (1H, m), 5.14 (2H, s), 5.39 (1H, m), 6.66 (1H, d), 6.85 (1H, d), 7.00 (1H, d), 7.10 (1H, s), 7.25 (1H, m), 7.37 (1H, d), 7.65 (1H, d).

### (b) tert-Butyl (1S,4S)-8-[2-(aminosulphonyl)ethyl]-4-(3,4-dichlorophenyl)-1,2,3,4-tetrahydro-1-naphthalenyl(methyl)carbamate

The subtitle compound was prepared from the product of step (a), using the method of Example 106(b).

### (c) 2-[(5S,8S)-5-(3,4-Dichlorophenyl)-8-(methylamino)-5,6,7,8-tetrahydro-1-naphthalenyl]-1-ethanesulphonamide hydrochloride

The title compound was prepared from the product of step (b), using the method of Example 94(c). MS *m*/*z* 413 (MH)⁺. ¹H-NMR (CDCl₃): δ = 1.73 (1H, m), 1.85 (1H, q), 2.00 (1H, m), 2.32 (1H, d), 2.57 (3H, s), 3.26 (2H, m), 3.48 (1H, m), 3.69 (1H, m), 3.88 (1H, s), 3.95 (1H, m), 6.67 (1H, m), 6.97 (1H, d), 7.10 (2H, m), 7.26 (1H, m), 7.38 (1H, d).

### Example 119

### Biological activity

A number of compounds were assayed for their ability to inhibit the up-take of serotonin by human serotonin transporters as described in Test A. Compounds having an IC₅₀ value less than or equal to100 nM included the title compounds of Examples 1-4, 6-31, 33-35, 37, 39-53, 55, 56, 58-68, 70-72, 74, 76-87, 89-91 and 93.

## Claims

1. A compound of formula I, 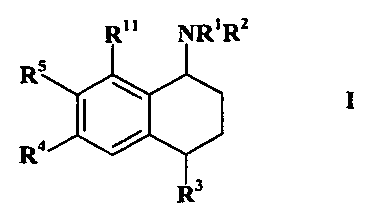 wherein
R¹ and R² independently represent H or C₁₋₆ alkyl;
R³ represents phenyl substituted by at least one group selected from halo, CF₃, OCF₃, CN, OH, C₁₋₆ alkyl and C₁₋₆ alkoxy; and
R⁴, R⁵ and R¹¹ independently represent H or -(CH₂)ₙ-A, wherein n represents 0, 1 or 2, provided that at least one of R⁴, R⁵ and R¹¹ is other than H;
A represents:
CONR⁶R⁷ or SO₂NR⁶R⁷, wherein R⁶ and R⁷ independently represent H, C₃₋₆ cycloalkyl or C₁₋₆ alkyl,
the C₁₋₆ alkyl group being optionally substituted by one or more groups selected from OH, CO₂H, C₃₋₆ cycloalkyl, NH₂, CONH₂, C₁₋₆ alkoxy, C₁₋₆ alkoxycarbonyl and a 5- or 6-membered heterocyclic ring (containing 1, 2 or 3 heteroatoms selected from N, S and O);
in addition, R⁶ and R⁷ may, together with the N atom to which they are attached, represent a pyrrolidine or piperidine ring (which rings are optionally substituted by OH or CONH₂) or a morpholine ring (which is optionally substituted by CONH₂);
CO₂R⁸, wherein R⁸ represents H or C₁₋₆ alkyl;
NR⁹R¹⁰, wherein R⁹ and R¹⁰ independently represent H, C₁₋₆ alkyl (optionally substituted by OH or C₁₋₆ alkoxy), (C₁₋₆ alkyl)SO₂-, (C₁₋₆ alkyl)CO-, H₂NSO₂- or H₂NCO-;
a 5- or 6-membered heterocyclic ring containing 1, 2 or 3 heteroatoms selected from N, S and O, optionally substituted by one or more groups selected from C₁₋₆ alkyl, NH₂, OH, =O and CONHCH₃;
S(O)ₓ(C₁₋₆ alkyl), wherein x represents 0, 1 or 2;
OH;
CN;
NO₂; or
C₁₋₆ alkoxy substituted by one or more groups selected from SO₂NH₂ and CONH₂;
and pharmaceutically acceptable salts thereof.

2. A compound as claimed in claim 1, wherein NR¹R² represents NH(C₁₋₆ alkyl).

3. A compound as claimed in claim 1 or claim 2, wherein R³ represents phenyl disubstituted with halo.

4. A compound as claimed in any one of the preceding claims, wherein R⁴ represents H.

5. A compound as claimed in any one of the preceding claims, wherein R⁵ represents -(CH₂)ₙ-A.

6. A compound as claimed in any one of the preceding claims, wherein R¹¹ represents H.

7. A compound as claimed in any one of the preceding claims, wherein A represents CONR⁶R⁷, SO₂NR⁶R⁷ or NO₂.

8. A compound as claimed in any one of the preceding claims, wherein one of R⁶ and R⁷ represents H, and the other represents H or C₁₋₆ alkyl optionally substituted by OH.

9. A compound as claimed in any one of the preceding claims, wherein n represents 0.

10. A compound as claimed in claim 1, having the stereochemistry shown in formula Ia, 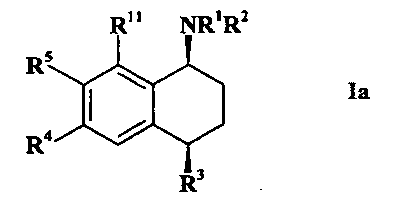 wherein R¹⁻⁵ and R¹¹ are as defined in claim 1.

11. A compound as defined in claim 1, for use as a pharmaceutical.

12. A pharmaceutical formulation containing a compound as defined in claim 1, and a pharmaceutically acceptable adjuvant, diluent or carrier.

13. Use of a compound as defined in claim 1, in the manufacture of a medicament for the treatment or prevention of a disorder in which the regulation of monoamine transporter function is implicated.

14. The use as claimed in claim 13, wherein the disorder is depression, attention deficit hyperactivity disorder, obsessive-compulsive disorder, post-traumatic stress disorder, substance abuse disorders or sexual dysfunction including premature ejaculation.

15. A process for the production of a compound as defined in claim 1, which includes:
(a) when R⁴, R⁵ or R¹¹ represents CONR⁶R⁷, reaction of a compound of formula II, 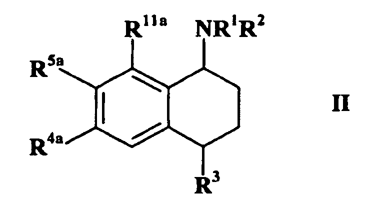 wherein R¹⁻³ are as defined in claim 1, the corresponding group R^{4a}, R^{5a} or R^{11a} represents iodo and the remainder represent H or -(CH₂)ₙ-A, wherein n and A are as defined in claim 1; with CO and an amine of formula III,
**HNR**^{**6**}**R**^{**7**} **III**
wherein R⁶ and R⁷ are as defined in claim 1, in the presence of a Pd(0) catalyst;
(b) when R⁴, R⁵ or R¹¹ represents SO₂NR⁶R⁷, reaction of a compound of formula IV, 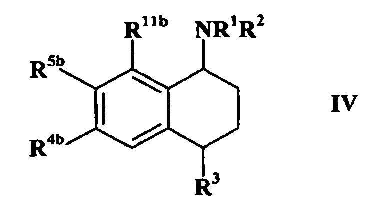 wherein R¹⁻³ are as defined in claim 1, the corresponding group R^{4b}, R^{5b} or R^{11b} represents SO₂Cl and the remainder represent H or -(CH₂)ₙ-A, wherein n and A are as defined in claim 1; with an amine of formula III, as defined above;
(c) when R⁴, R⁵ or R¹¹ represents CO₂(C₁₋₆ alkyl), reaction of a compound of formula II, as defined above; with CO and a C₁₋₆ alkanol in the presence of a Pd(0) catalyst;
(d) when R⁴, R⁵ or R¹¹ represents NO₂, reaction of a compound of formula V, 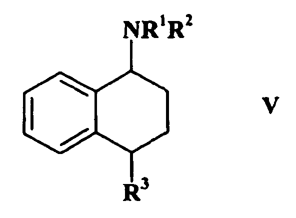 wherein R¹⁻³ are as defined in claim 1; with an alkali metal nitrate and a sulphonic acid;
(e) when R⁴, R⁵ or R¹¹ represents a heterocyclic ring attached to the rest of the molecule by a N atom, reaction of a compound of formula II as defined above; with a heterocyclic compound containing an NH group in the ring, in the presence of a copper catalyst;
(f) when R⁴, R⁵ or R¹¹ represents a heterocyclic ring attached to the rest of the molecule by a C atom, reaction of a compound of formula VI, 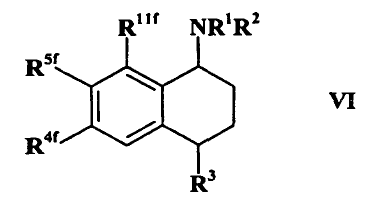 wherein R¹⁻³ are as defined in claim 1, the corresponding group R^{4f}, R^{5f} or R^{11f} represents a group of formula F, 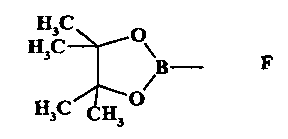 and the remainder represent H or -(CH₂)ₙ-A, wherein n and A are as defined in claim 1; with a heterocyclic compound containing a C-Br or C-I group in the ring, in the presence of a Pd(0) catalyst;
(g) when R⁴, R⁵ or R¹¹ represents a heterocyclic ring attached to the rest of the molecule by a C atom, reaction of a compound of formula II, as defined above; with a heterocyclic compound which is optionally substituted with iodo on the C atom by which the heterocyclic ring will be attached to the rest of the molecule, in the presence of butyl lithium and a Pd(0) catalyst;
(h) when R⁴, R⁵ or R¹¹ represents S(O)ₓ(C₁₋₆ alkyl), reaction of a compound of formula II, as defined above; with a compound of formula VII,
**NaS(C**_{**1-6**} **alkyl) VII**
in the presence of a copper or palladium catalyst, followed by oxidation if desired to give compounds in which x is 1 or 2;
(i) when R⁴, R⁵ or R¹¹ represents CN, reaction of a compound of formula II, as defined above; with zinc cyanide, in the presence of a Pd(0) catalyst;
(j) when R⁴, R⁵ or R¹¹ represents CH₂CH₂SO₂NR⁶R⁷ or CH₂CH₂SO₂(C₁₋₆ alkyl), reaction of a compound of formula U, as defined above; with a compound of formula IX,
**CH**_{**2**}**=CHA**^{**k**} **IX**
wherein A^{k} represents SO₂NR⁶R⁷ or SO₂(C₁₋₆ alkyl) as appropriate, in which R⁶ and R⁷ are as defined in claim 1, in the presence of a Pd(II) catalyst, followed by reduction of the resulting alkene;
(k) when R⁴, R⁵ or R¹¹ represents CH₂CH₂CO₂(C₁₋₆ alkyl), reaction of a compound of formula II, as defined above; with a compound of formula X,
**CH**_{**2**}**=CHCO**_{**2**}**(C**_{**1-6**} **alkyl) X**
in the presence of a Pd(II) catalyst, followed by reduction of the resulting alkene; or
(l) when one of R¹ and R² represents C₁₋₆ alkyl and the other represents H, removing a protecting group from a compound of formula XI, 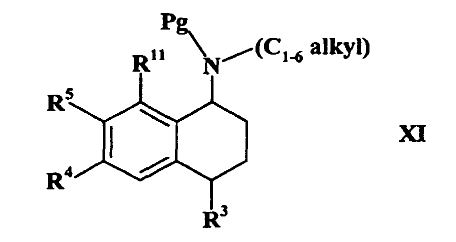 wherein R³⁻⁵ and R¹¹ are as defined in claim 1, and Pg is a protecting group;
and where desired or necessary, converting the resulting compound into a pharmaceutically acceptable salt, or vice versa.

16. Compounds of formulae II, IV, VI and XI as defined below: 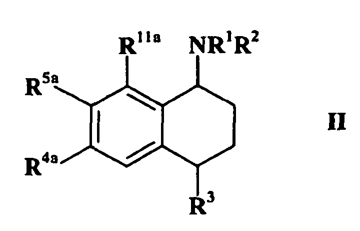 wherein R¹ and R² independently represent H or C₁₋₆ alkyl;
R³ represents phenyl substituted by at least one group selected from halo, CF₃, OCF₃, CN, OH, C₁₋₆ alkyl and C₁₋₆ alkoxy;
one of R^{4a}, R^{5a} or R^{11a} represents iodo and the remainder represent H or -(CH₂)ₙ-A, wherein n represents 0, 1 or 2; and
A represents:
CONR⁶R⁷ or SO₂NR⁶R⁷, wherein R⁶ and R⁷ independently represent H, C₃₋₆ cycloalkyl or C₁₋₆ alkyl,
the C₁₋₆ alkyl group being optionally substituted by one or more groups selected from OH, CO₂H, C₃₋₆ cycloallcyl, NH₂, CONH₂, C₁₋₆ alkoxy, C₁₋₆ alkoxycarbonyl and a 5- or 6-membered heterocyclic ring (containing 1, 2 or 3 heteroatoms selected from N, S and O);
in addition, R⁶ and R⁷ may, together with the N atom to which they are attached, represent a pyrrolidine or piperidine ring (which rings are optionally substituted by OH or CONH₂) or a morpholine ring (which is optionally substituted by CONH₂);
CO₂R⁸, wherein R⁸ represents H or C₁₋₆ alkyl;
NR⁹R¹⁰, wherein R⁹ and R¹⁰ independently represent H, C₁₋₆ alkyl (optionally substituted by OH or C₁₋₆ alkoxy), (C₁₋₆ alkyl)SO₂-, (C₁₋₆ alkyl)CO-, H₂NSO₂- or H₂NCO-;
a 5- or 6-membered heterocyclic ring containing 1, 2 or 3 heteroatoms selected from N, S and O, optionally substituted by one or more groups selected from C₁₋₆ alkyl, NH₂, OH, =O and CONHCH₃;
S(O)ₓ(C₁₋₆ alkyl), wherein x represents 0, 1 or 2;
OH;
CN;
NO₂; or
C₁₋₆ alkoxy substituted by one or more groups selected from SO₂NH₂ and CONH₂;
And pharmaceutically acceptable salts thereof. 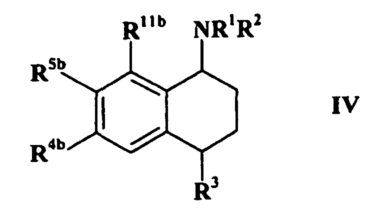 wherein R¹⁻³ are as defined above;
one of R^{4b}, R^{5b} or R^{11b} represents SO₂Cl and the remainder represent H or -(CH₂)ₙ-A,
wherein n and A are as defined above. 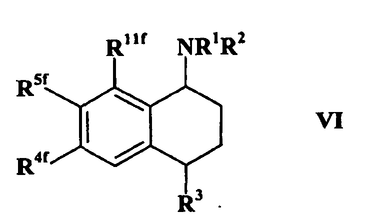 wherein R¹⁻³ are as defined as above;
one of R^{4f}, R^{5f} or R^{11f} represents a group of formula F, 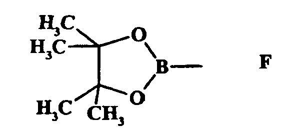 and the remainder represent H or -(CH₂)ₙ-A, wherein n and A are as defined as above. 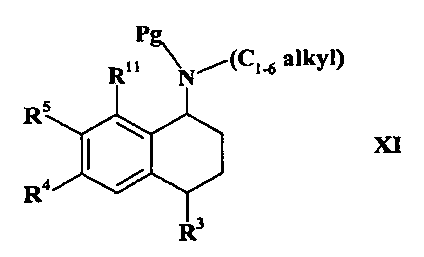 wherein R³ represents phenyl substituted by at least one group selected from halo, CF₃, OCF₃, CN, OH, C₁₋₆ alkyl and C₁₋₆ alkoxy; and
R⁴, R⁵ and R¹¹ independently represent H or -(CH₂)ₙ-A, wherein n represents 0, 1 or 2, provided that at least one of R⁴, R⁵ and R¹¹ is other than H;
A is as defined above;
and Pg is a protecting group.

## Patentansprüche

1. Verbindung der Formel I 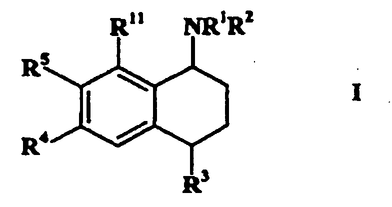 wobei
R¹ und R² unabhängig voneinander H oder C₁₋₆-Alkyl bedeuten;
R³ Phenyl bedeutet, das durch mindestens eine Gruppe substituiert ist, die aus Halogen, CF₃, OCF₃, CN, OH, C₁₋₆-Alkyl und C₁₋₆-Alkoxy ausgewählt ist; und
R⁴, R⁵ und R¹¹ unabhängig voneinander H oder -(CH₂)ₙ-A bedeuten,
wobei n den Wert 0, 1 oder 2 hat, mit der Maßgabe, dass mindestens einer der Reste R⁴, R⁵ und R¹¹ eine von H abweichende Bedeutung hat;
A folgendes bedeutet:
CONR⁶R⁷ oder SO₂NR⁶R⁷, wobei R⁶ und R⁷ unabhängig voneinander H, C₃₋₆-Cycloalkyl oder C₁₋₆-Alkyl bedeuten,
wobei die C₁₋₆-Alkylgruppe optional durch eine oder mehrere Gruppen substituiert ist, die aus OH, CO₂H, C₃₋₆-Cycloalkyl, NH₂, CONH₂, C₁₋₆-Alkoxy, C₁₋₆-Alkoxycarbonyl und einem 5- oder 6-gliedrigen heterocyclischen Ring (enthaltend 1, 2 oder 3 Heteroatome, die aus N, S und O ausgewählt sind) ausgewählt sind;
wobei ferner R⁶ und R⁷ zusammen mit dem N-Atom, an das sie gebunden sind, einen Pyrrolidin- oder Piperidinring (wobei diese Ringe optional durch OH oder CONH₂ substituiert sind) oder einen Morpholinring (der optional durch CONH₂ substituiert ist) bedeuten;
CO₂R⁸, wobei R⁸ H oder C₁₋₆-Alkyl bedeutet;
NR⁹R¹⁰, wobei R⁹ und R¹⁰ unabhängig voneinander H, C₁₋₆-Alkyl (optional substituiert durch OH oder C₁₋₆-Alkoxy), (C₁₋₆-Alkyl)SO₂-, (C₁₋₆-Alkyl)CO-, H₂NSO₂- oder H₂NCO- bedeuten;
einen 5- oder 6-gliedrigen heterocyclischen Ring enthaltend 1, 2 oder 3 Heteroatome, die aus N, S und O ausgewählt sind, optional substituiert durch eine oder mehrere Gruppen, die aus C₁₋₆-Alkyl, NH₂, OH, =O und CONHCH₃ ausgewählt sind;
S(O)ₓ(C₁₋₆-Alkyl), wobei x den Wert 0, 1 oder 2 hat;
OH;
CN;
NO₂; oder
C₁₋₆-Alkoxy, das durch eine oder mehrere Gruppen substituiert ist, die aus SO₂NH₂ und CONH₂ ausgewählt sind;
und pharmazeutisch akzeptable Salze davon.

2. Verbindung nach Anspruch 1, wobei NR¹R² die Bedeutung NH(C₁₋₆-Alkyl) hat.

3. Verbindung nach Anspruch 1 oder 2, wobei R³ Phenyl bedeutet, das durch Halogen disubstituiert ist.

4. Verbindung nach einem der vorstehenden Ansprüche, wobei R⁴ H bedeutet.

5. Verbindung nach einem der vorstehenden Ansprüche, wobei R⁵ die Bedeutung (CH₂)ₙ-A hat.

6. Verbindung nach einem der vorstehenden Ansprüche, wobei R¹¹ H bedeutet.

7. Verbindung nach einem der vorstehenden Ansprüche, wobei A CONR⁶R⁷, SO₂NR⁶R⁷ oder NO₂ bedeutet.

8. Verbindung nach einem der vorstehenden Ansprüche, wobei einer der Reste R⁶ und R⁷ H bedeutet und der andere Rest H oder C₁₋₆-Alkyl, das optional durch OH substituiert ist, bedeutet.

9. Verbindung nach einem der vorstehenden Ansprüche, wobei n den Wert 0 hat.

10. Verbindung nach Anspruch 1, mit der in der Formel Ia gegebenen stereochemischen Beschaffenheit 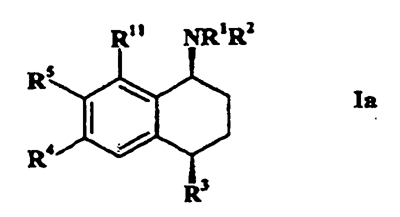 wobei R¹⁻⁵ und R¹¹ die in Anspruch 1 definierten Bedeutungen haben.

11. Verbindung nach Anspruch 1 zur Verwendung als Pharmazeutikum.

12. Pharmazeutische Formulierung, enthaltend eine Verbindung nach Anspruch 1 und ein pharmazeutisch akzeptables Adjuvans, Verdünnungsmittel oder Trägerstoff.

13. Verwendung einer Verbindung nach Anspruch 1 bei der Herstellung eines Arzneimittels zur Therapie oder Prophylaxe einer Störung, bei der die Regulierung der Monoamin-Transporter-Funktion impliziert ist.

14. Verwendung nach Anspruch 13, wobei es sich bei der Störung um eine Depression, eine Aufmerksamkeitsdefizit-Hyperaktivitätsstörung, eine obsessiv-kompulsive Störung, eine posttraumatische Belastungsstörung, Störungen bei Missbrauch von Substanzen oder eine sexuelle Funktionsstörung unter Einschluss einer vorzeitigen Ejakulation handelt.

15. Verfahren zur Herstellung einer Verbindung nach Anspruch 1, umfassend:
(a) wenn R⁴, R⁵ oder R¹¹ die Bedeutung CONR⁶R⁷ hat, die Umsetzung einer Verbindung der Formel II 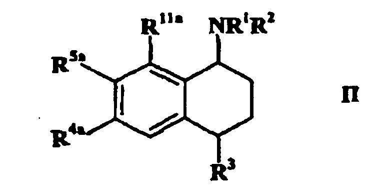 wobei R¹⁻³ die in Anspruch 1 definierten Bedeutungen haben, die entsprechende Gruppe R^{4a}, R^{5a} oder R^{11a} Iod bedeutet und die restlichen Gruppen H oder -(CH₂)ₙ-A bedeuten, wobei n und A die in Anspruch 1 definierte Bedeutung haben,
mit CO und einem Amin der Formel III
HNR⁶R⁷ III
wobei R⁶ und R⁷ die in Anspruch 1 definierten Bedeutungen haben, in Gegenwart eines Pd(0)-Katalysators;
(b) wenn R⁴ , R⁵ oder R¹¹ die Bedeutung SO₂NR⁶R⁷ hat, die Umsetzung einer Verbindung der Formel IV 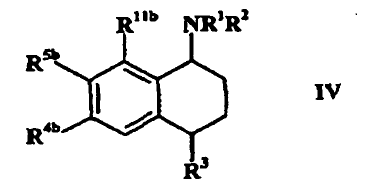 wobei R¹⁻³ die in Anspruch 1 definierten Bedeutungen haben, die entsprechende Gruppe R^{4b}, R^{5b} oder R^{11b} die Bedeutung SO₂Cl hat und die restlichen Gruppen H oder -(CH₂)ₙ-A bedeuten, wobei n und A die in Anspruch 1 definierten Bedeutungen haben,
mit einem Amin der Formel III gemäß der vorstehenden Definition;
(c) wenn R⁴, R⁵ oder R¹¹ die Bedeutung CO₂(C₁₋₆-Alkyl) hat, die Umsetzung einer Verbindung der Formel II gemäß der vorstehenden Definition mit CO und einem C₁₋₆-Alkanol in Gegenwart eines Pd(0)-Katalysators;
(d) wenn R⁴, R⁵ oder R¹¹ die Bedeutung NO₂ hat, die Umsetzung einer Verbindung der Formel V 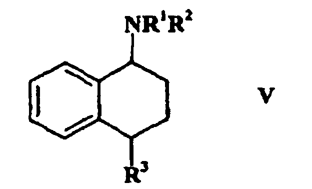 wobei R¹⁻³ die in Anspruch 1 definierten Bedeutungen haben, mit einem Alkalimetallnitrat und einer Sulfonsäure;
(e) wenn R⁴, R⁵ oder R¹¹ einen heterocyclischen Ring bedeutet, der über ein N-Atom an den Rest des Moleküls gebunden ist, die Umsetzung einer Verbindung der Formel II gemäß der vorstehenden Definition mit einer heterocyclischen Verbindung, die eine NH-Gruppe im Ring enthält, in Gegenwart eines Kupferkatalysators;
(f) wenn R⁴, R⁵ oder R¹¹ einen heterocyclischen Ring bedeutet, der über ein C-Atom an den Rest des Moleküls gebunden ist, die Umsetzung einer Verbindung der Formel VI 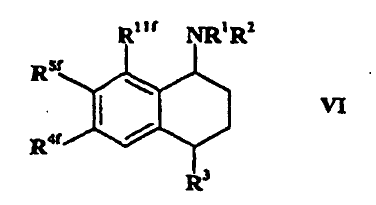 wobei R¹⁻³ die in Anspruch 1 definierten Bedeutungen haben, die entsprechende Gruppe R^{4f}, R^{5f} oder R^{11f} eine Gruppe der Formel F bedeutet 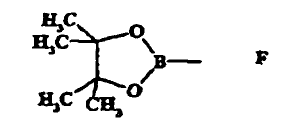 und die restlichen Gruppen H oder -(CH₂)ₙ-A bedeuten, wobei n und A die in Anspruch 1 definierten Bedeutungen haben,
mit einer heterocyclischen Verbindung, die im Ring eine C-Br- oder C-I-Gruppe enthält, in Gegenwart eines Pd(0)-Katalysators;
(g) wenn R⁴, R⁵ oder R¹¹ einen heterocyclischen Ring bedeutet, der über ein C-Atom an den Rest des Moleküls gebunden ist, die Umsetzung einer Verbindung der Formel II gemäß der vorstehenden Definition mit einer heterocyclischen Verbindung, die optional durch Iod am C-Atom, mit dem der heterocyclische Ring an den Rest des Moleküls gebunden ist, substituiert ist, in Gegenwart von Butyllithium und einem Pd(0)-Katalysator;
(h) wenn R⁴, R⁵ oder R¹¹ die Bedeutung S(O)ₓ(C₁₋₆-Alkyl) hat, die Umsetzung einer Verbindung der Formel II gemäß der vorstehenden Definition mit einer Verbindung der Formel VII
NaS(C₁₋₆-Alkyl) VII
in Gegenwart eines Kupfer- oder Palladiumkatalysators, optional unter anschließender Oxidation, unter Bildung von Verbindungen, in denen x den Wert 1 oder 2 hat;
(i) wenn R⁴, R⁵ oder R¹¹ die Bedeutung CN hat, die Umsetzung einer Verbindung der Formel II gemäß der vorstehenden Definition mit Zinkcyanid in Gegenwart eines Pd(0)-Katalysators;
(j) wenn R⁴, R⁵ oder R¹¹ die Bedeutung CH₂CH₂SO₂NR⁶R⁷ oder CH₂CH₂SO₂(C₁₋₆-Alkyl) hat, die Umsetzung einer Verbindung der Formel II gemäß der vorstehenden Definition mit einer Verbindung der Formel IX
CH₂=CHA^{k} IX
wobei A^{k} in geeigneter Weise die Bedeutung SO₂NR⁶R⁷ oder SO₂(C₁₋₆-Alkyl) hat, wobei R⁶ und R⁷ die in Anspruch 1 definierten Bedeutungen haben, in Gegenwart eines Pd(II)-Katalysators, gefolgt von einer Reduktion des erhaltenen Alkens;
(k) wenn R⁴, R⁵ oder R¹¹ die Bedeutung CH₂CH₂CO₂(C₁₋₆-Alkyl) hat, die Umsetzung einer Verbindung der Formel II gemäß der vorstehenden Definition mit einer Verbindung der Formel X
CH₂=CHCO₂(C₁₋₆-Alkyl) X
in Gegenwart eines Pd(II)-Katalysators, gefolgt von einer Reduktion des erhaltenen Alkens; oder
(l) wenn einer der Reste R¹ und R² die Bedeutung C₁₋₆-Alkyl hat und der andere die Bedeutung H hat, die Entfernung einer Schutzgruppe von einer Verbindung der Formel XI 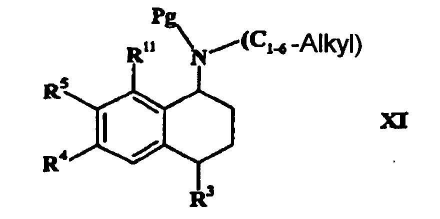 wobei R³⁻⁵ und R¹¹ die in Anspruch 1 definierten Bedeutungen haben und Pg eine Schutzgruppe bedeutet;
und, falls erwünscht oder notwendig, die Überführung der erhaltenen Verbindung in ein pharmazeutisch akzeptables Salz oder umgekehrt.

16. Verbindungen der Formeln II, IV, VI und XI gemäß den nachstehenden Definitionen 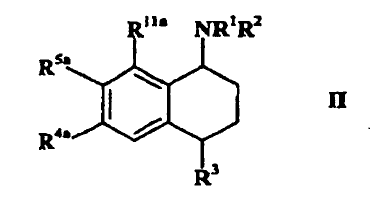 wobei R¹ und R² unabhängig voneinander H oder C₁₋₆-Alkyl bedeuten;
R³ Phenyl bedeutet, das durch mindestens eine Gruppe substituiert ist, die aus Halogen, CF₃, OCF₃, CN, OH, C₁₋₆-Alkyl und C₁₋₆-Alkoxy ausgewählt ist;
einer der Reste R^{4a}, R^{5a} oder R^{11a} Iod bedeutet und die übrigen Reste H oder -(CH₂)ₙ-A bedeuten, wobei n den Wert 0, 1 oder 2 hat; und
A folgendes bedeutet:
CONR⁶R⁷ oder SO₂NR⁶R⁷, wobei R⁶ und R⁷ unabhängig voneinander H, C₃₋₆-Cycloalkyl oder C₁₋₆-Alkyl bedeuten,
wobei die C₁₋₆-Alkylgruppe optional durch eine oder mehrere Gruppen substituiert ist, die aus OH, CO₂H, C₃₋₆-Cycloalkyl, NH₂, CONH₂, C₁₋₆-Alkoxy, C₁₋₆-Alkoxycarbonyl und einem 5- oder 6-gliedrigen heterocyclischen Ring (enthaltend 1, 2 oder 3 Heteroatome, die aus N, S und O ausgewählt sind) ausgewählt sind;
wobei ferner R⁶ und R⁷ zusammen mit dem N-Atom, an das sie gebunden sind, einen Pyrrolidin- oder Piperidinring (wobei diese Ringe optional durch OH oder CONH₂ substituiert sind) oder einen Morpholinring (der optional durch CONH₂ substituiert ist) bedeuten können;
CO₂R⁸, wobei R⁸ H oder C₁₋₆-Alkyl bedeutet;
NR⁹R¹⁰, wobei R⁹ und R¹⁰ unabhängig voneinander H, C₁₋₆-Alkyl (optional substituiert durch OH oder C₁₋₆-Alkoxy), (C₁₋₆-Alkyl)SO₂-, (C₁₋₆-Alkyl)CO-, H₂NSO₂- oder H₂NCO- bedeuten;
einen 5- oder 6-gliedrigen heterocyclischen Ring enthaltend 1, 2 oder 3 Heteroatome, die aus N, S und O ausgewählt sind, optional substituiert durch eine oder mehrere Gruppen, die aus C₁₋₆-Alkyl, NH₂, OH, =O und CONHCH₃ ausgewählt sind;
S(O)ₓ(C₁₋₆-Alkyl), wobei x den Wert 0, 1 oder 2 hat;
OH;
CN;
NO₂; oder
C₁₋₆-Alkoxy, das durch eine oder mehrere Gruppen substituiert ist, die aus SO₂NH₂ und CONH₂ ausgewählt sind;
und pharmazeutisch akzeptable Salze davon; 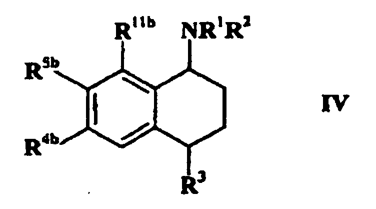 wobei R¹⁻³ die vorstehend definierten Bedeutungen haben;
einer der Reste R^{4b}, R^{5b} oder R^{11b} die Bedeutung SO₂Cl hat und die übrigen Reste H oder -(CH₂)ₙ-A bedeuten, wobei n und A die vorstehend definierten Bedeutungen haben; 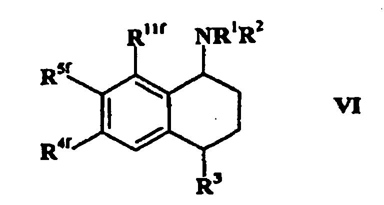 wobei R¹⁻³ die vorstehend definierten Bedeutungen haben;
einer der Reste R^{4f}, R^{5f} oder R^{11f} eine Gruppe der Formel F bedeutet 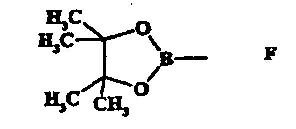 und die übrigen Reste H oder -(CH₂)ₙ-A bedeuten, wobei n und A die vorstehend definierten Bedeutungen haben; 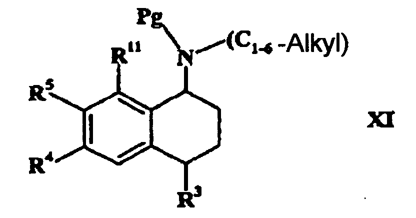 wobei R³ Phenyl bedeutet, das durch mindestens eine Gruppe substituiert ist, die aus Halogen, CF₃, OCF₃, CN, OH, C₁₋₆-Alkyl und C₁₋₆-Alkoxy ausgewählt ist; und R⁴, R⁵ und R¹¹ unabhängig voneinander H oder -(CH₂)ₙ-A bedeuten, wobei n den Wert 0, 1 oder 2 hat, mit der Maßgabe, dass mindstens einer der Reste R⁴, R⁵ und R¹¹ eine von H abweichende Bedeutung hat;
A die vorstehend definierte Bedeutung hat;
und Pg eine Schutzgruppe bedeutet.

## Revendications

1. Composé de formule I, 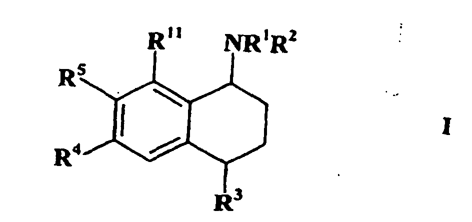 dans laquelle
R¹ et R² représentent indépendamment H ou un groupe alkyle en C₁ à C₆ ;
R³ représente un groupe phényle substitué avec au moins un groupe choisi entre des groupes halogéno, CF₃, OCF₃, CN, OH, alkyle en C₁ à C₆ et alkoxy en C₁ à C₆ ; et
R⁴, R⁵ et R¹¹ représentent indépendamment H ou un groupe -(CH₂)ₙ-A, dans lequel N est égal à 0, 1 ou 2, sous réserve qu'au moins un des groupes R⁴, R⁵ et R¹¹ soient autres que H ;
A représente :
un groupe CONR⁶R⁷ ou SO₂NR⁶R⁷, dans lequel R⁶ et R⁷ représentent indépendamment H, un groupe cycloalkyle
en C₃ à C₆ ou alkyle en C₁ à C₆,
le groupe alkyle en C₁ à C₆ étant facultativement substitué avec un ou plusieurs groupes choisis entre des groupes OH, CO₂H, cycloalkyle en C₃ à C₆, NH₂, CONH₂, alkoxy en C₁ à C₆, (alkoxy en C₁ à C₆)carbonyle et un noyau hétérocyclique penta- ou hexagonal (contenant 1, 2 ou 3 hétéroatomes choisis entre les atomes de N, S et O) ;
en outre, R⁶ et R⁷ peuvent, conjointement avec l'atome de N auquel ils sont fixés, représenter un noyau pyrrolidine ou pipéridine (ces noyaux étant facultativement substitués avec des substituants OH ou CONH₂) ou un noyau morpholine (qui est facultativement substitué avec un substituant CONH₂) ;
un groupe CO₂R⁸ dans lequel R⁸ représente H ou un groupe alkyle en C₁ à C₆ ;
un groupe NR⁹R¹⁰, dans lequel R⁹ et R¹⁰ représentent indépendamment H, un groupe alkyle en C₁ à C₆ (facultativement substitué avec un substituant OH ou alkoxy en C₁ à C₆), (alkyle en C₁ à C₆)SO₂-, (alkyle en C₁ à C₆)CO-, H₂NSO₂- ou H₂NCO- ;
un noyau hétéroxyclique penta- ou hexagonal contenant 1, 2 ou 3 hétéroatomes choisis entre des atomes de N, S et O, facultativement substitué avec un ou plusieurs groupes choisis entre des groupes alkyle en C₁ à C₆, NH₂, OH, =O et CONHCH₃ ;
un groupe S(O)ₓ(alkyle en C₁ à C₆) dans lequel x est égal à 0, 1 ou 2 ;
un groupe OH ;
un groupe CN ;
un groupe NO₂ ; ou
un groupe alkoxy en C₁ à C₆ substitué avec un ou plusieurs groupes choisis entre des groupes SO₂NH₂ et CONH₂ ;
et ses sels pharmaceutiquement acceptables.

2. Composé suivant la revendication 1, dans lequel NR¹R² représentent un groupe NH(alkyle en C₁ à C₆).

3. Composé suivant la revendication 1 ou la revendication 2, dans lequel R³ représente un groupe phényle disubstitué avec un substituant halogéno.

4. Composé suivant l'une quelconque des revendications précédentes, dans lequel R⁴ représente H.

5. Composé suivant l'une quelconque des revendications précédentes, dans lequel R⁵ représente un groupe -(CH₂)ₙ-A.

6. Composé suivant l'une quelconque des revendications précédentes, dans lequel R¹¹ représente H.

7. Composé suivant l'une quelconque des revendications précédentes, dans lequel A représente un groupe CONR⁶R⁷, SO₂NR⁶R⁷ ou NO₂.

8. Composé suivant l'une quelconque des revendications précédentes, dans lequel un des groupes R⁶ et R⁷ représente H et l'autre représente H ou un groupe alkyle en C₁ à C₆ facultativement substitué avec un substituant OH.

9. Composé suivant l'une quelconque des revendications précédentes, dans lequel n est égal à 0.

10. Composé suivant la revendication 1, ayant la stéréochimie représentée dans la formule Ia, 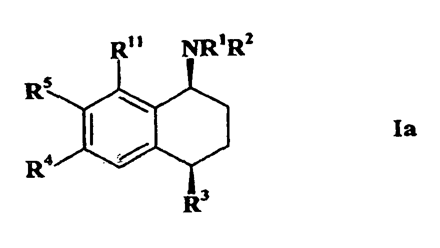 dans laquelle R¹⁻⁵ et R¹¹ répondent aux définitions figurant dans la revendication 1.

11. Composé tel que défini dans la revendication 1, destiné à être utilisé comme agent pharmaceutique.

12. Formulation pharmaceutique contenant un composé tel que défini dans la revendication 1, et un adjuvant, diluant ou support pharmaceutiquement acceptable.

13. Utilisation d'un composé tel que défini dans la revendication 1, dans la production d'un médicament destiné au traitement ou à la prévention d'une affection dans laquelle est impliquée la régulation de la fonction de transporteur de monoamines.

14. Utilisation suivant la revendication 13, dans laquelle l'affection est la dépression, un trouble d'hyperactivité avec déficit d'attention, le syndrome obsessionnel compulsif, le syndrome de stress post-traumatique, des troubles par abus de substances ou un dysfonctionnement sexuel, y compris l'éjaculation précoce.

15. Procédé pour la production d'un composé tel que défini dans la revendication 1, qui comprend :
(a) lorsque R⁴, R⁵ ou R¹¹ représente un groupe CONR⁶R⁷, la réaction d'un composé de formule (II), 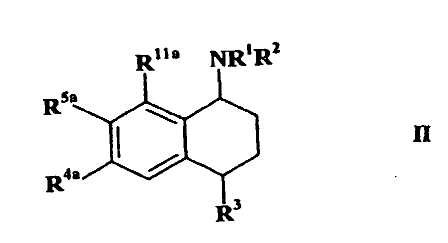 dans laquelle R¹⁻³ sont tels que définis dans la revendication 1, le groupe R^{4a}, R^{5a} ou R^{11a} correspondant représente un groupe iodo et le reste représente H ou des groupes -(CH₂)ₙ-A, dans lesquels n et A sont tels que définis dans la revendication 1 ; avec CO et une amine de formule III,
**HNR**^{**6**}**R**^{**7**} **III**
dans laquelle R⁶ et R⁷ sont tels que définis dans la revendication 1, en présence d'un catalyseur au Pd(0) ;
(b) lorsque R⁴, R⁵ ou R¹¹ représente un groupe SO₂NR⁶R⁷, la réaction d'un composé de formule IV, 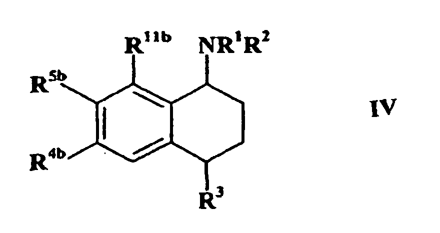 dans laquelle R¹⁻³ sont tels que définis dans la revendication 1, le groupe R^{4b}, R^{5b} ou R^{11b} correspondant représente un groupe SO₂Cl et le reste représente H ou des groupes -(CH₂)ₙ-A, dans lesquels n et A sont tels que définis dans la revendication 1 ; avec une amine de formule III, répondant à la définition précitée ;
(c) lorsque R⁴, R⁵ ou R¹¹ représente un groupe CO₂ (alkyle en C₁ à C₆), la réaction d'un composé de formule II répondant à la définition précitée, avec CO et un alcanol en C₁ à C₆ en présence d'un catalyseur au Pd(0) ;
(d) lorsque R⁴, R⁵ ou R¹¹ représente un groupe NO₂, la réaction d'un composé de formule V, 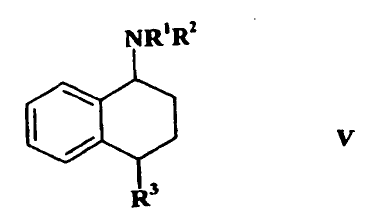 dans laquelle R¹⁻³ sont tels que définis dans la revendication 1 ; avec un nitrate de métal alcalin et un acide sulfonique ;
(e) lorsque R⁴, R⁵ ou R¹¹ représente un noyau hétérocyclique fixé au reste de la molécule par un atome de N, la réaction d'un composé de formule II répondant à la définition précitée ; avec un composé hétérocyclique contenant un groupe NH dans le noyau, en présence d'un catalyseur au cuivre ;
(f) lorsque R⁴, R⁵ ou R¹¹ représente un noyau hétérocyclique fixé au reste de la molécule par un atome de C, la réaction d'un composé de formule VI, 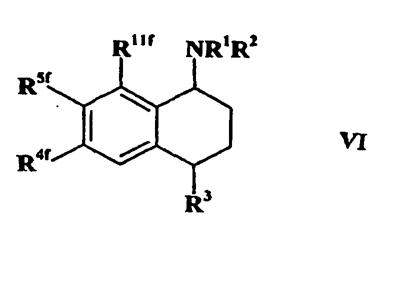 dans laquelle R¹⁻³ sont tels que définis dans la revendication 1, le groupe R^{4f}, R^{5f} ou R^{11f} correspondant représente un groupe de formule F, 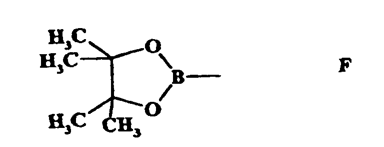 et le reste représente H ou des groupes -(CH₂)ₙ-A, dans lesquels n et A sont tels que définis dans la revendication 1 ; avec un composé hétérocyclique contenant un groupe C-Br ou C-I dans le noyau, en présence d'un catalyseur au Pd(0) ;
(g) lorsque R⁴, R⁵ ou R¹¹ représente un noyau hétérocyclique fixé au reste de la molécule par un atome de C, la réaction d'un composé de formule II, répondant à la définition précitée, avec un composé hétérocyclique qui est facultativement substitué avec un substituant iodo sur l'atome de C par lequel le noyau hétérocyclique est fixé au reste de la molécule, en présence de butyllithium et d'un catalyseur au Pd(0) ;
(h) lorsque R⁴, R⁵ ou R¹¹ représente un groupe S(O)ₓ(alkyle en C₁ à C₆), la réaction d'un composé de formule II, répondant à la définition précitée ; avec un composé de formule VII,
**NaS (alkyle en C**_{**1**} **à C**_{**6**}**) VII**
en présence d'un catalyseur au cuivre ou au palladium, avec ensuite une oxydation si cela est désiré pour obtenir des composés dans lesquels x est égal à 1 ou 2 ;
(i) lorsque R⁴, R⁵ ou R¹¹ représente un groupe CN, la réaction des composés de formule II, répondant à la définition précitée, avec du cyanure de zinc, en présence d'un catalyseur au Pd(0) ;
(j) lorsque R⁴, R⁵ ou R¹¹ représente un groupe CH₂CH₂SO₂NR⁶R⁷ ou CH₂CH₂SO₂(alkyle en C₁ à C₆), la réaction d'un composé de formule II, répondant à la définition précitée ; avec un composé de formule IX,
**CH**_{**2**}**=CHA**^{**k**} **IX**
dans laquelle A^{k} représente un groupe SO₂NR⁶R⁷ ou SO₂(alkyle en C₁ à C₆) de la manière appropriée, dans lequel R⁶ et R⁷ sont tels que définis dans la revendication 1, en présence d'un catalyseur au Pd(II), avec ensuite une réduction de l'alcène résultant ;
(k) lorsque R⁴, R⁵ ou R¹¹ représente un groupe CH₂CH₂CO₂(alkyle en C₁ à C₆), la réaction d'un composé de formule II, répondant à la définition précitée, avec un composé de formule X,
**CH**_{**2**}**=CHCO**_{**2**}**(alkyle en C**_{**1**} **à C**_{**6**}**) X**
en présence d'un catalyseur au Pd(II), avec ensuite une réduction de l'alcène résultant ; ou
(l) lorsqu'un des groupes R¹ et R² représente un groupe alkyle en C₁ à C₆ et l'autre représente H, l'élimination d'un groupe protecteur d'un composé de formule XI, 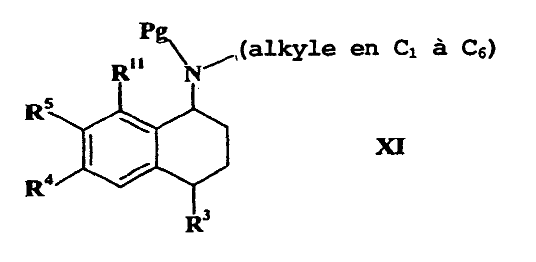 dans laquelle R³⁻⁵ et R¹¹ sont tels que définis dans la revendication 1 et Pg représente un groupe protecteur et, lorsque cela est désiré ou nécessaire, la conversion du composé résultant en un sel pharmaceutiquement acceptable ou vice-versa.

16. Composés de formules II, IV, VI et XI tels que définis ci-dessous : 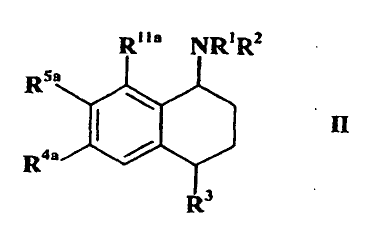 dans laquelle R¹ et R² représentent indépendamment H ou des groupes alkyle en C₁ à C₆.
R³ représente un groupe phényle substitué avec au moins un groupe choisi entre des groupes halogéno, CF₃, OCF₃, CN, OH, alkyle en C₁ à C₆ et alkoxy en C₁ à C₆.
un des groupes R^{4a}, R^{5a} ou R^{11a} représente un groupe iodo et le reste représente H ou des groupes -(CH₂)ₙ-A, dans lesquels n est égal à 0, 1 ou 2 ; et
A représente :
un groupe CONR⁶R⁷ ou SO₂NR⁶R⁷, dans lequel R⁶ et R⁷ représentent indépendamment H, un groupe cycloalkyle
en C₃ à C₆ ou alkyle en C₁ à C₆,
le groupe alkyle en C₁ à C₆ étant facultativement substitué avec un ou plusieurs groupes choisis entre des groupes OH, CO₂H, cycloalkyle en C₃ à C₆, NH₂, CONH₂, alkoxy en C₁ à C₆, (alkoxy en C₁ à C₆)carbonyle et un noyau hétérocyclique penta- ou hexagonal (contenant 1, 2 ou 3 hétéroatomes choisis entre les atomes de N, S et O) ;
en outre, R₆ et R₇ peuvent, conjointement avec l'atome de N auquel ils sont fixés, représenter un noyau pyrrolidine ou pipéridine (ces noyaux étant facultativement substitués avec des substituants OH ou CONH₂) ou un noyau morpholine (qui est facultativement substitué avec un substituant CONH₂) ;
un substituant CO₂R⁸, dans lequel R⁸ représente H ou un groupe alkyle en C₁ à C₆ ;
un groupe NR⁹R¹⁰, dans lequel R⁹ et R¹⁰ représentent indépendamment H, un groupe alkyle en C₁ à C₆ (facultativement substitué avec un substituant OH ou alkoxy en C₁ à C₆), (alkyle en C₁ à C₆)SO₂-, (alkyle en C₁ à C₆)CO-, H₂NSO₂- ou H₂NCO- ;
un noyau hétérocyclique penta- ou hexagonal contenant 1, 2 ou 3 hétéroatomes choisis entre des atomes de N, S et O, facultativement substitué avec un ou plusieurs groupes choisis entre des groupes alkyle en C₁ à C₆, NH₂, OH, =O et CONHCH₃ ;
un groupe S(O)ₓ(alkyle en C₁ à C₆) dans lequel x est égal à 0, 1 ou 2 ;
un groupe OH ;
un groupe CN ;
un groupe NO₂ ; ou
un groupe alkoxy en C₁ à C₆ substitué avec un ou plusieurs groupes choisis entre des groupes SO₂NH₂ et CONH₂ ;
et leurs sels pharmaceutiquement acceptables. 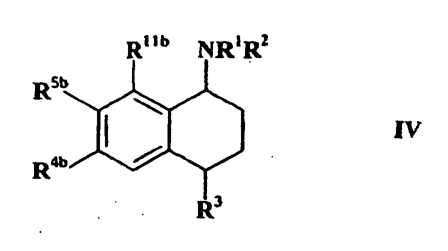 formule dans laquelle R¹⁻³ répond aux définitions précitées ;
un des groupes R^{4b}, R^{5b} et R^{11b} représente un groupe SO₂Cl et le reste représente H ou des groupes -(CH₂)ₙ-A, dans lesquels n et A répondent aux définitions précitées. 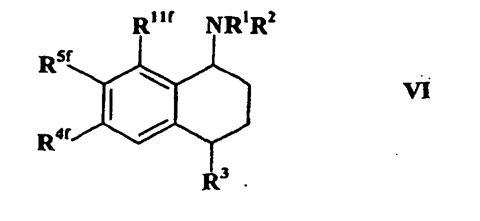 formule dans laquelle R¹⁻³ répondent aux définitions précitées ;
un des groupes R^{4f}, R^{5f} et R^{11f} représente un groupe de formule F, 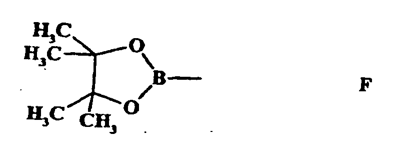 et le reste représente H ou des groupes -(CH₂)ₙ-A, dans lesquels n et A répondent aux définitions précitées, 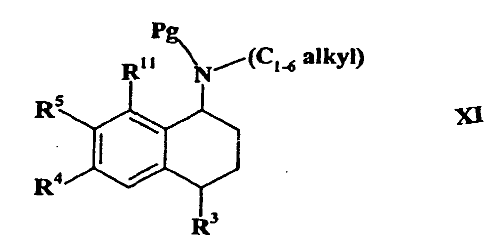 formule dans laquelle R³ représente un groupe phényle substitué avec au moins un groupe choisi entre des groupes halogéno, CF₃, OCF₃, CN, OH, alkyle en C₁ à C₆ et alkoxy en C₁ à C₆ ; et
R⁴, R⁵ et R¹¹ représentent indépendamment H ou un groupe -(CH₂)ₙ-A, dans lequel n est égal à 0, 1 ou 2, sous réserve qu'au moins un des groupes R⁴, R⁵ et R¹¹ soit autre que H ;
A répond à la définition précitée :
et Pg représente un groupe protecteur.
